# EUROPÄISCHE PATENTSCHRIFT

(11) **EP 1 006 098 B1**
(45) Veröffentlichungstag und Bekanntmachung des Hinweises auf die Patenterteilung: **07.04.2004**
(21) Anmeldenummer: 99123223.2
(22) Anmeldetag: 25.11.1999
(51) Int. Cl.: C07C 13/28, C07C 69/75, C07C 43/18, C09K 19/30

(54) **Querverbrückte Cyclohexan-Derivate und flüssigkristallines Medium**
Cross-bridged cyclohexane derivatives and liquid crystal medium
Dérivés de cyclohexane pontés perpendiculairement et milieu liquide cristallin

(30) Priorität: 03.12.1998 DE 19855757
(43) Veröffentlichungstag der Anmeldung: 07.06.2000
(73) Patentinhaber: MERCK PATENT GmbH, 64293 Darmstadt (DE)
(72) Erfinder: Reiffenrath, Volker, 64380 Rossdorf (DE); Heckmeier, Michael, Dr., 64625 Bensheim (DE); Bremer, Matthias, Dr., 64295 Darmstadt (DE)

(56) Entgegenhaltungen:
- EP-A- 0 672 742
- EP-A- 0 733 610
- CH-A- 684 409
- US-A- 2 809 988
- THURMES W N ET AL: "NEGATIVE BIREFRINGENCE FERROELECTRIC LIQUID CRYSTALS" LIQUID CRYSTALS,GB,TAYLOR AND FRANCIS LTD, LONDON, Bd. 25, Nr. 2, 1. August 1998 (1998-08-01), Seiten 149-151, XP000773013 ISSN: 0267-8292

## Beschreibung

Die Erfindung betrifft neue Cyclohexan-Derivate der Formel I 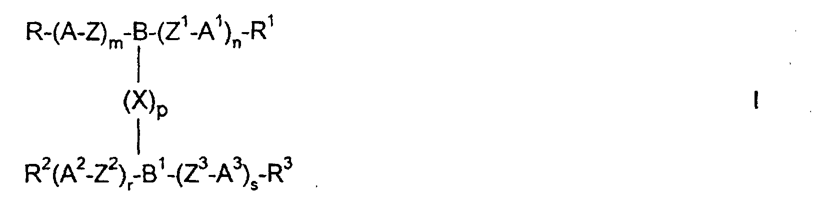 worin
- B und B¹: unabhängig voneinander 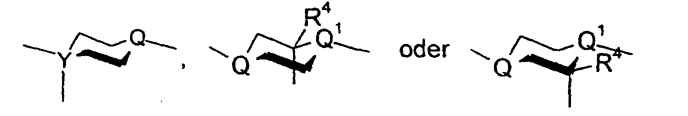
bedeuten,
wobei die Ringe B und B¹ jeweils über die axiale Bindung an die Gruppe (X)ₚ gebunden sind und in diesen Ringen auch ein oder zwei nicht benachbarte CH₂-Gruppen durch O ersetzt sein können,
- Y: bei mehrmaligem Auftreten unabhängig voneinander C oder Si,
- Q, Q¹: unabhängig voneinander CH oder SiH
- X: bei mehrmaligem Auftreten unabhängig voneinander -C≡C-, -CH=CH-, -CF=CF-, -CF=CH-, -CH₂-, -COO- oder 1,4-Phenylen, worin auch eine oder mehrere CH-Gruppen anabhängig voneinander durch N oder CF ersetzt sein können, jedoch mit der Maßgabe, dass mindestens ein X-C≡C- ist,
- p: 1, 2, 3 oder 4
- R, R¹, R², R³, R⁴: unabhängig voneinander H, einen unsubstituierten, einen mindestens einfach durch Halogen substituierten Alkylrest mit 1-12 C-Atomen, worin auch eine oder mehrere CH₂-Gruppen jeweils unabhängig voneinander durch -O-, -S-, -CO-, 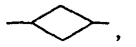 -CO-O-, -O-CO-, -O-CO-O oder -CH=CH- so ersetzt sein können, daß Heteroatome nicht direkt verbunden sind, CN, F, -CF₃, -OCHF₂, -OCF₃, -OCHFCF₃, -OCH₂CF₃, -OCF₂CF₃ oder -CH=CF₂ bedeutet,
- A, A¹, A² und A³: unabhängig voneinander 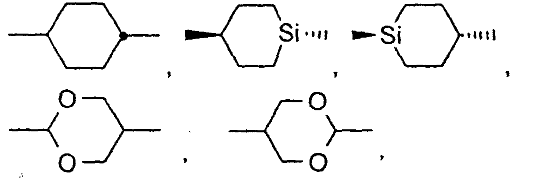 oder einen 1,4-Phenylenrest, worin auch eine oder zwei CH-Gruppen durch N ersetzt sein können, wobei die Ringe A, A¹, A² und A³ unabhängig voneinander durch eines oder mehrere F-Atome oder -NCS, -CN, CF₃ oder OCF₃ substituiert sein können.
- Z, Z¹, Z², Z³: jeweils unabhängig voneinander -CO-O-, -O-CO-; -CH₂O-, -CH=CH-, -O-, -O-CH₂-, -CH₂CH₂-, -(CH₂)₃-, -(CH₂)₄-, -CF₂-CF₂-, -OCF₂- oder eine Einfachbindung
und
- n, m, r, s: unabhängig voneinander 0, 1 oder 2
bedeutet.

Die Erfindung betrifft außerdem die Verwendung der Verbindungen der Formel I als Komponenten flüssigkristalliner Medien sowie Flüssigkristall- und elektrooptische Anzeigeelemente, die die erfindungsgemäßen flüssigkristallinen Medien enthalten.

Die Verbindungen der Formel I weisen häufig einen geringen positiven oder negativen Wert der dielektrischen Anisotropie auf und können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (Electrically controlles birefringence) oder dem Effekt der dynamischen Streuung beruhen.

Die bisher für diesen Zweck eingesetzten Substanzen haben stets gewisse Nachteile, beispielsweise zu geringe Stabilität gegenüber der Einwirkung von Wärme, Licht oder elektrischen Feldern, ungünstige elastische und/oder dielektrische Eigenschaften.

Der Erfindung lag die Aufgabe zugrunde, neue stabile flüssigkristalline oder mesogene Verbindungen mit besonders kleiner optischer Anisotropie Δn und negativer oder positiver dielektrischen Anisotropie aufzufinden, die als Komponenten flüssigkristalliner Medien, insbesondere für TFT- und STN-Displays, geeignet sind.

Es wurde nun gefunden, daß die Verbindungen der Formel I vorzüglich als Komponenten flüssigkristalliner Medien geeignet sein. Mit ihrer Hilfe lassen sich stabile flüssigkristalline Medien, insbesondere geeignet für TFT- oder STN-Displays, erhalten. Die neuen Verbindungen zeichnen sich vor allem durch eine hohe thermische Stabilität aus, die für eine hohe "holding ratio" vorteilhaft ist und zeigen günstige Werte der Klärpunkte. Die Verbindungen der Formel I weisen bei einer reduzierten Temperatur von 0.9 und einer Wellenlänge von 589 nm einen Wert für die optische Anisotropie Δn von vorzugsweise < 0,05 insbesondere bevorzugt von < 0.03 auf, der auf einen besonders hohen Wert von n_{⊥} zurückgeht. Hierbei ist die reduzierte Temperatur wie folgt definiert:$\frac{\text{Meßtemperatur in K}}{\text{Klärpunktstemperatur in K}} \text{= reduzierte Temperatur}$

Kleine Werte des Δn werden erfindungsgemäß dadurch erreicht, daß die Gruppe (X)ₚ durch ihre axiale Position senkrecht zur Moleküllängsachse steht. Aus der somit erhöhten Polarisierbarkeit quer zu dieser Achse resultiert ein erhöhtes n_{⊥}. Mit Δn = n_{∥}-n_{⊥} (z.B. L. Pohl, U. Finkenzeller in Liquid Crystals Vol. 1, 152, Ed. B. Bahadur, World Scientific, 1990) ergeben sich somit kleine Werte der optischen Anisotropie.

Flüssigkristalline Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt.

Mit der Bereitstellung von Verbindungen der Formel I wird ganz allgemein die Palette der flüssigkristallinen Substanzen, die sich unter verschiedenen anwendungstechnischen Gesichtspunkten zur Herstellung flüssigkristalliner Gemische eignen, erheblich verbreitert.

Die Verbindungen der Formel I besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch Verbindungen der Formel flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren. Durch Zusatz der Verbindungen der Formel I zu flüssigkristallinen Dielektrika lassen sich Δn-Werte solcher Medien deutlich reduzieren.

Die Bedeutung der Formel schließt alle Isotope der in den Verbindungen der Formel I gebundenen chemischen Elemente ein. In enantiomerenreiner oder -angereicherter Form eignen sich die Verbindungen der Formel I auch als chirale Dotierstoffe und generell zur Erzielung chiraler Mesophasen.

Die Verbindungen der Formel I sind in reinem Zustand farblos und bilden flüssigkristalline Mesophasen in einem für die elektrooptische Verwendung günstig gelegenen Temperaturbereich. Chemisch, thermisch und gegen Licht sind sie stabil.

Bei konventionellen calamitischen Flüssigkristallen steigt n_{II} mit abnehmender Wellenlänge des Lichtes stärker als n_{⊥}, so daß der Wert von Δn zunimmt, da gilt:${\text{Δn = n}}_{\text{II}} {\text{- n}}_{\text{⊥}} \text{.}$

Im Gegensatz zu diesen herkömmlichen Materialien weisen die erfindungsgemäßen Verbindungen eine ungewöhnliche Frequenzabhängigkeit von n_{II} und n_{⊥} auf: Bei abnehmender Wellenlänge nimmt bei den Verbindungen der Formel I der Wert von n_{⊥} stärker zu als der Wert von n_{II}. Somit ergibt sich eine Verringerung des Wertes für Δn beim Übergang zu kürzeren Wellenlängen.

Bevorzugte Verbindungen der Formel I weisen sehr geringe Werte des Δn unter den gewöhnlichen Meßbedingungen auf (20 °C, 598 nm). Bei bestimmten Wellenlängen weisen n_{II} und n_{⊥} dieser Verbindungen denselben Wert auf (Δn = 0, siehe Beispiel 266). Bei noch kürzeren Wellenlängen ist n_{⊥} dann größer als n_{II}, und Δn nimmt einen negativen Wert an. Somit weisen die erfindungsgemäßen Verbindungen in Abhängigkeit der Wellenlänge des verwendeten Lichtes eine Inversion des Δn auf.

Die erfindungsgemäßen Verbindungen sind daher bevorzugt für die Kompensation der Frequenzabhängigkeit des Δn herkömmlicher flüssigkristalliner Mischungen geeignet. Weiterhin können diese Verbindungen für die Herstellung von optischen Schaltern oder Filtern verwendet werden, die nur bestimmte Wellenlängen des einfallenden Lichtes ausblenden.

Die Verbindungen der Formel I können durch erhöhte Temperatur oder Anwendung von UV-Licht in flüssigkristalline Polymere übergeführt werden.

Gegenstand der Erfindung sind somit die Verbindungen der Formel I sowie die Verwendung dieser Verbindungen als Komponenten flüssigkristalliner Medien. Gegenstand der Erfindung sind ferner flüssigkristalline Medien mit einem Gehalt an mindestens einer Verbindung der Formel I sowie Flüssigkristallanzeigeelemente, optische Schalter oder Filter, insbesondere elektrooptische Anzeigeelemente, die derartige Medien enthalten.

Vor- und nachstehend haben B, B¹, R, R¹, R², R³, R⁴, A, A¹, A², A³, X, p, Z, Z¹, Z², Z³, Q, Q¹, Y, n, m, r und s die angegebene Bedeutung, sofern nicht ausdrücklich etwas anderes vermerkt ist. Kommt der Rest X mehrfach vor, so kann er gleiche oder verschiedene Bedeutungen annehmen. Dasselbe gilt für alle anderen mehrfach auftretenden Gruppen.

Der Einfachheit halber bedeuten im folgenden Cyc einen Cyclohexan-1,4-diylrest oder einen 1- oder 4-Silacyclohexan-1,4-diylrest und Dio einen 1,3-Dioxan-2,5-diylrest wobei Cyc unsubstituiert oder vorzugsweise ein- oder mehrfach durch F oder CN substituiert sein kann.

In der Formel I nehmen die Gruppen R-(A-Z)ₘ-B-(Z¹-A¹)ₙ-R¹ und R²-(A²-Z²)ᵣ-B¹-(Z³-A³)ₛ-R³ bevorzugt eine der Bedeutungen der Teilformeln Ia1 bis Ia12 an, die neben der Gruppe W, die die Bedeutung B oder B¹ aufweist, einen sechsgliedrigen Ring enthalten:

R⁵-W-Cyc-R⁶ Ia1

R⁵-W-CH₂CH₂-Cyc-R⁶ Ia2

R⁵-W-COO-Cyc-R⁶ Ia3

R⁵-W-Dio-R⁶ Ia4

R⁵-W-CH₂CH₂-Dio-R⁶ Ia5

R⁵-W-COO-Dio-R⁶ Ia6

R⁵-Cyc-W-R⁶ Ia7

R⁵-Dio-W-R⁶ Ia8

R⁵-Cyc-CH₂CH₂-W-R⁶ Ia9

R⁵-Dio-CH₂CH₂-W-R⁶ Ia10

R⁵-Cyc-COO-W-R⁶ Ia11

R⁵-Dio-COO-W-R⁶ Ia12

ferner die ebenfalls bevorzugten Verbindungen der Teilformeln Ib1 bis Ib72, die zusätzlich zur Gruppe W zwei sechsgliedrige Ringe enthalten:

R⁵-Cyc-Cyc-W-R⁶ Ib1

R⁵-Dio-Cyc-W-R⁶ Ib2

R⁵-Cyc-CH=CH-Cyc-W-R⁶ Ib3

R⁵-Dio-CH₂CH₂-Cyc-W-R⁶ Ib4

R⁵-Cyc-COO-Cyc-W-R⁶ Ib5

R⁵-Dio-COO-Cyc-W-R⁶ Ib6

R⁵-Cyc-Dio-W-R⁶ Ib7

R⁵-Dio-Dio-W-R⁶ Ib8

R⁵-Cyc-CH₂CH₂-Dio-W-R⁶ Ib9

R⁵-Dio-CH₂CH₂-Dio-W-R⁶ Ib10

R⁵-Cyc-COO-Dio-W-R⁶ Ib11

R⁵-Dio-COO-Dio-W-R⁶ Ib12

R⁵-Cyc-Cyc-CH₂CH₂-W-R⁶ Ib13

R⁵-Dio-Cyc-CH₂CH₂-W-R⁶ Ib14

R⁵-Cyc-Dio-CH₂CH₂-W-R⁶ Ib15

R⁵-Dio-Dio-CH₂CH₂-W-R⁶ Ib16

R⁵-Cyc-Cyc-COO-W-R⁶ Ib17

R⁵-Dio-Cyc-COO-W-R⁶ Ib18

R⁵-Cyc-Dio-COO-W-R⁶ Ib19

R⁵-Dio-Dio-COO-W-R⁶ Ib20

R⁵-Cyc-W-Cyc-R⁶ Ib21

R⁵-Dio-W-Cyc-R⁶ Ib22

R⁵-Cyc-CH₂CH₂-W-Cyc-R⁶ Ib23

R⁵-Dio-CH₂CH₂-W-Cyc-R⁶ Ib24

R⁵-Cyc-CH=CH-W-Cyc-R⁶ Ib25

R⁵-Dio-COO-W-Cyc-R⁶ Ib26

R⁵-Cyc-W-CH₂CH₂-Cyc-R⁶ Ib27

R⁵-Dio-W-CH₂CH₂-Cyc-R⁶ Ib28

R⁵-Cyc-W-COO-Cyc-R⁶ Ib29

R⁵-Dio-W-COO-Cyc-R⁶ Ib30

R⁵-Cyc-W-Dio-R⁶ Ib31

R⁵-Dio-W-Dio-R⁶ Ib32

R⁵-Cyc-CH₂CH₂-W-Dio-R⁶ Ib33

R⁵-Dio-CH₂CH₂ W-Dio-R⁶ Ib34

R⁵-Cyc-COO-W-Dio-R⁶ Ib35

R⁵-Dio-COO-W-Dio-R⁶ Ib36

R⁵-Cyc-W-CH₂CH₂-Dio-R⁶ Ib37

R⁵-Dio-W-CH₂CH₂-Dio-R⁶ Ib38

R⁵-Cyc-W-COO-Dio-R⁶ Ib39

R⁵-Dio-W-COO-Dio-R⁶ Ib40

R⁵-W-Cyc-Cyc-R⁶ Ib41

R⁵-W-CH₂CH₂-Cyc-Cyc-R⁶ Ib42

R⁵-W-COO-Cyc-Cyc-R⁶ Ib43

R⁵-W-Dio-Cyc-R⁶ Ib44

R⁵-W-CH₂CH₂-Dio-Cyc-R⁶ Ib45

R⁵-W-COO-Dio-Cyc-R⁶ Ib46

R⁵-W-Cyc-CH₂CH₂-Cyc-R⁶ Ib47

R⁵-W-Dio-CH₂CH₂-Cyc-R⁶ Ib48

R⁵-W-Cyc-COO-Cyc-R⁶ Ib49

R⁵-W-Dio-COO-Cyc-R⁶ Ib50

R⁵-W-Cyc-Dio-R⁶ Ib51

R⁵-W-CH₂CH₂-Cyc-Dio-R⁶ Ib52

R⁵-W-COO-Cyc-Dio-R⁶ Ib53

R⁵-W-Dio-Dio-R⁶ Ib54

R⁵-W-CH₂CH₂-Dio-Dio-R⁶ Ib55

R⁵-W-CH₂CH₂-Dio-Dio-R⁶ Ib56

R⁵-W-Cyc-CH₂CH₂-Dio-R⁶ Ib57

R⁵-W-Dio-CH₂CH₂-Dio-R⁶ Ib58

R⁵-W-Cyc-COO-Dio-R⁶ Ib59

R⁵-W-Dio-COO-Dio-R⁶ Ib60

R⁵-Cyc-CH₂CH₂-W-CH₂CH₂-Cyc-R⁶ Ib61

R⁵-Dio-CH₂CH₂-W-CH₂CH₂-Cyc-R⁶ Ib62

R⁵-Cyc-CH₂CH₂-W-CH₂CH₂-Dio-R⁶ Ib63

R⁵-Dio-CH₂CH₂-W-CH₂CH₂-Dio-R⁶ Ib64

R⁵-Cyc-CH₂CH₂-Cyc-CH₂CH₂-W-R⁶ Ib65

R⁵-Dio-CH₂CH₂-Cyc-CH₂CH₂-W-R⁶ Ib66

R⁵-Cyc-CH₂CH₂-Dio-CH₂CH₂-W-R⁶ Ib67

R⁵-Dio-CH₂CH₂-Dio-CH₂CH₂-W-R⁶ Ib68

R⁵-W-CH₂CH₂-Cyc-CH₂CH₂-Cyc-R⁶ Ib69

R⁵-W-CH₂CH₂-Dio-CH₂CH₂-Cyc-R⁶ Ib70

R⁵-W-CH₂CH₂-Cyc-CH₂CH₂-Dio-R⁶ Ib71

R⁵-W-CH₂CH₂-Dio-CH₂CH₂-Dio-R⁶ Ib72

sowie die bevorzugten Verbindungen der Teilformeln Ic1 bis Ic55, die neben der Gruppe W drei sechsgliedrige Ringe enthalten:

R⁵-W-Cyc-Cyc-Cyc-R⁶ Ic1

R⁵-W-CH₂CH₂-Cyc-Cyc-Cyc-R⁶ Ic2

R⁵-W-Dio-Cyc-Cyc-R⁶ Ic3

R⁵-W-CH₂CH₂-Dio-Cyc-Cyc-R⁶ Ic4

R⁵-W-Cyc-CH₂CH₂-Cyc-Cyc-R⁶ Ic5

R⁵-W-Dio-CH₂CH₂-Cyc-Cyc-R⁶ Ic6

R⁵-W-Cyc-Cyc-CH₂CH₂-Cyc-R⁶ Ic7

R⁵-W-Dio-Cyc-CH₂CH₂-Cyc-R⁶ Ic8

R⁵-W-Cyc-Dio-Cyc-R⁶ Ic9

R⁵-W-CH₂CH₂-Cyc-Dio-Cyc-R⁶ Ic10

R⁵-W-Dio-Dio-Cyc-R⁶ Ic11

R⁵-W-CH₂CH₂-Dio-Dio-Cyc-R⁶ Ic12

R⁵-W-Cyc-CH₂CH₂-Dio-Cyc-R⁶ Ic13

R⁵-W-Dio-CH₂CH₂-Dio-Cyc-R⁶ Ic14

R⁵-W-Cyc-Dio-CH₂CH₂-Cyc-R⁶ Ic15

R⁵-Cyc-Dio-CH₂CH₂-Cyc-W-R⁶ Ic16

R⁵-Dio-Dio-CH₂CH₂-Cyc-W-R⁶ Ic17

R⁵-Cyc-Cyc-Cyc-CH₂CH₂-W-R⁶ Ic18

R⁵-Dio-Cyc-Cyc-CH₂CH₂-W-R⁶ Ic19

R⁵-Cyc-Dio-Cyc-CH₂CH₂-W-R⁶ Ic20

R⁵-Dio-Dio-Cyc-CH₂CH₂-W-R⁶ Ic21

R⁵-Cyc-Cyc-Dio-W-R⁶ Ic22

R⁵-Dio-Cyc-Dio-W-R⁶ Ic23

R⁵-Cyc-CH₂CH₂-Cyc-Dio-W-R⁶ Ic24

R⁵-Dio-CH₂CH₂-Cyc-Dio-W-R⁶ Ic25

R⁵-Cyc-Dio-Dio-W-R⁶ Ic26

R⁵-Dio-Dio-Dio-W-R⁶ Ic27

R⁵-Cyc-CH₂CH₂-Dio-Dio-W-R⁶ Ic28

R⁵-Dio-CH₂CH₂-Dio-Dio-W-R⁶ Ic29

R⁵-Cyc-Cyc-CH₂CH₂-Dio-W-R⁶ Ic30

R⁵-Dio-Cyc-CH₂CH₂-Dio-W-R⁶ Ic31

R⁵-Cyc-CH₂CH₂-Dio-W-Dio-R⁶ Ic32

R⁵-Dio-CH₂CH₂-Dio-W-Dio-R⁶ Ic33

R⁵-Cyc-Cyc-CH₂CH₂-W-Dio-R⁶ Ic34

R⁵-Dio-Cyc-CH₂CH₂-W-Dio-R⁶ Ic35

R⁵-Cyc-Dio-CH₂CH₂-W-Dio-R⁶ Ic36

R⁵-Dio-Dio-CH₂CH₂-W-Dio-R⁶ Ic37

R⁵-Cyc-Cyc-W-CH₂CH₂-Dio-R⁶ Ic38

R⁵-Dio-Cyc-W-CH₂CH₂-Dio-R⁶ Ic39

R⁵-Cyc-Dio-W-CH₂CH₂-Dio-R⁶ Ic40

R⁵-Dio-Dio-W-CH=CH-Dio-R⁶ Ic41

R⁵-Cyc-W-Dio-CH₂CH₂-Cyc-R⁶ Ic42

R⁵-Dio-W-Dio-CH₂CH₂-Cyc-R⁶ Ic43

R⁵-Cyc-W-Cyc-Dio-R⁶ Ic44

R⁵-Dio-W-Cyc-Dio-R⁶ Ic45

R⁵-Cyc-CH₂CH₂-W-Cyc-Dio-R⁶ Ic46

R⁵-Dio-CH₂CH₂-W-Cyc-Dio-R⁶ Ic47

R⁵-Cyc-W-CH₂CH₂-Cyc-Dio-R⁶ Ic48

R⁵-Dio-W-CH₂CH₂-Cyc-Dio-R⁶ Ic49

R⁵-Cyc-W-Cyc-CH₂CH₂-Dio-R⁶ Ic50

R⁵-Dio-W-Cyc-CH₂CH₂-Dio-R⁶ Ic51

R⁵-Cyc-W-Dio-Dio-R⁶ Ic52

R⁵-Dio-W-Dio-Dio-R⁶ Ic53

R⁵-Cyc-CH₂CH₂-W-Dio-Dio-R⁶ Ic54

R⁵-Dio-CH₂CH₂-W-Dio-Dio-R⁶ Ic55

worin Cyc und Dio die oben angegebene Bedeutung aufweisen, R⁵ und R⁶ unabhängig voneinander die Bedeutung von R, R¹, R² und R³ annehmen und W B oder B¹ bedeutet.

B und B¹ bedeuten unabhängig voneinander bevorzugt

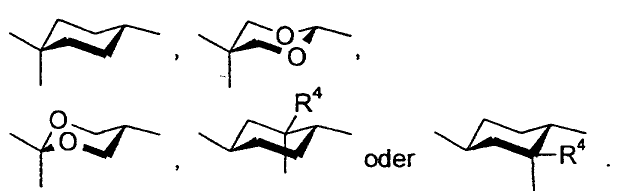

Y bedeutet bevorzugt C.

Q und Q¹ bedeuten bevorzugt CH.

(X)ₚ nimmt vorzugsweise eine der folgenden Bedeutungen an:
-C≡C-, -C≡C-C≡C, -C≡C-C≡C-C≡C-, -C≡C-C≡C-C≡C-C≡C-, -CH=CH-, -CH=CH-CH=CH-, -C≡C-CH=CH-, -C≡C-CH=CH-C≡C-, -CH=CH-C≡C-CH=CH-, -C≡C-COO-, -CH=CH-COO-, -CH₂-CH₂-C≡C-, -CH₂-CH₂-C≡C-C≡C-, -CH₂-CH₂-C≡C-CH=CH-, 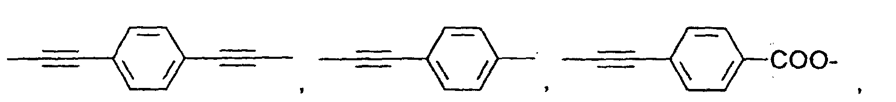 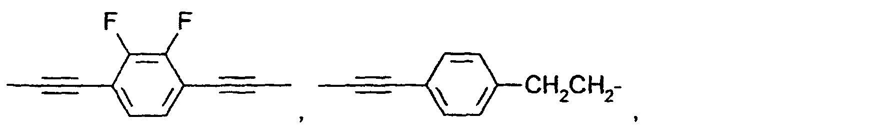 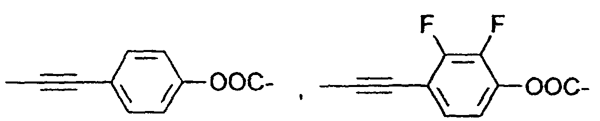 oder

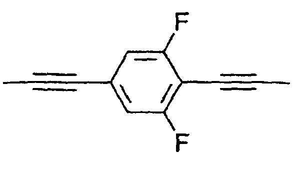

Insbesondere bevorzugt nimmt die Gruppe (X)ₚ eine der folgenden Bedeutungen an:
-C≡C-C≡C, -C≡C-C≡C-C≡C-, -C≡C-C≡C-C≡C-C≡C-, -C≡C-CH=CH-, -CH=CH-C≡C-CH=CH, -C≡C-COO-, -CH₂-CH₂-C≡C-C≡C-, -C≡C-CH=CH-C≡C-,

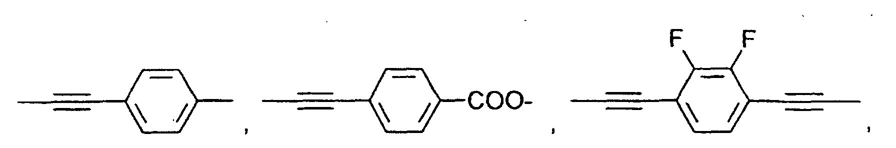

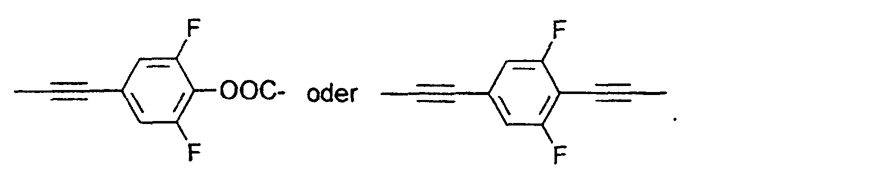

R, R¹, R², R³ und R⁴ bedeuten bevorzugt -CN, F, OCF₃, CF₃, OCF₂CF₃, -CH=CF₂ Alkyl oder Alkoxy mit 1 bis 10 C-Atomen, Alkenyl oder Alkenyloxy mit 2 bis 10 C-Atomen, insbesondere F, CF₃, OCF₃, -CH=CF₂, Alkyl oder Alkoxy mit 1 bis 7 C-Atomen.

Bevorzugt sind Verbindungen der Formel I sowie aller Teilformeln, in denen A, A¹, A² und/oder A³ ein- oder zweifach durch F oder CN substituiertes Cyclohexan-1,4-diyl bedeutet.

R⁴ bedeutet vorzugsweise H, CH₃, F oder CF₃, insbesondere H.

Besonders bevorzugt bedeutet A, A¹, A² und/oder A³ unabhängig voneinander

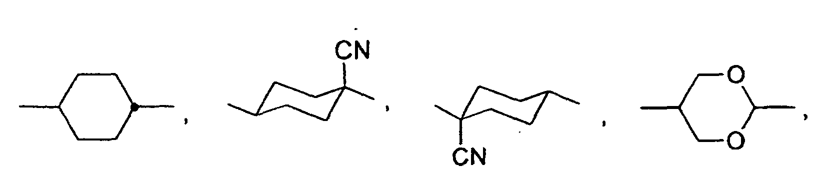

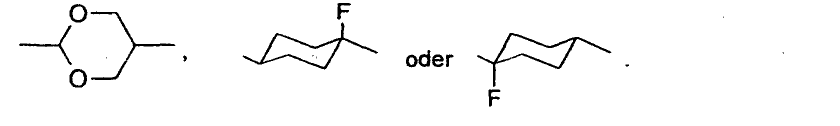

Z, Z¹, Z² und Z³ bedeuten unabhängig voneinander bevorzugt -CH₂CH₂-, -COO-, -OOC- oder eine Einfachbindung, insbesondere bevorzugt eine Einfachbindung oder -CH₂-CH₂-.

Verbindungen der Formel I sind bevorzugt, in denen R, R¹, R² und R³ gleichzeitig Alkyl oder Alkoxy mit 1 bis 10 C-Atomen bedeuten.

Verbindungen der Formel I, die nicht mehr als einen Dioxanring enthalten, stellen eine bevorzugte Ausführungsform der Erfindung dar.

m, n, r und s sind unabhängig voneinander bevorzugt 0 oder 1.

Die Verbindungen der Formel I umfassen die bevorzugten Verbindungen der Teilformeln I1 bis 122: 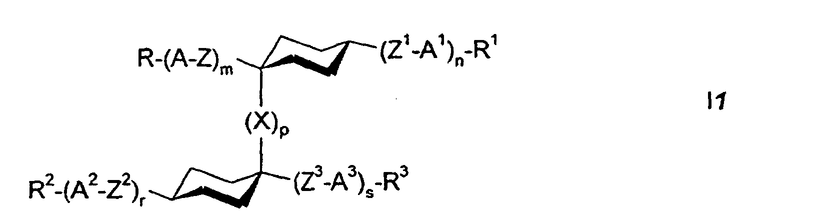 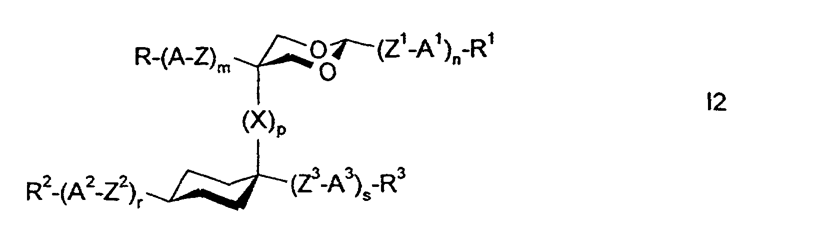 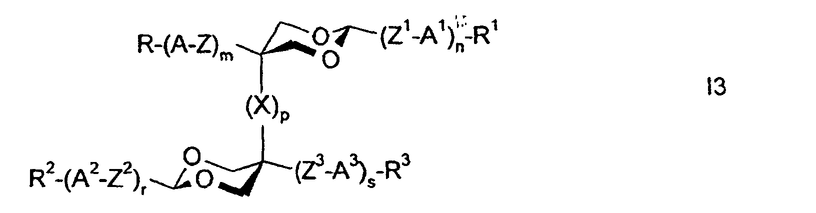 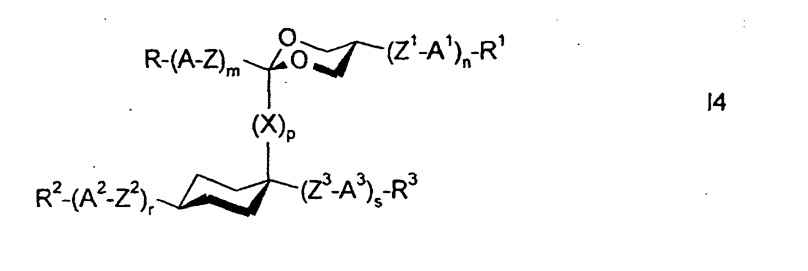 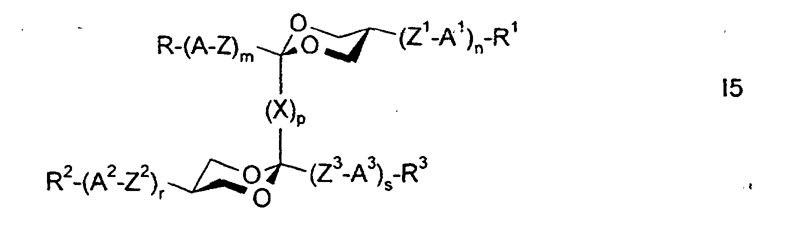 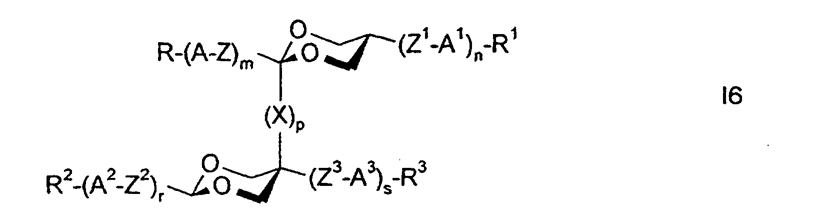 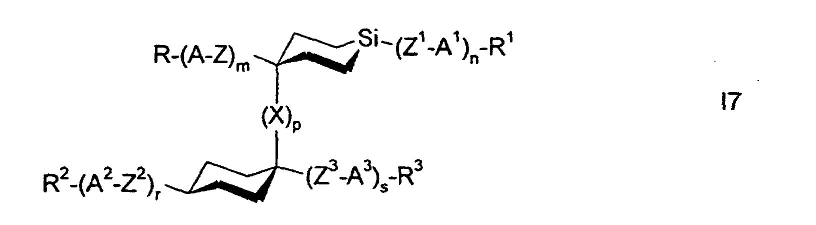 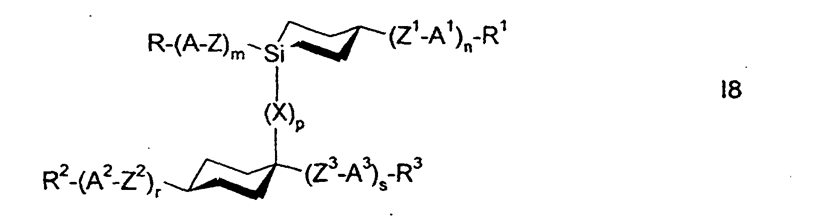 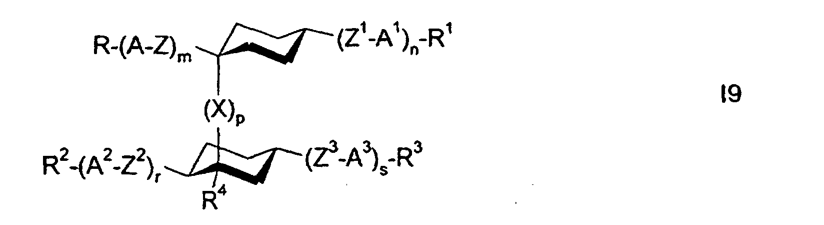 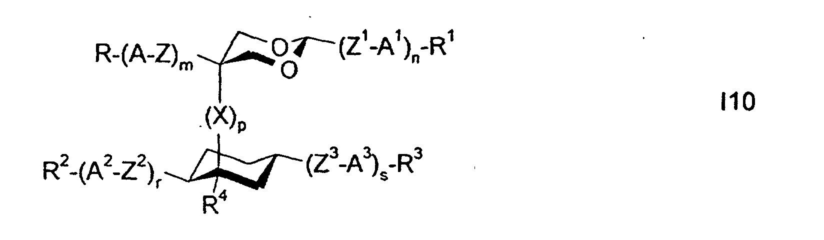 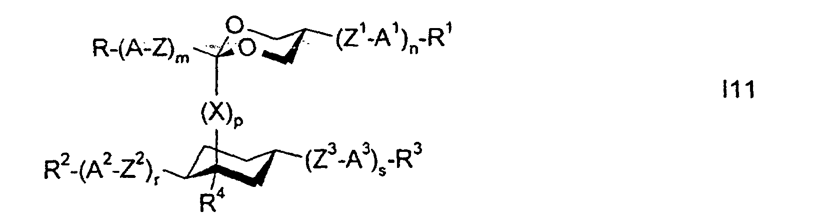 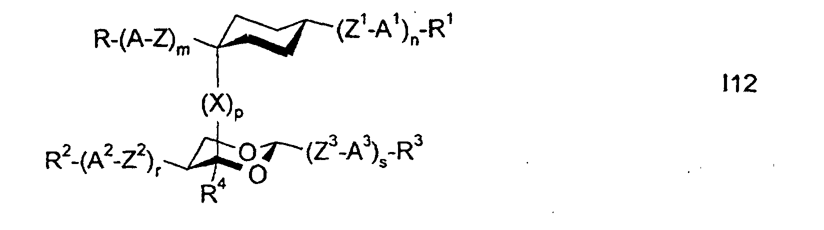 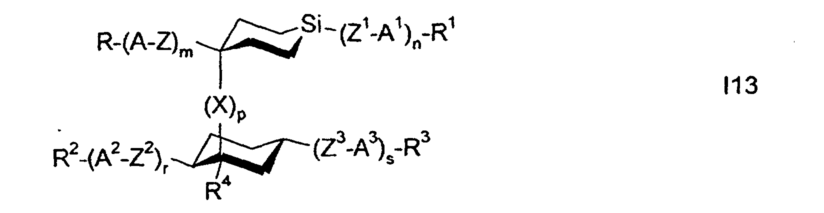 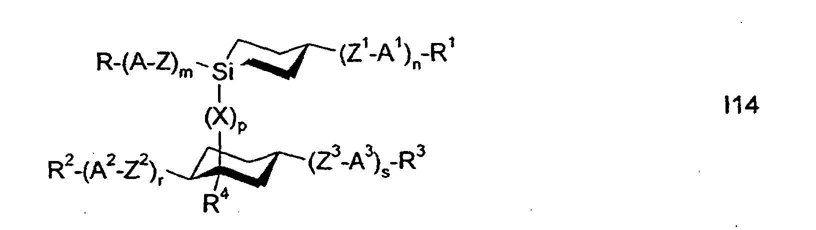 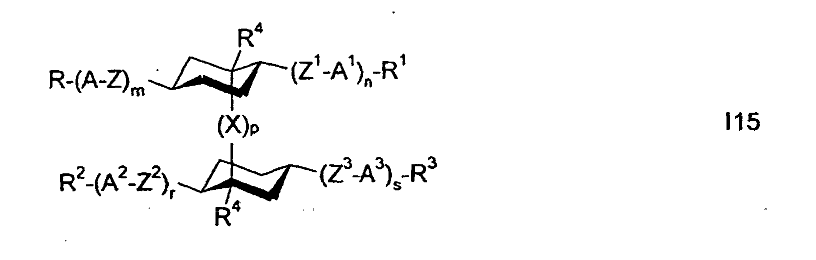 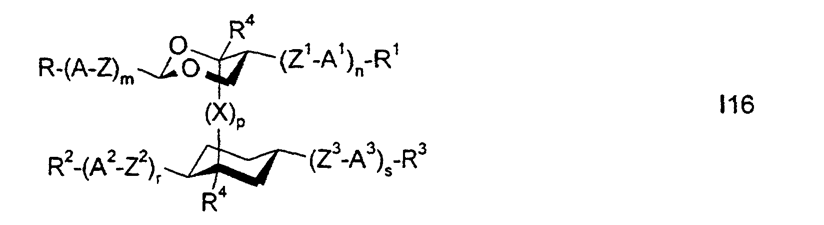 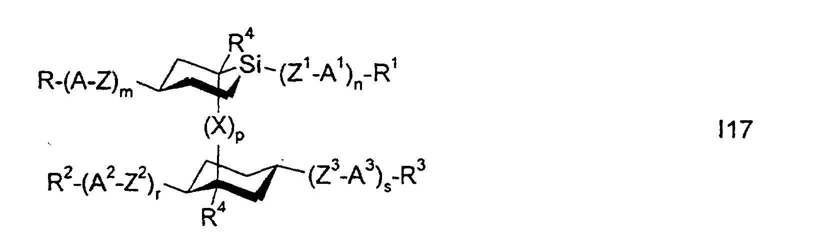 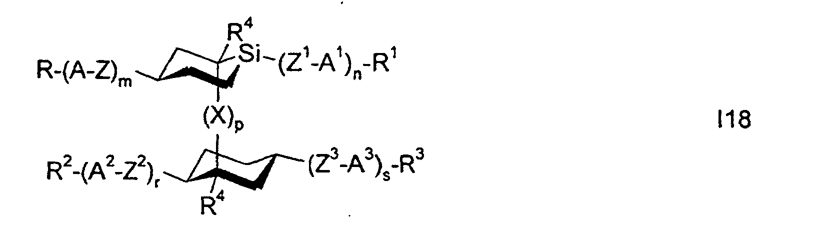 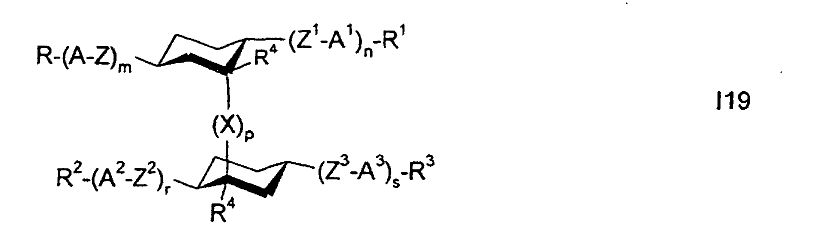 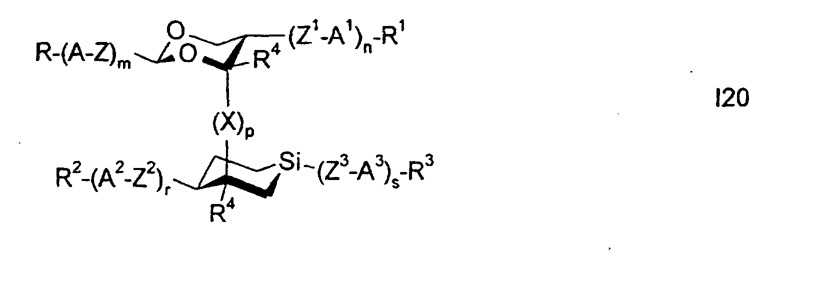 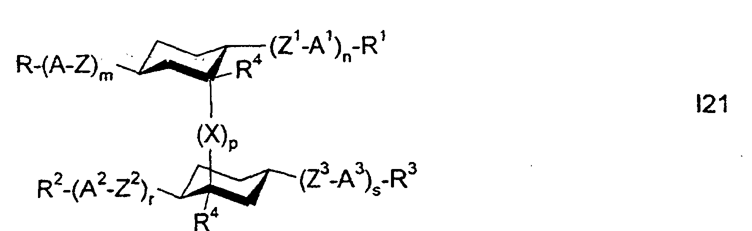 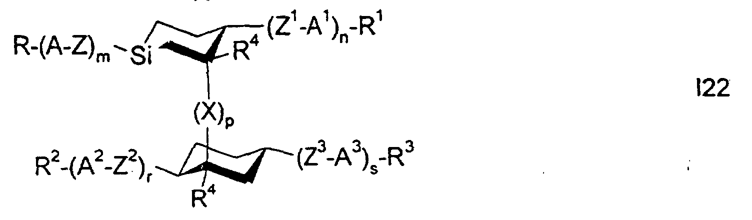 worin R, R¹, R², R³, R⁴, A, A¹, A², A³, Z, Z¹, Z², Z³, X, p, m, n, r und s die oben angegebene Bedeutung aufweisen.

Insbesondere die Verbindungen der Formeln I1 bis I6 zeichnen sich dadurch aus, daß sie eine Inversion der optischen Anisotropie aufweisen und sich daher zur Herstellung optischer Schalter oder Filter eignen.

Falls R, R¹, R², R³ und/oder R⁴ in den vor- und nachstehenden Formeln einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentyloxy, Hexyloxy oder Heptyloxy, ferner Methyl, Octyl, Nonyl, Decyl, Undecyl, Dodecyl, Tridecyl, Tetradecyl, Pentadecyl, Methoxy, Octyloxy, Nonyloxy, Decyloxy, Undecyloxy, Dodecyloxy, Tridecyloxy oder Tetradecyloxy.

Oxaalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-,4-, 5-, 6-, 7- oder 8-Oxanonyl, 2-, 3-, 4-, 5-, 6-, 7-, 8- oder 9-Oxadecyl.

Falls R, R¹, R², R³ und/oder R⁴ einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Vorzugsweise sind diese geradkettig und haben 2 bis 6 C-Atome.

Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)butyl.

Falls R, R¹, R², R³ und/oder R⁴ einen mindestens einfach durch Halogen substituierten Alkylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigter Flügelgruppe R, R¹, R², R³ und/oder R⁴ können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R, R¹, R², R³ und/oder R⁴ sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy, 1-Methylheptyloxy.

Falls R, R¹, R², R³ und/oder R⁴ einen Alkenylrest bedeutet, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3-, oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6-, Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Besonders bevorzugt bedeutet R, R¹, R², R³ und/oder R⁴ einen Alkenylrest aus der folgenden Gruppe:

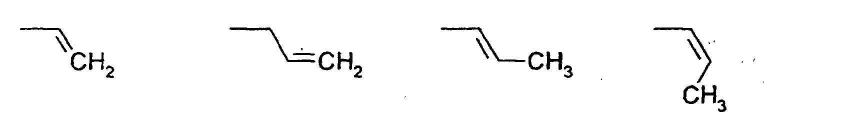

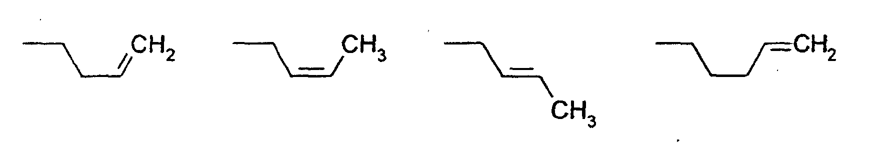

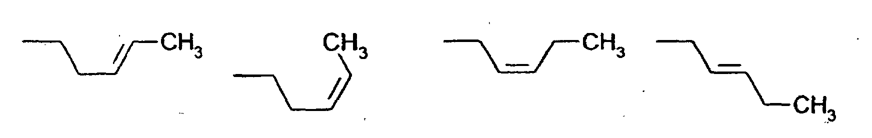

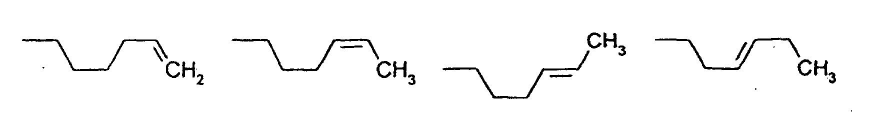

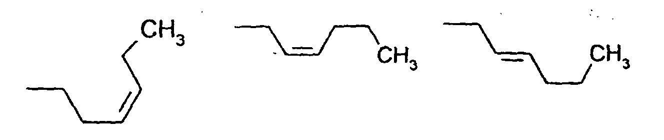

Falls R¹, R, R¹, R², R³ und/oder R⁴ einen Alkenyloxyrest bedeutet, so kann dieser geradkettig oder verzweigt sein. Vorzugsweise ist er geradkettig und hat 2 bis 10 C-Atome. Er bedeutet besonders bevorzugt einen Rest der folgenden Gruppe:

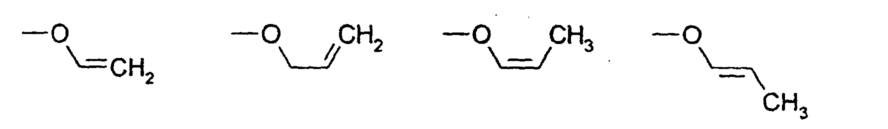

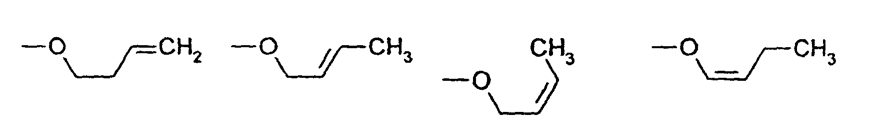

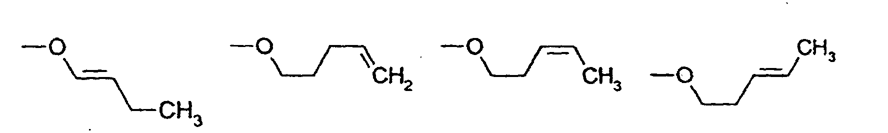

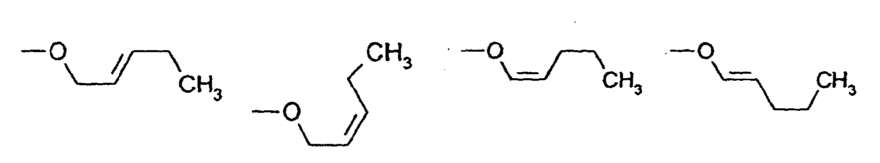

Formel I umfaßt sowohl die Racemate dieser Verbindungen als auch die optischen Antipoden sowie deren Gemische.

Unter diesen Verbindungen der Formel I sowie den Unformeln sind diejenigen bevorzugt, in denen mindestens einer der darin enthaltenen Reste eine der angegebenen bevorzugten Bedeutungen hat.

Einige ganz besonders bevorzugte kleinere Gruppen von Verbindungen der Formel I sind diejenigen der Teilformeln I23 bis I88: 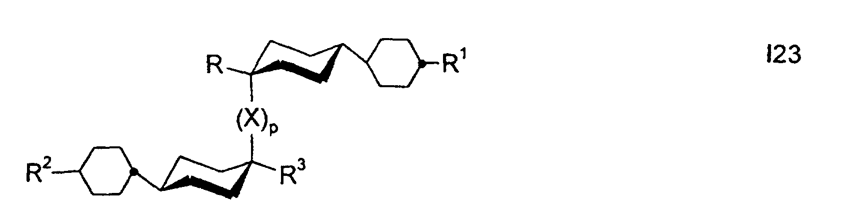 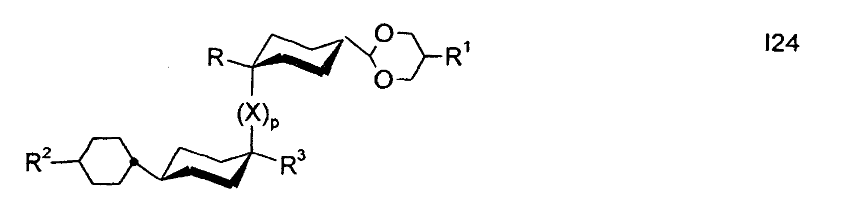 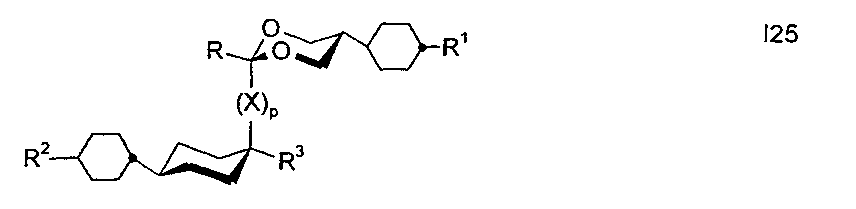 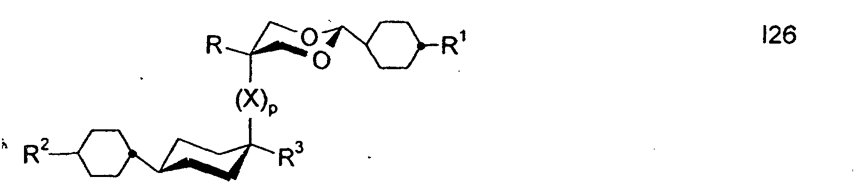 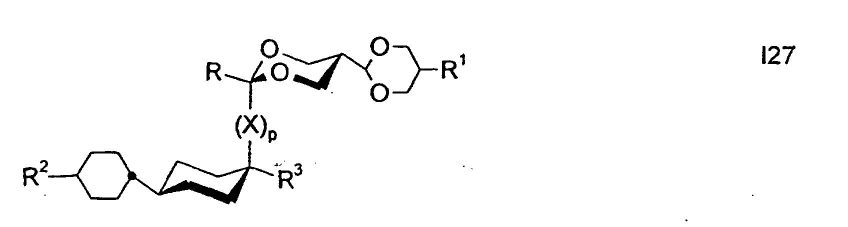 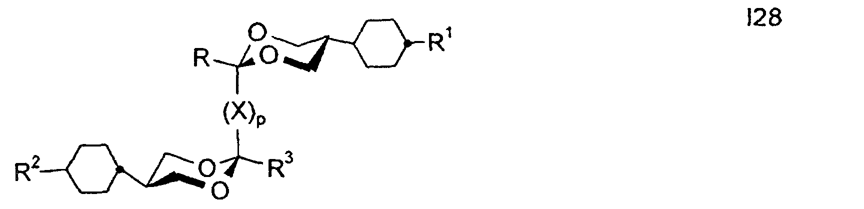 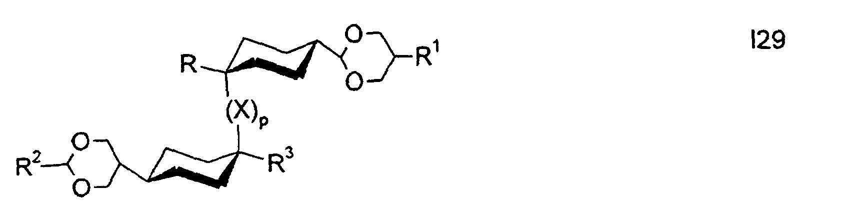 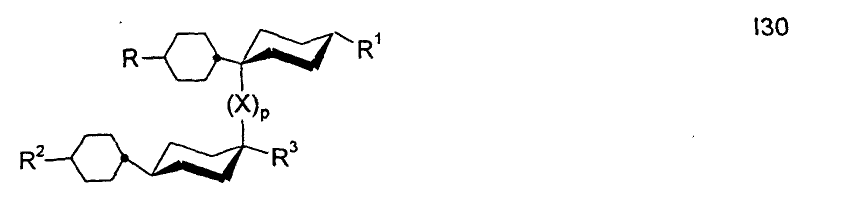 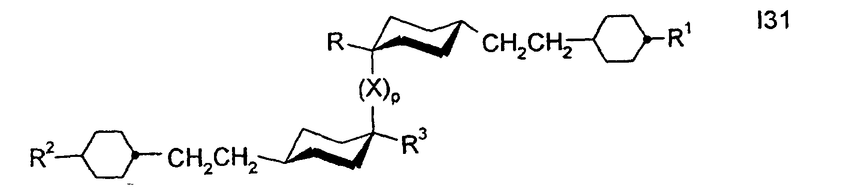 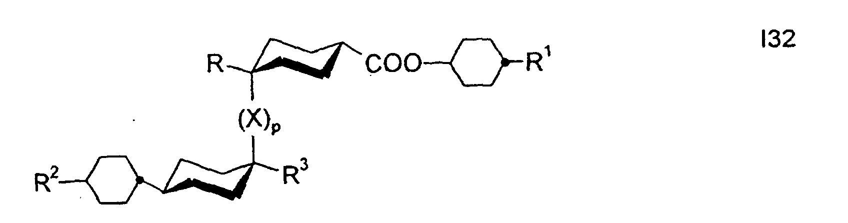 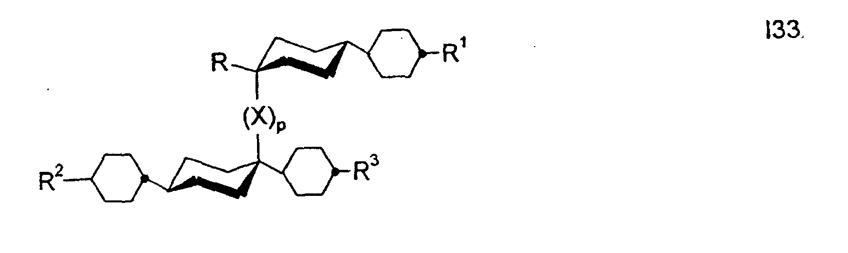 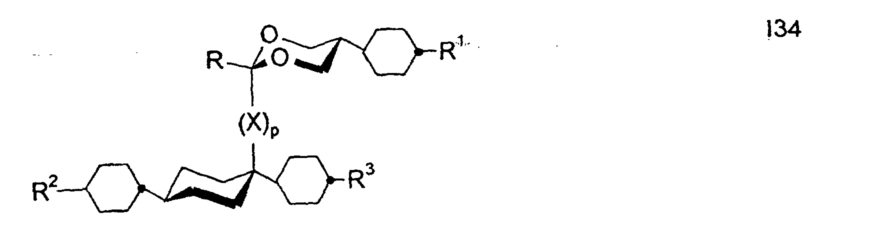 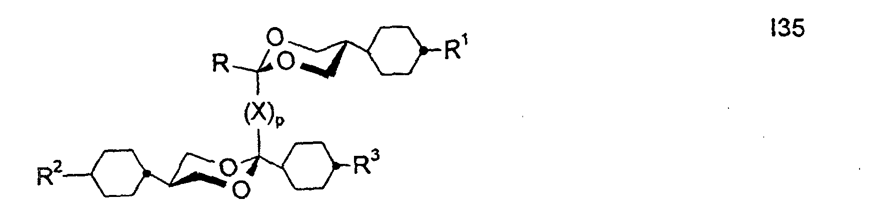 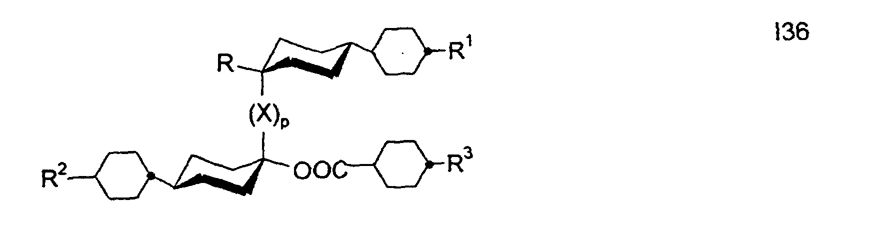 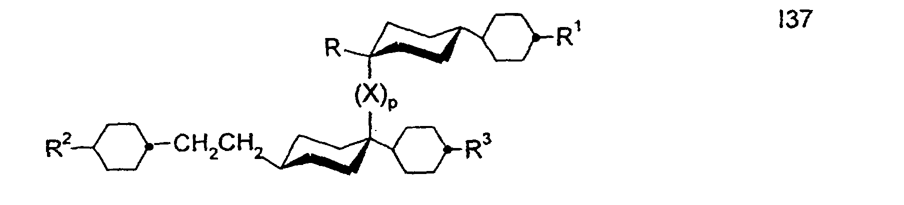 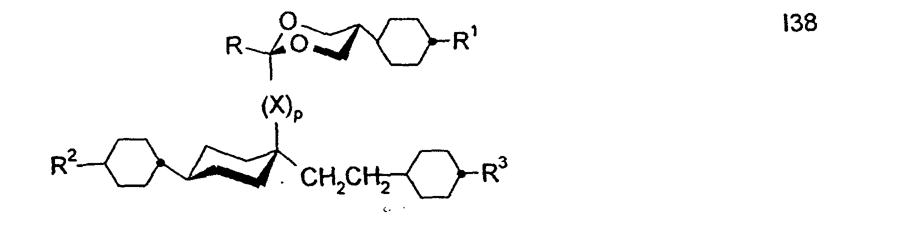 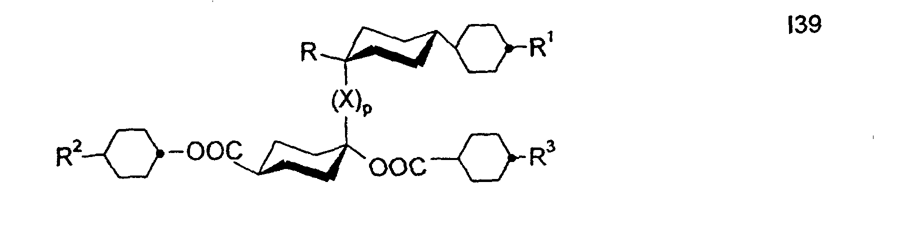 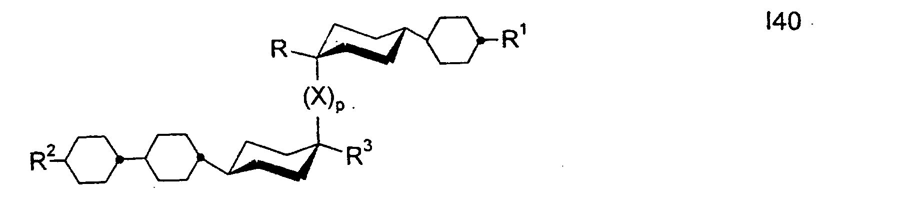 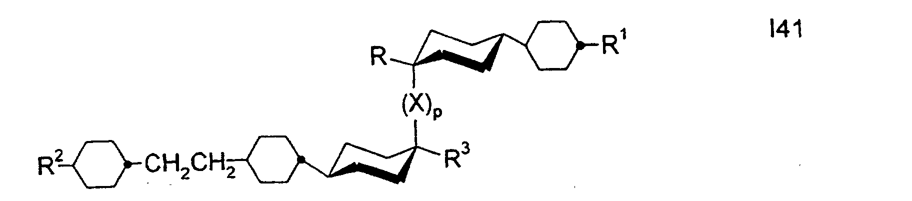 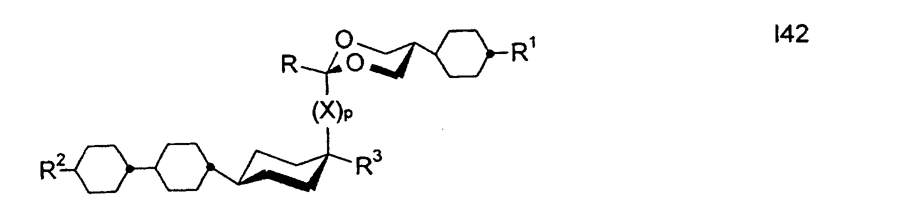 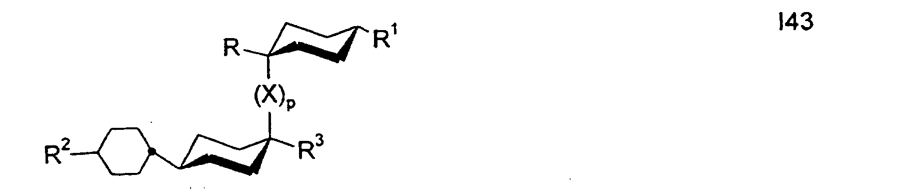 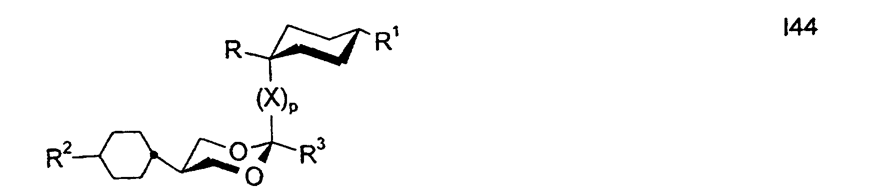 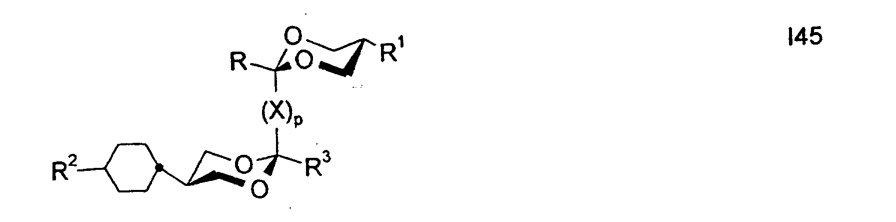 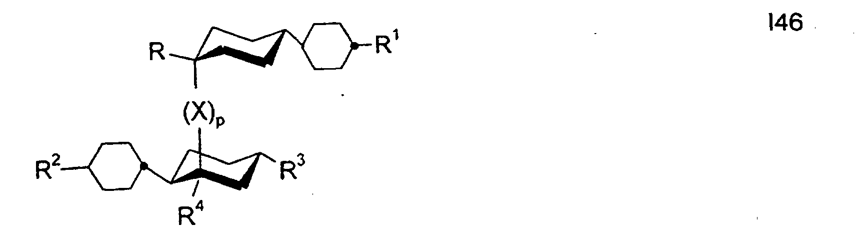 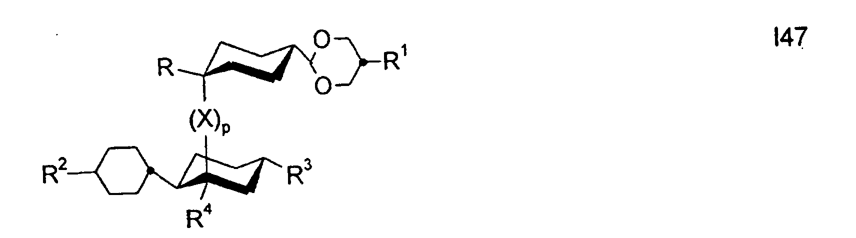 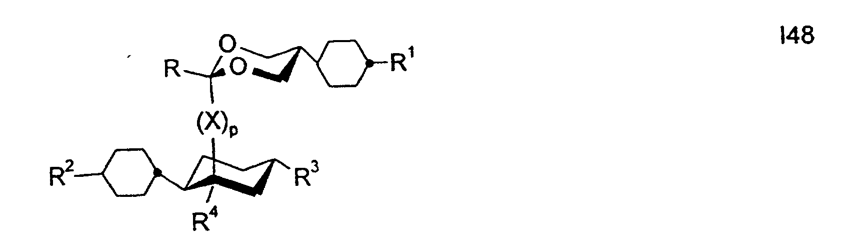 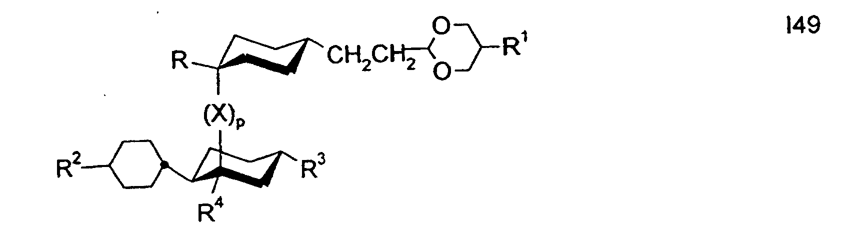 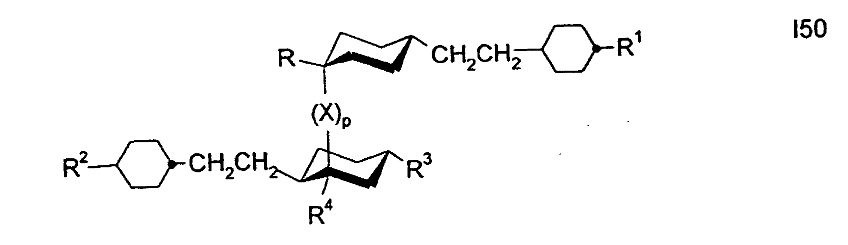 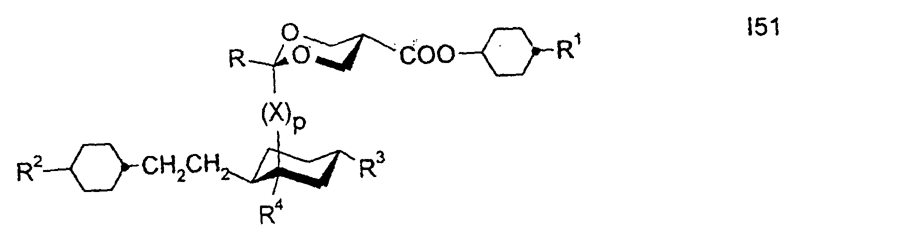 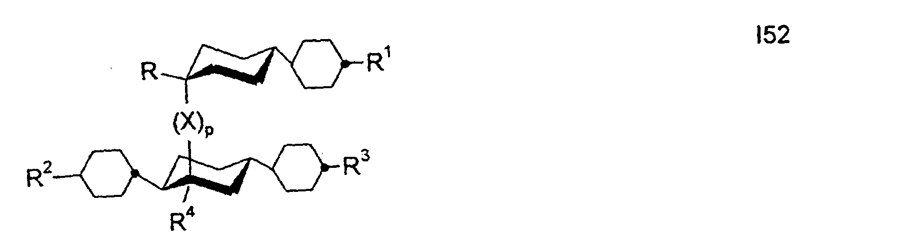 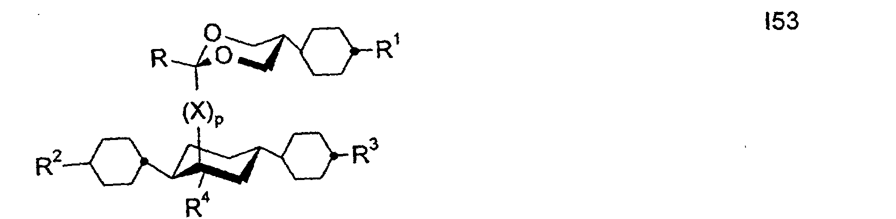 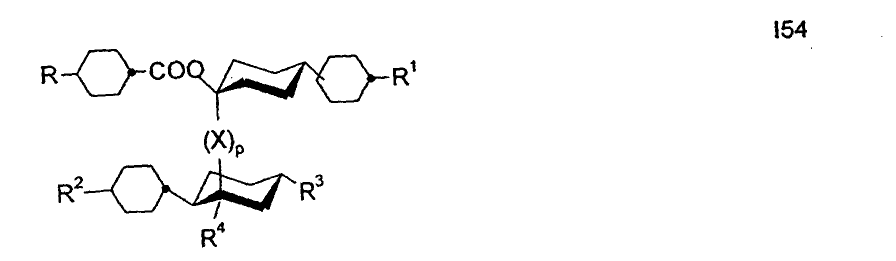 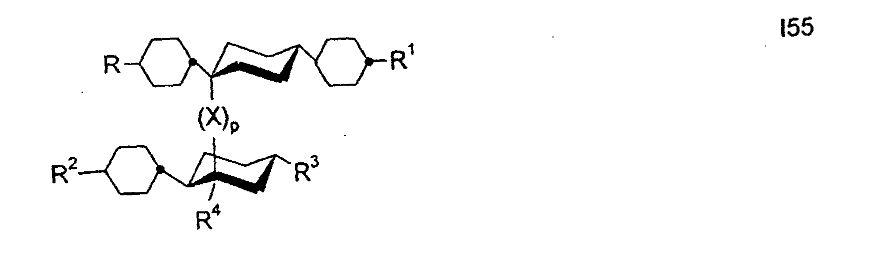 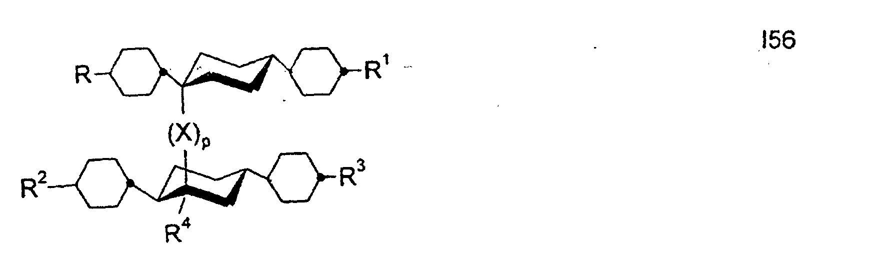 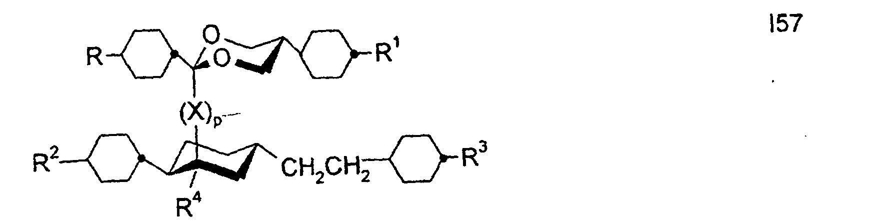 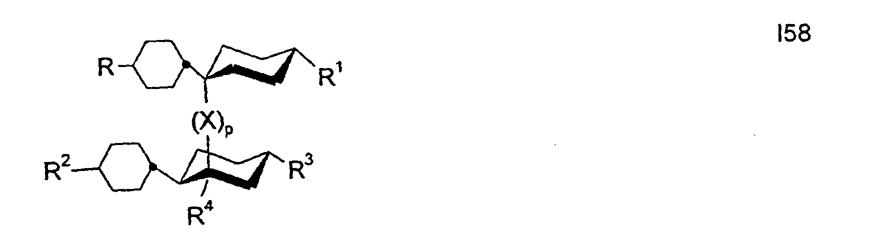 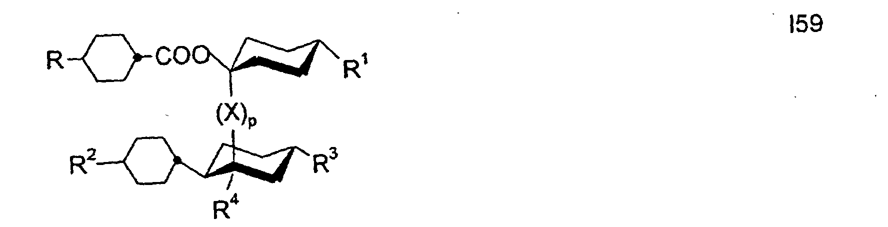 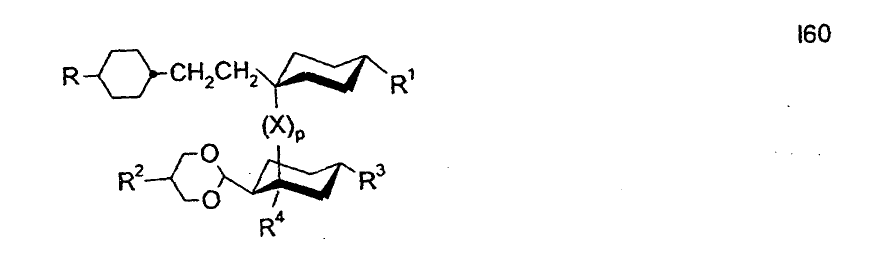 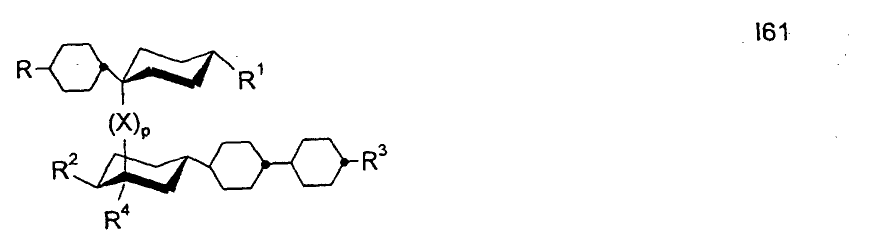 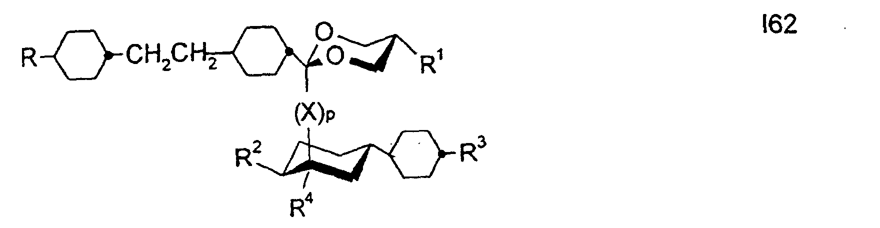 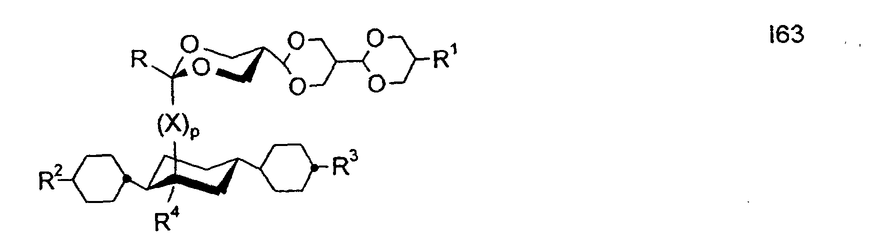 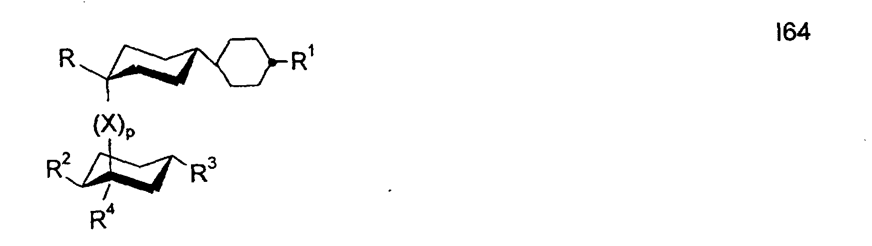 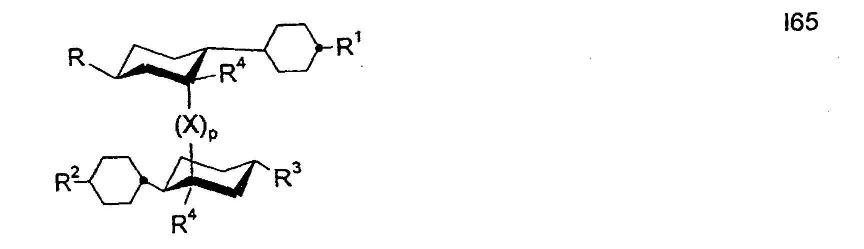 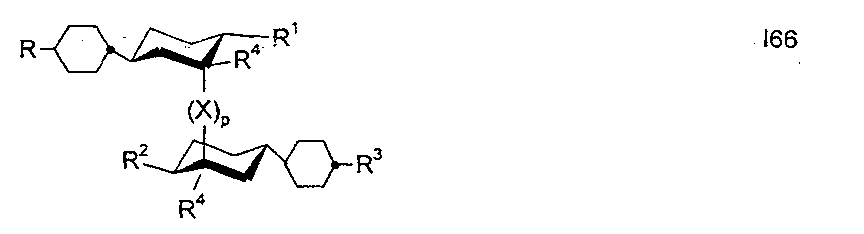 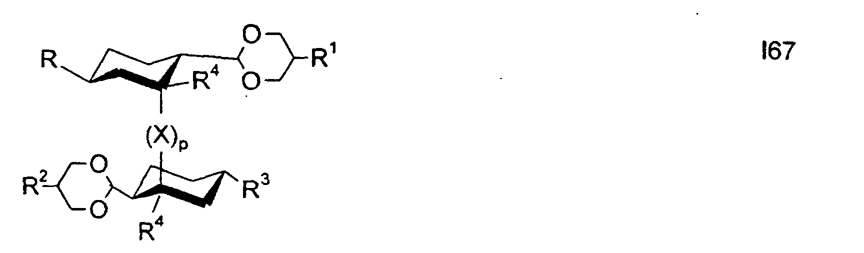 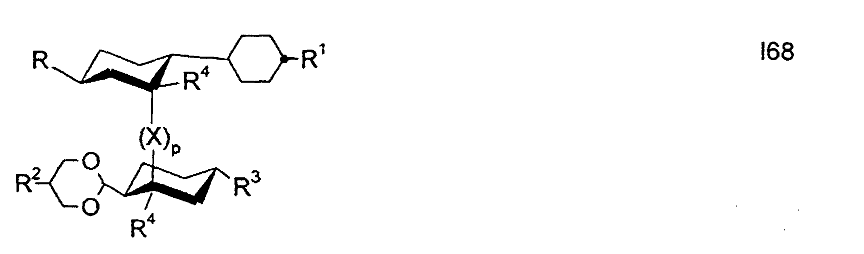 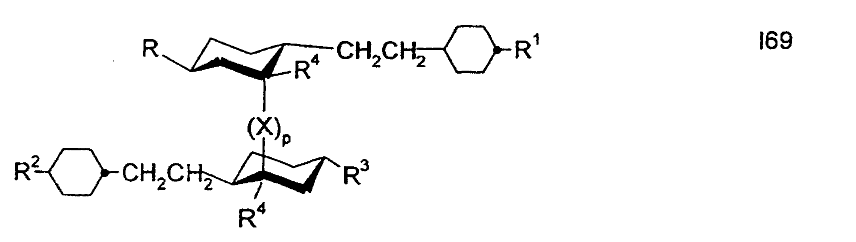 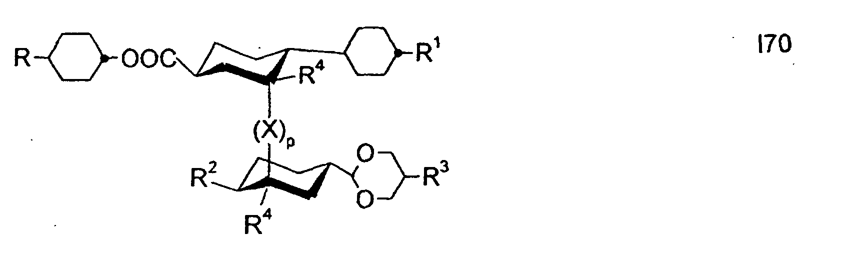 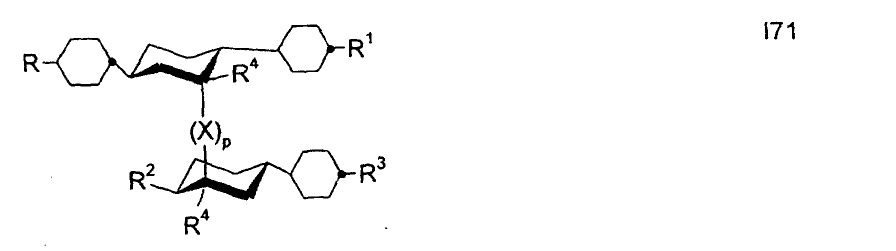 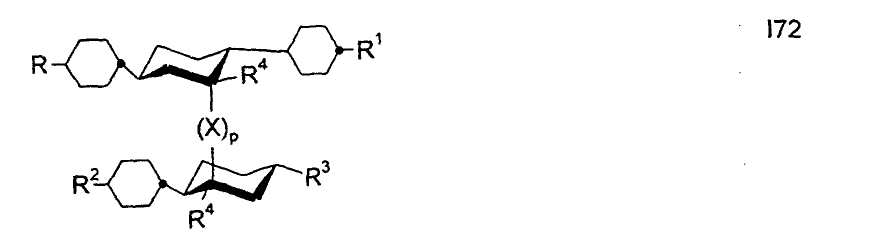 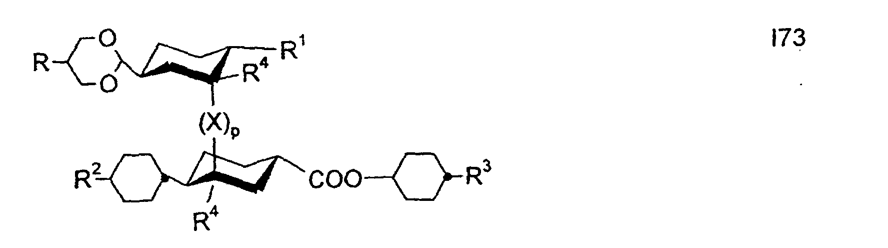 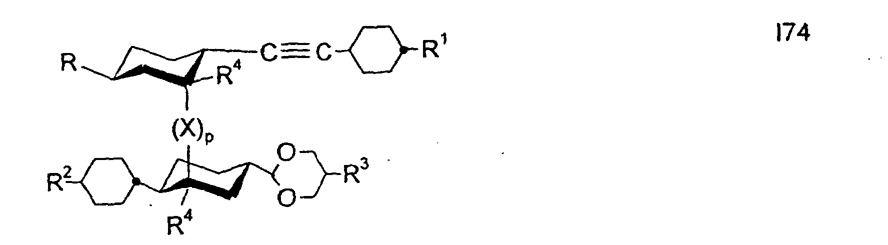 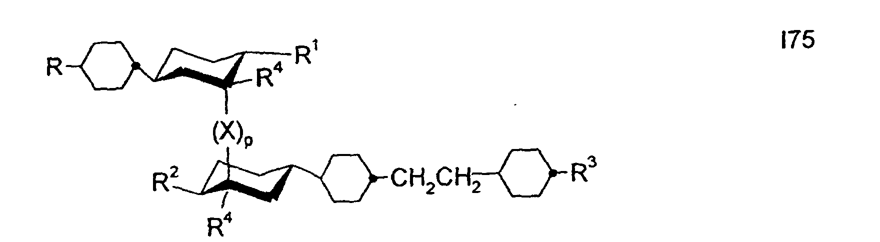 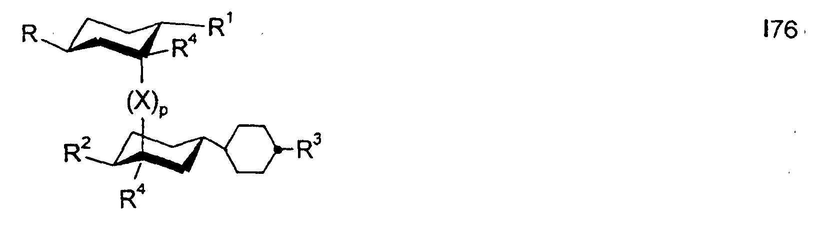 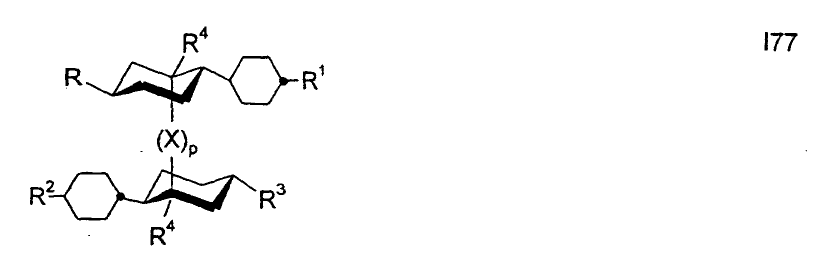 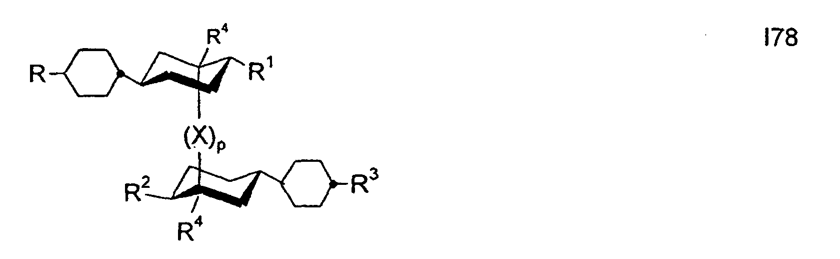 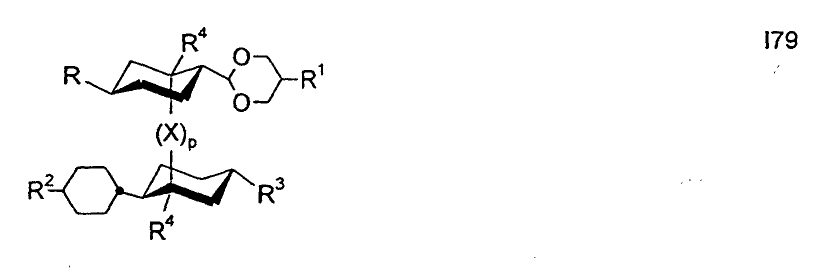 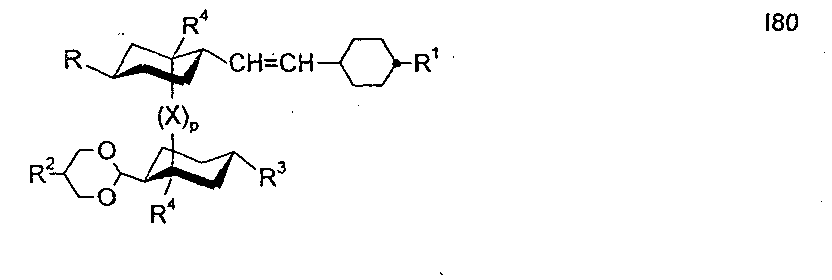 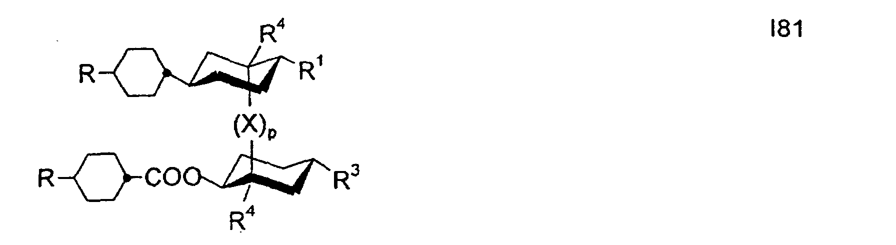 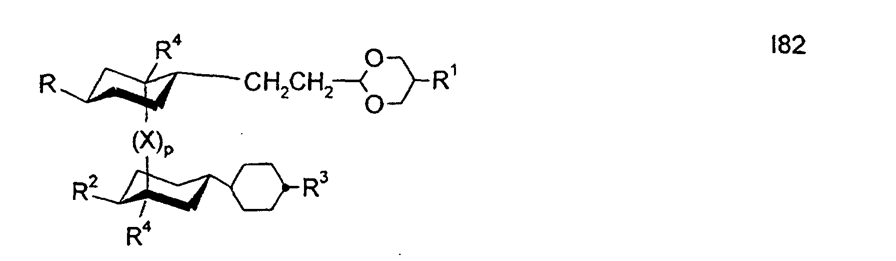 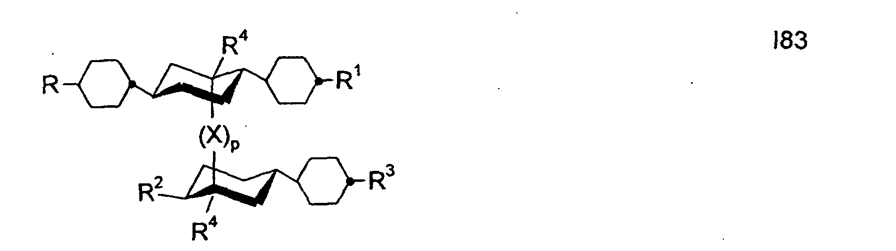 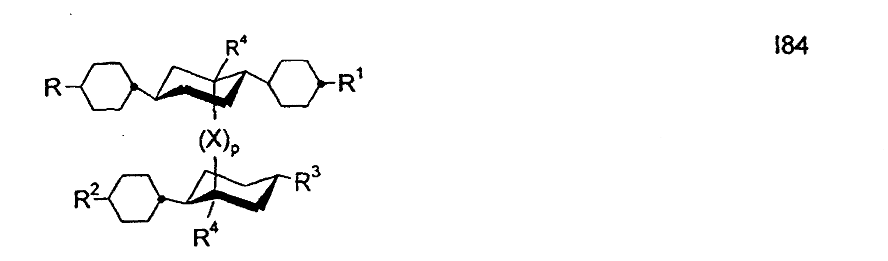 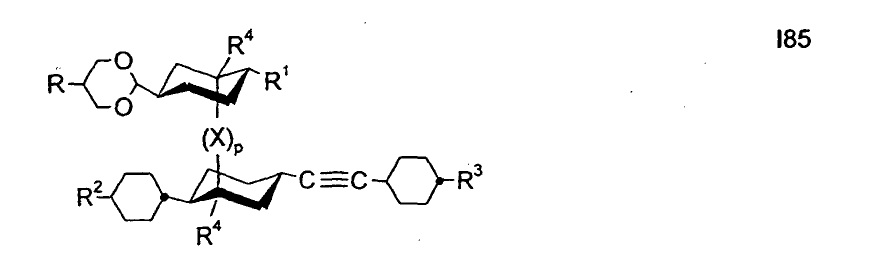 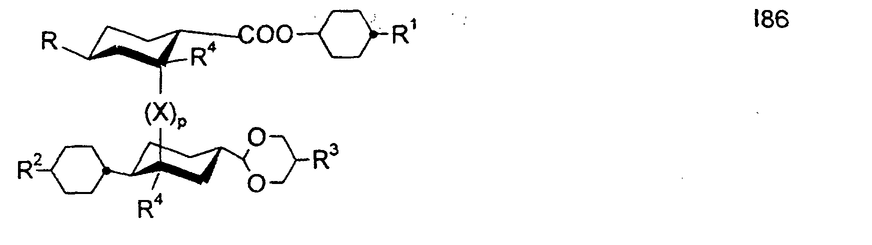 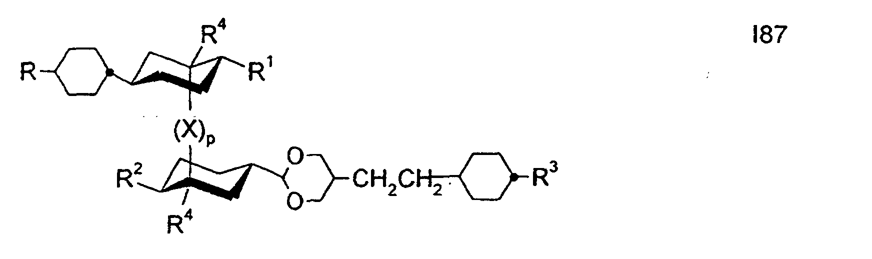 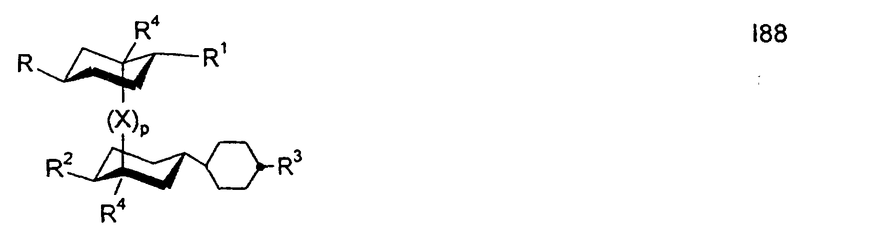 worin R, R¹, R², R³, R⁴, X und p die oben angegebene Bedeutung aufweisen.

Ganz besonders bevorzugte Verbindungen dieser Gruppe sind die der Formeln I23, 125, I28, I31, I33 und 145, I47, I64, 165, I66, I76, I77 und I85.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind.

Dabei kann man von an sich bekannten, hier nicht näher erwähnten Varianten Gebrauch machen.

Die Ausgangsstoffe können gewünschtenfalls auch in situ gebildet werden, derart, daß man sie aus dem Reaktionsgemisch nicht isoliert, sondern sofort weiter zu den Verbindungen der Formel I umsetzt.

Die Synthese der Verbindungen der Formel I worin A, A¹, A² und/oder axial fluoriertes Cyclohexan bedeutet, kann durch Anwendung von Fluorwasserstoff unter Druck oder durch Amin-Fluorwasserstoff-Addukte bewirkt werden (z.B. A. V. Grosse, C. B. Linn, J. Org. Chem. 3, (1938) 26; G. A. Olah, M. Nojima, I. Kerekes, Synthesis, (1973) 779); G. A. Olah, X-Y. Li, Q. Wang, G. K. S. Prakash, Synthesis (1993) 693).

Die Synthese der Verbindungen der Formel I, worin B, B¹, A, A¹, A² oder A³ einen Silacyclohexanrest bedeutet, kann beispielsweise analog zu B. T. Nguyen, F. K. Cartledge, J. Org. Chem. 51, 12, 1986, 2206-2210 und R. H. Peters, D. F. Crowe, M. Tanabe, M. A. Avery, W. K. M. Chong, J. Med. Chem. 30, 4, 1987, 646-652 erfolgen.

Die erfindungsgemäßen Verbindungen können z.B. nach folgenden Reaktionsschemata hergestellt werden:

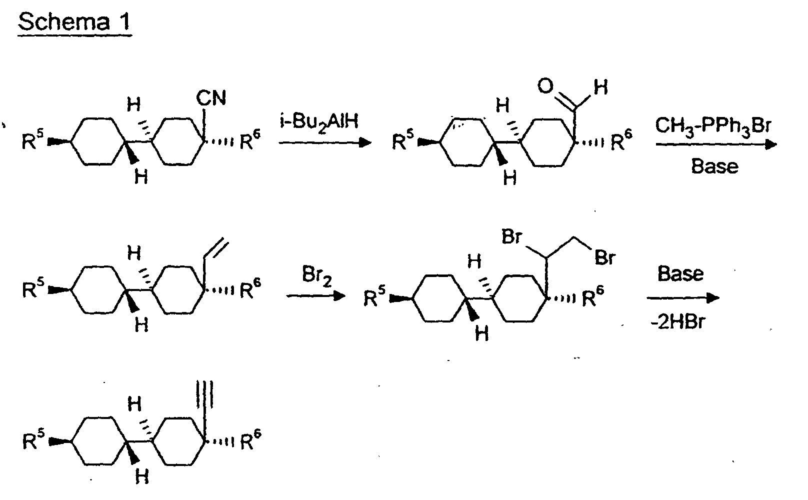

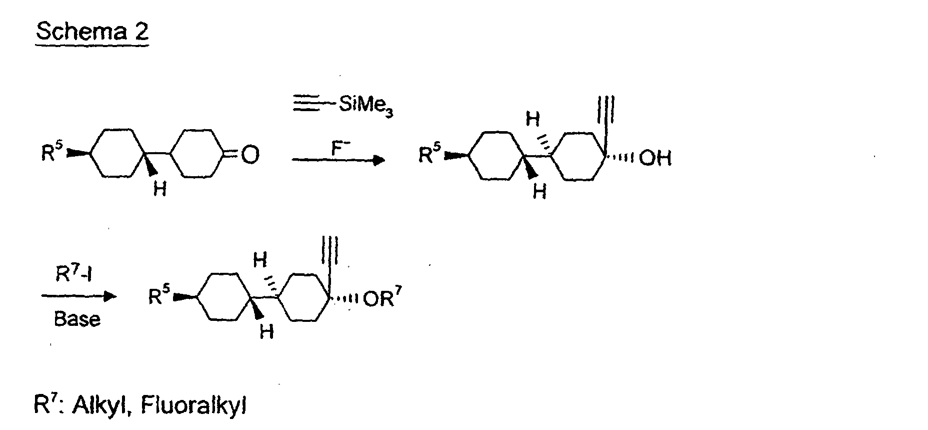

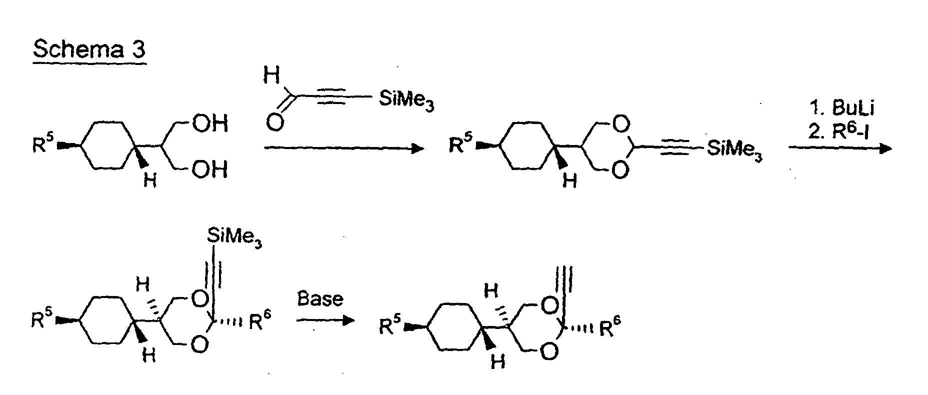

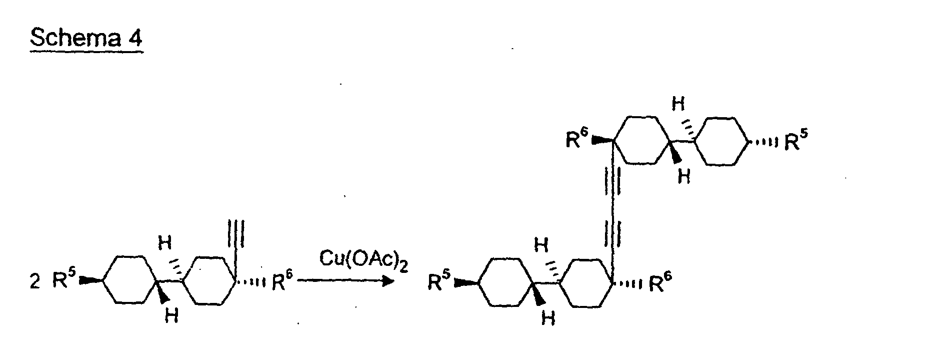

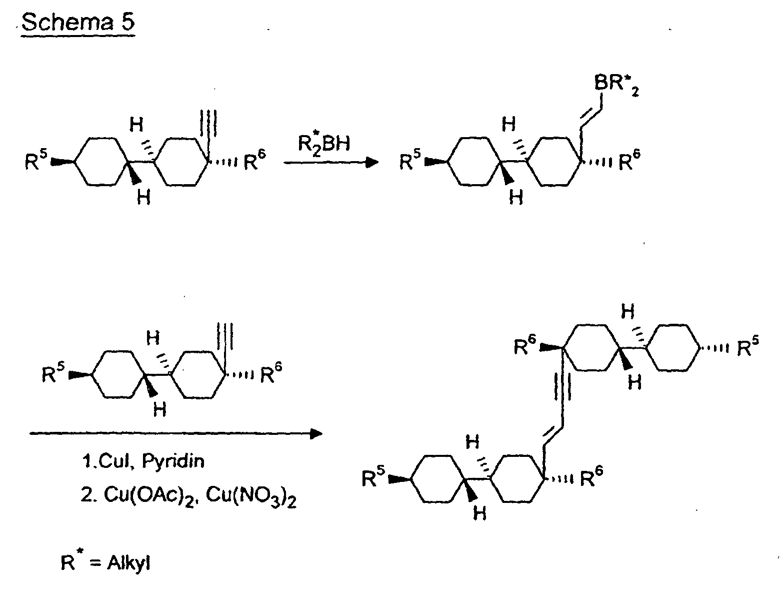

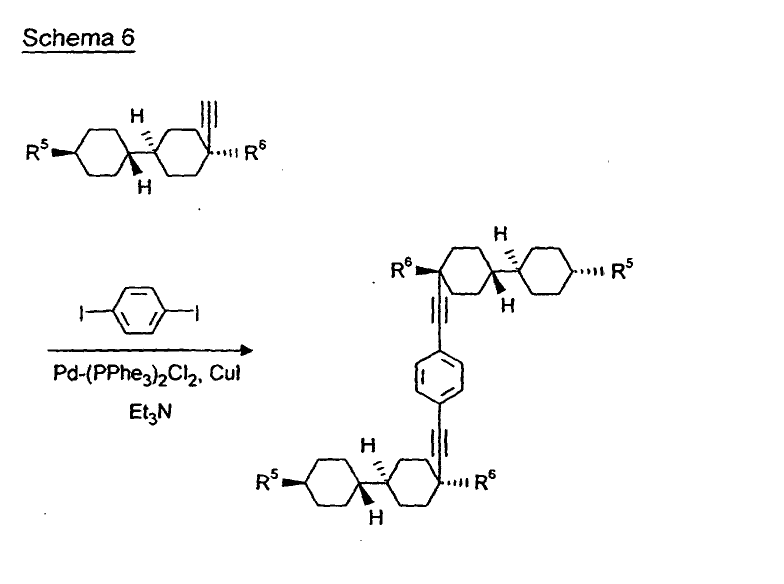

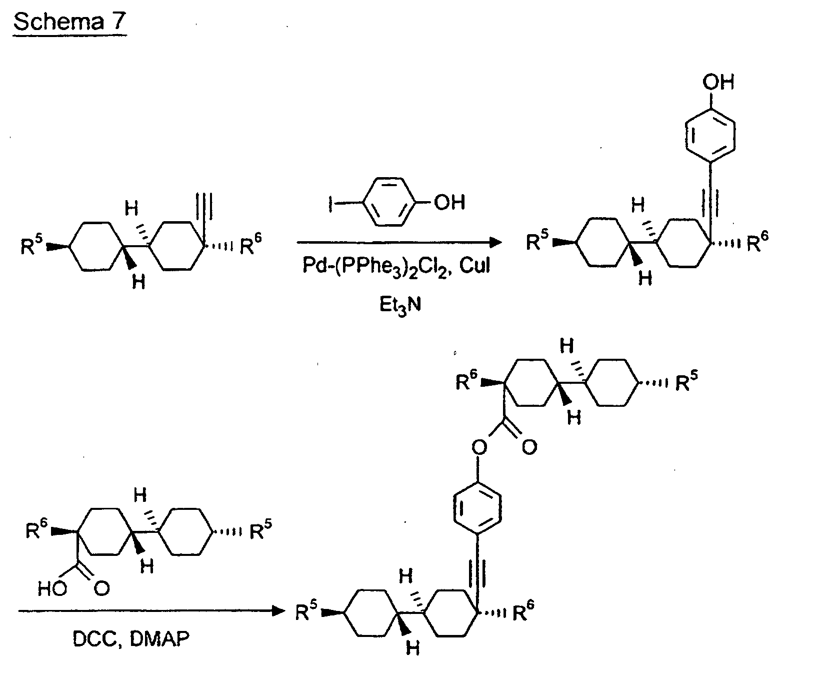

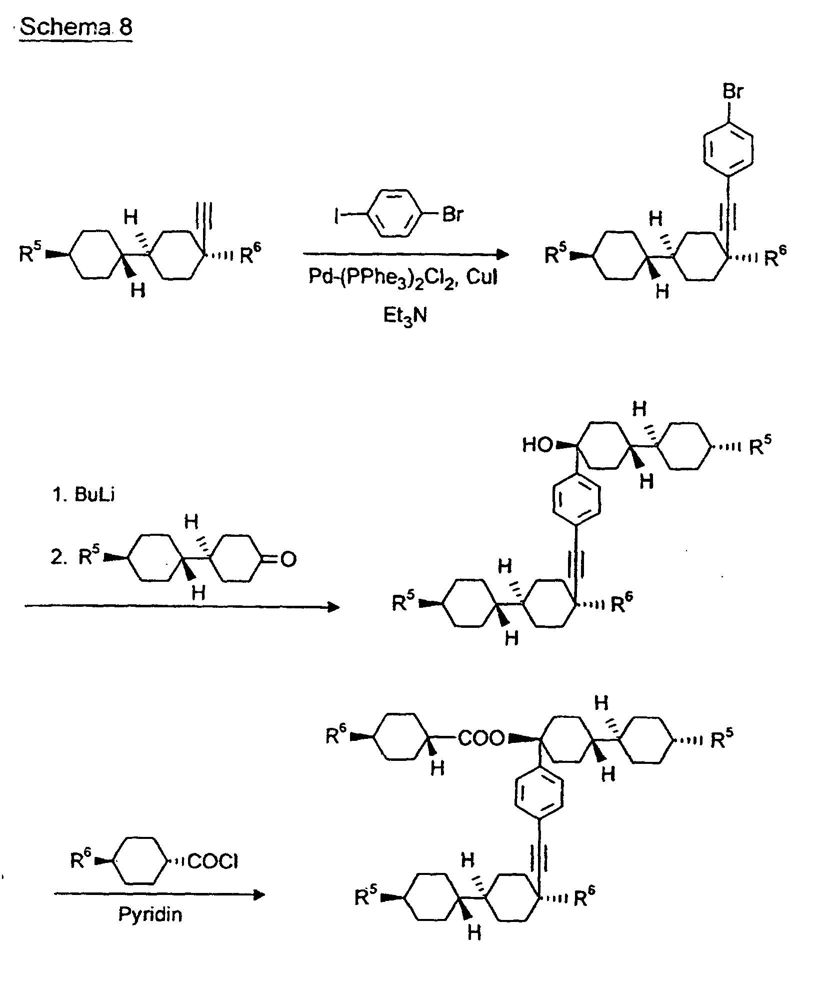

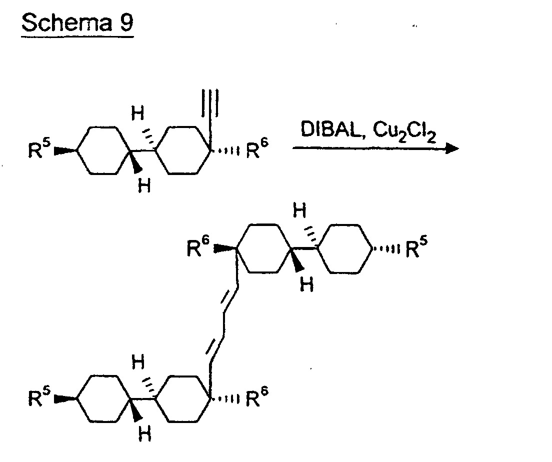

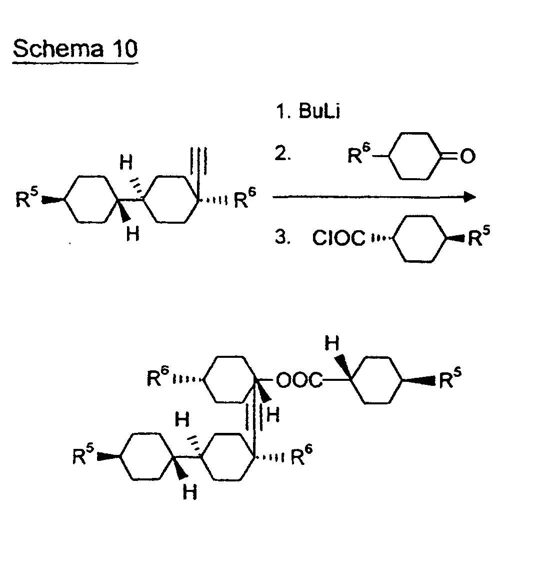

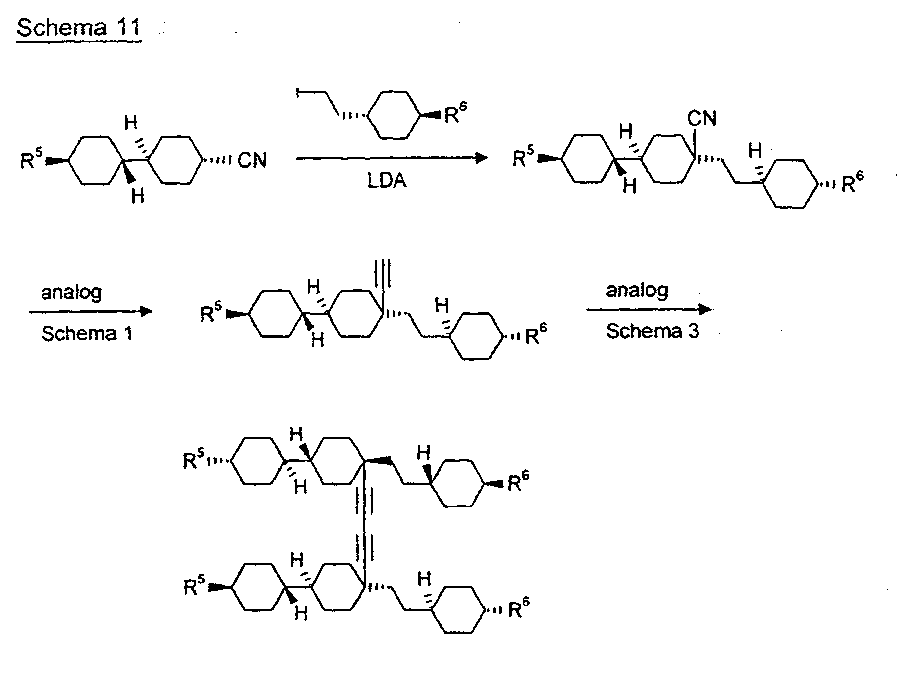

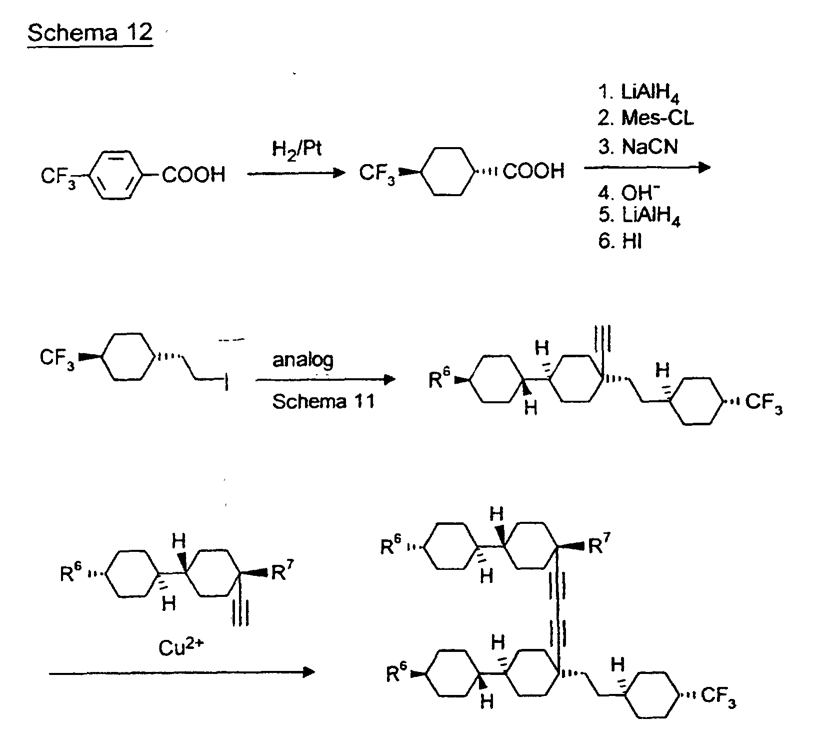

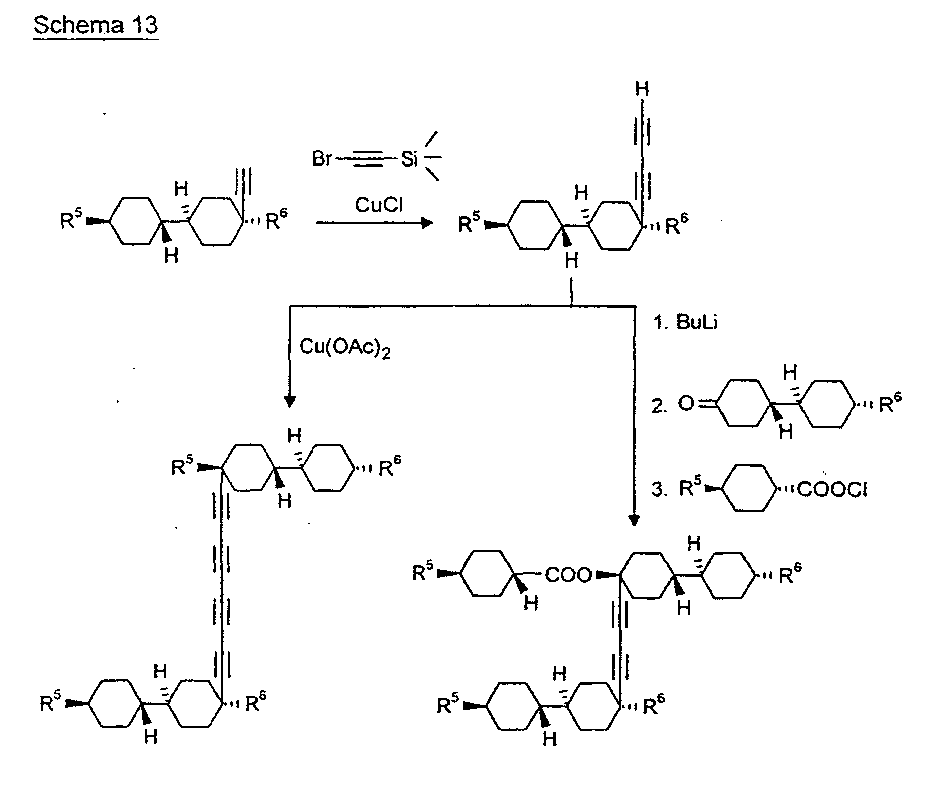

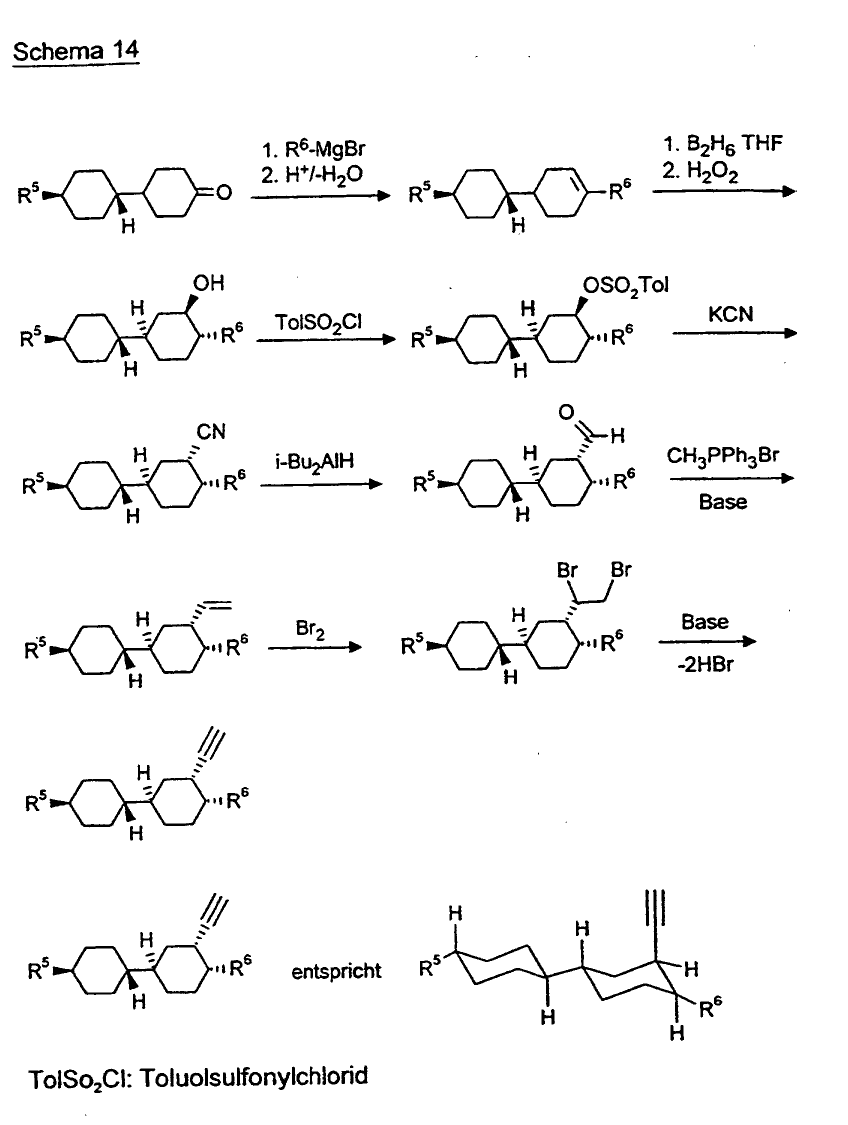

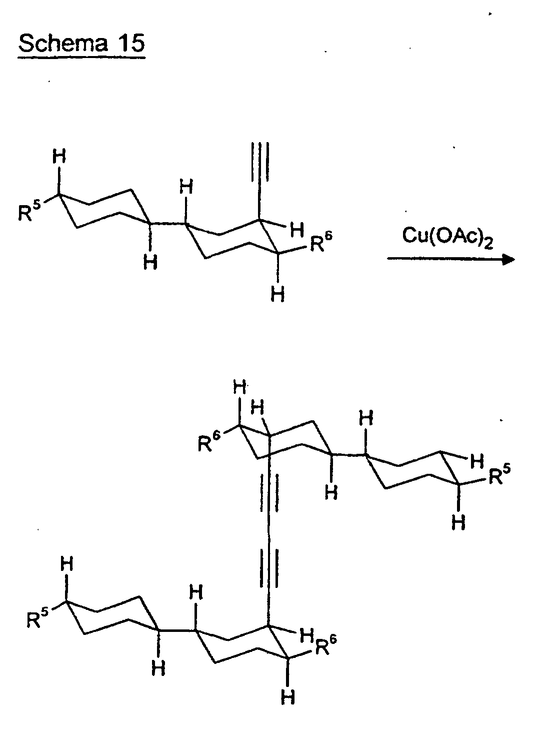

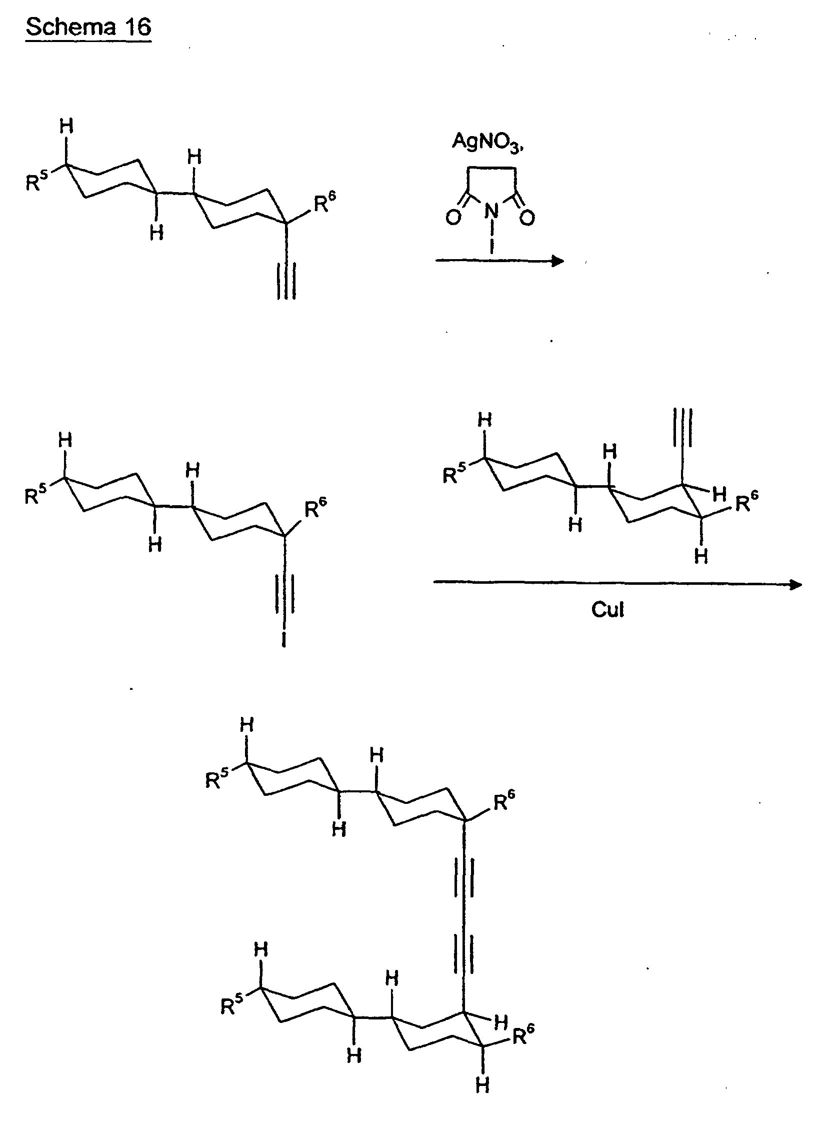

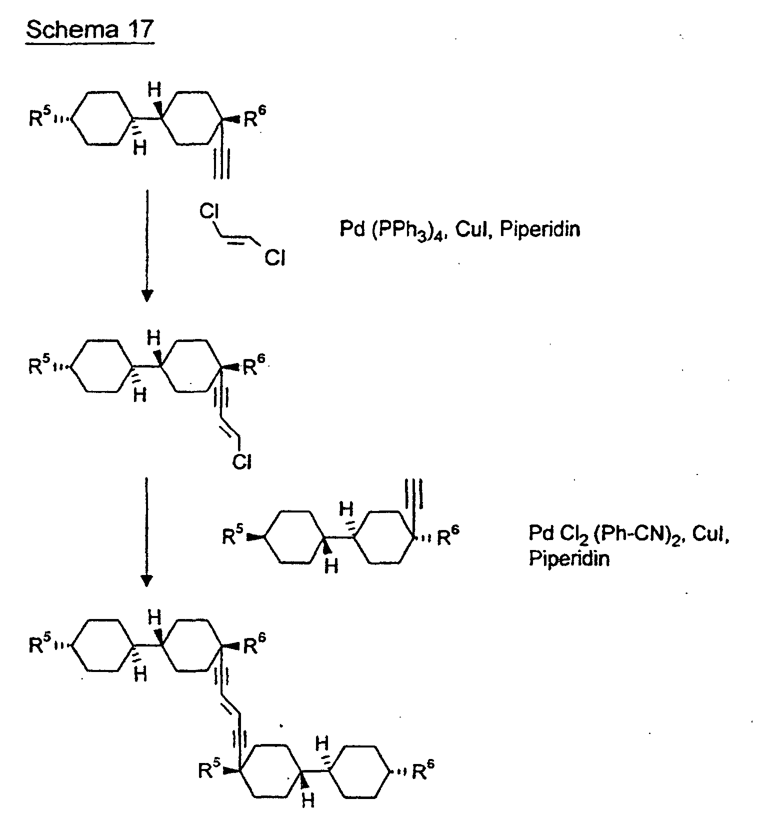

Die Umsetzungen nach Schema 17 können beispielsweise analog zu Alami et al., Tetrahedron Letters, Vol. 35, 3543-3544, 1994 erfolgen.

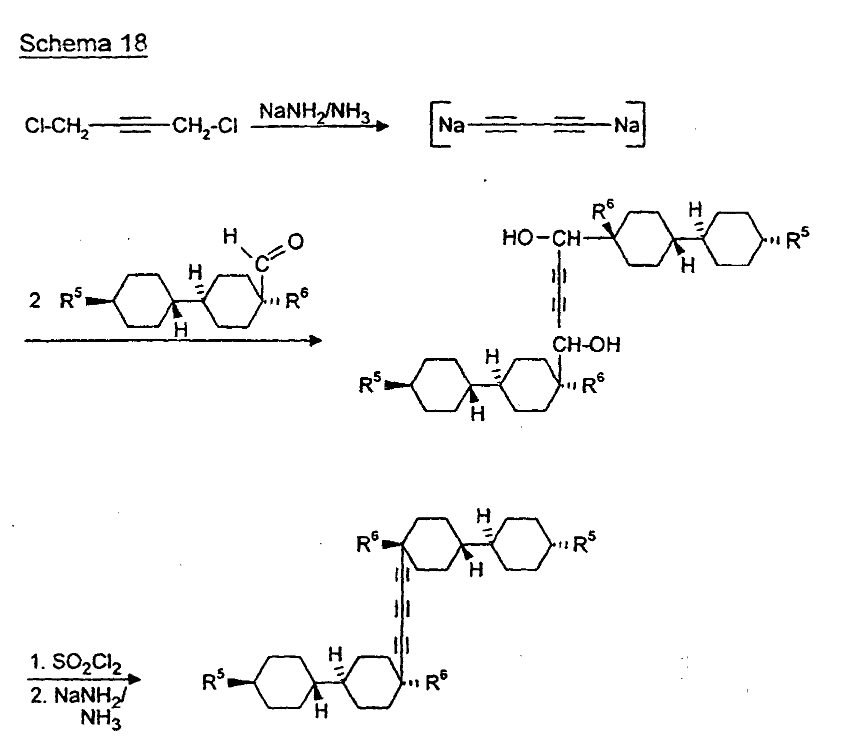

Das entsprechende Tetrain wird in analoger Weise unter Verwendung von 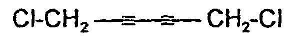 erhalten (Bohlmann, Chem. Berichte, Jahrgg. 86, Nr. 5, 1953, 663-664). 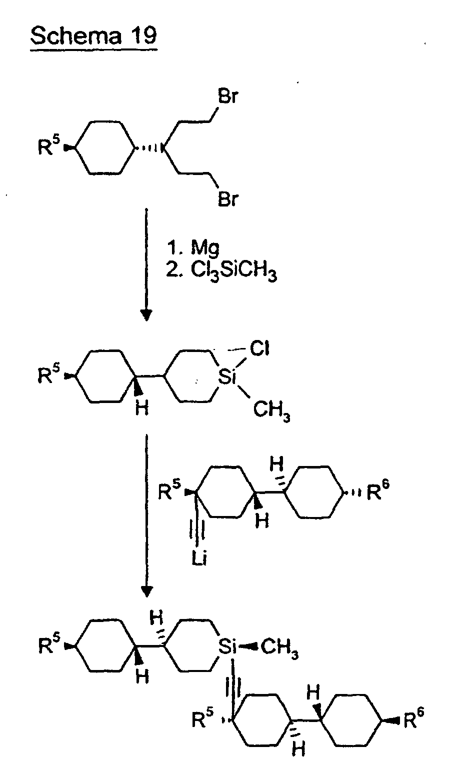 worin R⁵ und R⁶ die oben angegebne Bedeutung aufweisen.
Ester der Formel I können auch durch Versterung entsprechender Carbonsäuren (oder ihrer reaktionsfähigen Derivate) mit Alkoholen bzw. Phenolen (oder ihren reaktionsfähigen Derivaten) oder nach der DCC-Methode (DCC = Diclyhexylcarbodiimid) erhalten werden.

Die entsprechenden Carbonsäuren und Alkohole bzw. Phenole sind bekannt oder können in Analogie zu bekannten Verfahren hergestellt werden.

Als reaktionsfähige Derivate der genannten Carbonsäuren eignen sich insbesondere die Säurehalogenide, vor allem die Chloride und Bromide, ferner die Anhydride, Azide oder Ester, insbesondere Alkylester mit 1-4 C-Atomen in der Alkylgruppe.

Als reaktionsfähige Derivate der genannten Alkohole bzw. Phenole kommen insbesondere die entsprechenden Metallalkoholate bzw. Phenolate, vorzugsweise eines Alkalimetalls wie Na oder K, in Betracht.

Die Veresterung wird vorteilhaft in Gegenwart eines inerten Lösungsmittels durchgeführt. Gut geeignet sind insbesondere Ether wie Diethylether, Di-n-butylether, THF, Dioxan oder Anisol, Ketone wie Aceton, Butanon oder Cyclohexanon, Amide wie DMF oder Phosphorsäurehexamethyltriamid, Kohlenwasserstoffe wie Benzol, Toluol oder Xylol, Halogenkohlenwasserstoffe wie Tetrachlorkohlenstoff oder Tetrachlorethylen und Sulfoxide wie Dimethylsulfoxid oder Sulfolan. Mit Wasser nicht mischbare Lösungsmittel können gleichzeitig vorteilhaft zum azeotropen Abdestillieren des bei der Veresterung gebildeten Wassers verwendet werden. Gelegentlich kann auch ein Überschuß einer organischen Base, z.B. Pyridin, Chinolin oder Triethylamin, als Lösungsmittel für die Veresterung angewandt werden. Die Veresterung kann auch in Abwesenheit eines Lösungsmittels, z.B. durch einfaches Erhitzen der Komponenten in Gegenwart von Natriumacetat, durchgeführt werden. Die Reaktionstemperatur liegt gewöhnlich zwischen -50° und +250°, vorzugsweise zwischen -20° und +80°. Bei diesen Temperaturen sind die Veresterungsreaktionen in der Regel nach 15 Minuten bis 48 Stunden beendet.

Im einzelnen hängen die Reaktionsbedingungen für die Veresterung weitgehend von der Natur der verwendeten Ausgangsstoffe ab.

So wird eine freie Carbonsäure mit einem freien Alkohol oder Phenol in der Regel in Gegenwart einer starken Säure, beispielsweise einer Mineralsäure wie Salzsäure oder Schwefelsäure, umgesetzt. Eine bevorzugte Reaktionsweise ist die Umsetzung eines Säureanhydrids oder insbesondere eines Säurechlorids mit einem Alkohol, vorzugsweise in einem basischen Milieu, wobei als Basen insbesondere Alkalimetallhydroxide wie Natrium- oder Kaliumhydroxid, Alkalimetallcarbonate bzw. -hydrogencarbonate wie Natriumcarbonat, Natriumhydrogencarbonat, Kaliumcarbonat oder Kaliumhydrogencarbonat, Alkalimetallacetate wie Natrium- oder Kaliumacetat, Erdalkalimetallhydroxide wie Calciumhydroxid oder organische Basen wie Triethylamin, Pyridin, Lutidin, Kollidin oder Chinolin von Bedeutung sind. Eine weitere bevorzugte Ausführungsform der Veresterung besteht darin, daß man den Alkohol bzw. das Phenol zunächst in das Natrium- oder Kaliumalkoholat bzw. -phenolat überführt, z.B. durch Behandlung mit ethanolischer Natron- oder Kalilauge, dieses isoliert und mit einem Säureanhydrid oder insbesondere Säurechlorid umsetzt.

Nitrile können durch Austausch von Halogenen mit Kupfercyanid oder Alkalicyanid erhalten werden.

In einem weiteren Verfahren zur Herstellung der Verbindungen der Formel I, die eine Gruppe der Formel 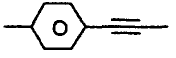 enthalten, setzt man ein Arylhalogenid mit einem Alkin um in Gegenwart einer Base und eines Palladiumkatalysators. Geeignete Arylhalogenide sind beispielsweise Chloride, Bromide und Iodide, insbesondere Bromide und Iodide. Die für das Gelingen der Kupplungsreaktion erforderliche Base, wie z.B. Triethylamin, eignen sich auch als Lösungsmittel. Als Palladiumkatalysatoren sind beispielsweise dessen Salze, insbesondere (Pd(II)-acetat, mit organischen Phosphor(III)-Verbindungen wie z.B. Triarylphosphanen geeignet.

Man kann dabei in Gegenwart oder Abwesenheit eines inerten Lösungsmittels bei Temperaturen zwischen etwa 0 °C und 150 °C, vorzugsweise zwischen 20 °C und 100 °C, arbeiten; als Lösungsmittel kommen z.B. Nitrile wie Acetonitril oder Kohlenwasserstoffe wie Benzol oder Toluol in Betracht. Die als Ausgangsstoffe eingesetzten Arylhalogenide und Alkine sind vielfach im Handel erhältlich oder können nach literaturbekannten Verfahren hergestellt werden, beispielsweise durch Halogenierung entsprechender Stammverbindungen bzw. durch Eliminierungsreaktionen an entsprechenden Alkoholen oder Halogeniden.

Kopplungen von Alkinyl-Verbindungen mit Arylhalogeniden können analog dem von A.O. King, E. Negishi, F.J. Villani und A. Silveira in J. Org. Chem 43, 358 (1978) beschriebenen Verfahren durchgeführt werden.

Ether der Formel I sind durch Veretherung entsprechender Hydroxyverbindungen, vorzugsweise entsprechender Phenole, erhältlich, wobei die Hydroxyverbindung zweckmäßig zunächst in ein entsprechendes Metallderivat, z.B. durch Behandeln mit NaH, NaNH₂, NaOH, KOH, Na₂CO₃ oder K₂CO₃ in das entsprechende Alkalimetallalkoholat oder Alkalimetallphenolat übergeführt wird. Dieses kann dann mit dem entprechenden Alkylhalogenid, -sulfonat oder Dialkylsulfat umgesetzt werden, zweckmäßig in einem inerten Lösungsmittel wie z.B. Aceton, 1,2-Dimethoxythan, DMF oder Dimethylsulfoxid oder auch mit einem Überschuß an wäßriger oder wäßrig-alkoholischer NaOH oder KOH bei Temperaturen zwischen etwa 20 °C und 100 °C.

Die Verknüpfung eines aromatischen Kern mit einem nicht aromatischen Kern oder zweier nicht aromatischer Kerne erhält man vorzugsweise durch Kondensation einer lithium- oder magnesiumorganischen Verbindung mit einem Keton, falls zwischen den Kernen eine aliphatische Gruppe Z, Z¹, Z² und/oder Z³ sein soll.

Die metallorganischen Verbindungen stellt man beispielsweise durch Metall-Halogenaustausch (z.B. nach Org. React. 6, 339-366 (1951)) zwischen der entsprechenden Halogen-Verbindung und einer lithiumrganischen Verbindung wie vorzugsweise tert-Butyllithium oder Lithium-Naphthalenid oder durch Umsatz mit Magnesiumspänen her.

Darüberhinaus können die Verbindungen der Formel hergestellt werden, indem man eine Verbindung, die sonst der Formel I entspricht, aber an Stelle von H-Atomen eine oder mehrere reduzierbare Gruppen und/oder C-C-Bindungen enthält, reduziert.

Als reduzierbare Gruppen kommen vorzugsweise Carbonylgruppen in Betracht, insbesondere Ketogruppen, ferner z.B. freie oder veresterte Hydroxygruppen oder aromatisch gebundene Halogenatome. Bevorzugte Ausgangsstoffe für die Reduktion sind Verbindungen entsprechend der Formel I, die aber an Stelle eines Cyclohexanringes einen Cyclohexenring oder Cyclohexanonring und/oder an Stelle einer -CH₂CH₂-Gruppe eine -CH=CH-Gruppe und/oder an Stelle einer -CH₂-Gruppe eine -CO-Gruppe und/oder an Stelle eine H-Atoms eine freie oder eine funktionell (z.B. in Form ihres p-Toluolsulfonats) abgewandelte OH-Gruppe enthalten.

Die Reduktion kann z.B. erfolgen durch katalytische Hydrierung bei Temperaturen zwischen etwa 0° und etwa 200° sowie Drucken zwischen etwa 1 und 200 bar in einem inerten Lösungsmittel, z.B. einem Alkohol wie Methanol, Ethanol oder Isopropanol, einem Ether wie Tetrahydrofuran (THF) oder Dioxan, einem Ester wie Tetrahydrofuran (THF) oder Dioxan, einem Ester wie Ethylacetat, einer Carbonsäure wie Essigsäure oder einem Kohlenwasserstoff wie Cyclohexan. Als Katalysatoren eignen sich zweckmäßig Edelmetalle wie Pt oder Pd, die in Form von Oxiden (z.B. PtO₂, PdO), auf einem Träger (z.B. Pd auf Kohle, Calciumcarbonat oder Strontiumcarbonat) oder in feinverteilter Form eingesetzt werden können.

Ketone können auch nach den Methoden von Clemmensen (mit Zink, amalgamiertem Zink oder Zinn und Salzsäure, zweckmäßig in wäßrig-alkoholischer Lösung oder in heterogener Phase mit Wasser/Toluol bei Temperaturen zwischen etwa 80 und 120°) oder Wolff-Kishner (mit Hydrazin, zweckmäßig in Gegenwart von Alkali wie KOH oder NaOH in einem hochsiedenden Lösungsmittel wie Diethylenglykol oder Triethylenglykol bei Temperturen zwischen etwa 100 und 200°) zu den entsprechendenn Verbindungen der Formel I, die Alkylgruppen und/oder -CH₂CH₂-Brücken enthalten, reduziert werden.

Weiterhin sind Reduktionen mit komplexen Hydriden möglich. Beispielsweise können Arylsulfonyloxygruppen mit LiAlH₄ reduktiv entfernt werden, insbesondere p-Toluolsulfonyloxymethylgruppen zu Methylgruppen reduziert werden, zweckmäßig in einem inerten Lösungsmittel wie Diethylether oder THF bei Temperaturen zwischen etwa 0 und 100°. Doppelbindungen können mit NaBH₄ oder Tributylzinnhydrid in Methanol hydriert werden.

Die Ausgangsmaterialien sind entweder bekannt oder können in Analogie zu bekannten Verbindungen hergestellt werden.

Die erfindungsgemäßen flüssigkristallinen Medien enthalten vorzugsweise neben einer oder mehreren erfindungsgemäßen Verbindungen als weitere Bestandteile 2 bis 40, insbesondere 4 bis 30 Komponenten. Ganz besonders bevorzugt enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexyl-ester der Cyclohexylcyclohexancarbonsäure, Cyclohexyl-phenylester der Benzoesäure, der Cyclohexancarbonsäure, bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile erfindungsgemäßer Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R" 4

R'-L-C≡C-E-R" 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, - Dio-, -G-Phe- und -G-Cyc- sowie deren Spiegelbilder gebildeten Gruppe, wobei Phe unsubstituiertes oder durch Fluor substituiertes 1,4-Phenylen, Cyc trans-1,4-Cyclohexylen oder 1,4-Cyclohexylen, Pyr Pyrimidin-2-5-diyl oder Pyridin-2,5-diyl, Dio 1,3-Dioxan-2,5-diyl und G 2-(trans-1,4-Cyclohexyl)-ethyl, Pyrimidin-2,5-diyl, Pyridin-2,5-diyl oder 1,3-Dioxan-2,5-diyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 Kohlenstoffatomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder - (O)ᵢ CH₃₋₍ₖ₊ₗ₎ FₖClₗ, wobei i 0 oder 1 und k und I 1, 2 oder 3 sind; die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1b, 2b, 3b, 4b und 5b, in denen R" die Bedeutung -F, -Cl, -NCS, -CF₃, -OCHF₂ oder -OCF₃ hat.

In den Verbindungen der Teilformeln 1b, 2b, 3b, 4b und 5b hat R' die bei den Verbindungen der Teilformeln 1a-5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN; diese Untergruppe wird im folgenden als Gruppe C bezeichnet und die Verbindungen dieser Untergruppe werden entsprechend mit Teilformeln 1c, 2c, 3c, 4c und 5c beschrieben.

In den Verbindungen der Teilformeln 1c, 2c, 3c, 4c und 5c hat R' die bei den Verbindungen der Teilformeln 1a-5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus der Gruppe A und/oder Gruppe B und/oder Gruppe C. Die Massenanteile der Verbindungen aus diesen Gruppen an den erfindungsgemäßen Medien sind vorzugsweise:

| | |
|---|---|
| Gruppe A | 0 bis 90 %, vorzugsweise 20 bis 90 %, insbesondere 30 bis 90 % |
| | |
| Gruppe B | 0 bis 80 %, vorzugsweise 10 bis 80 %, insbesondere 10 bis 65 % |
| | |
| Gruppe C | 0 bis 80 %, vorzugsweise 5 bis 80 %, insbesondere 5 bis 50 % |

wobei die Summe der Massenanteile der in den jeweiligen erfindungsgemäßen Medien enthaltenen Verbindungen aus den Gruppen A und/oder B und/oder C vorzugsweise 5 %-90 % und insbesondere 10 % bis 90 % beträgt.

Die erfindungsgemäßen Medien enthalten vorzugsweise 1 bis 40 %, insbesondere vorzugsweise 5 bis 30 % der erfindungsgemäßen Verbindungen. Weiterhin bevorzugt sind Medien, enthaltend mehr als 40 %, insbesondere 45 bis 90 % an erfindungsgemäßen Verbindungen.

Die Medien enthalten vorzugsweise drei, vier oder fünf erfindungsgemäße Verbindungen.

Die Herstellung der erfindungsgemäßen Medien erfolgt in an sich üblicher Weise. In der Regel werden die Komponenten ineinander gelöst, zweckmäßig bei erhöhter Temperatur. Durch geeignete Zusätze können die flüssigkristallinen Phasen nach der Erfindung so modifiziert werden, daß sie in allen bisher bekannt gewordenen Arten von Flüssigkristallanzeigeelementen verwendet werden können. Derartige Zusätze sind dem Fachmann bekannt und in der Literatur ausführlich beschrieben (H. Kelker/ R. Hatz, Handbook of Liquid Crystals, Verlag Chemie, Weinheim, 1980). Beispielsweise können pleochroitische Farbstoffe zur Herstellung farbiger Guest-Host-Systeme oder Substanzen zur Veränderung der dielektrischen Anisotropie, der Viskosität und/oder Orientierung der nematischen Phasen zugesetzt werden.

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Fp. bedeutet Schmelzpunkt Klp. = Klärpunkt und Tg = Glastemperatur. Ferner bedeuten K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm 20 °C) und Δε die dielektrische Anisotropie (1kHz, 20 °C).
Δn- und Δε-Werte der erfindungsgemäßen Verbindungen wurden durch Extrapolation aus flüssigkristallinen Mischungen erhalten, die zu 10 % aus der jeweiligen erfindungsgemäßen Verbindung und zu 90 % aus dem kommerziell erhältlichen Flüssigkristall ZLI 4792 (Fa. Merck, Darmstadt) bestanden.
Die Viskosität (mm²/sec) wurde bei 20 °C bestimmt.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie.

Folgende Abkürzungen werden verwendet:

| | |
|---|---|
| THF | Tetrahydrofuran |
| KOtBu | Kalium-tert.-butylat |
| RT | Raumtemperatur |
| MTB-Ether | Methyl-tert.-butylether |

### Beispiel 1

### 4-[4-(4,4'-Dipentylbicyclohexyl-4-yl)-buta-1,3-diinyl]-4,4'-dipentylbicyclohexyl

a) 4,4'-Dipentylbicyclohexyl-4-carbaldehyd
   61.00 g 4,4'-Dipentylbicyclohexyl-4-carbonitril (z.B. erhältlich nach EP 0 107 759) werden in 225 ml Toluol gelöst langsam zu einer 16.5 %igen Lösung von Diisobutylaluminiumhydrid in Hexan getropft. Die Reaktionsmischung wird 2 h bei RT gerührt und anschließend auf eine Mischung aus Eis und verdünnter Salzsäure gegossen. Das entstandene Gemisch wird für 1 h gerührt, mit Toluol extrahiert und wie üblich aufgearbeitet, wodurch 4,4'-Dipentylbicyclohexyl-4-carbaldehyd erhalten wird.
b) 4,4'-Dipentyl-4-vinyl-bicyclohexyl
   Zu einer Suspension aus 53.20 g 4,4'-Dipentylbicyclohexyl-4-carbaldehyd und 56.80 g Methyltriphenylphosphoniumbromid in 300 ml THF wird bei 0°C eine Lösung von 17.84 g KOtBu in 100 ml THF getropft. Man rührt über Nacht bei RT, versetzt mit Wasser und extrahiert mit MTB-Ether. Die vereinigten organischen Phasen werden wie üblich aufgearbeitet, wodurch 4,4'-Dipentyl-4-vinyl-bicyclohexyl erhalten wird.
c) 4-(1,2-Dibromethyl)-4,4'-dipentylbicyclohexyl
   Zu 36.40 g 4,4'-Dipentyl-4-vinyl-bicyclohexyl in 120 ml Diehtylether werden bei 0°C bis -10°C 4.754 ml Brom getropft. Nach 1 h ohne Kühlung wird die Reaktionsmischung mit Wasser versetzt und wie üblich aufgearbeitet, wodurch 4-(1,2-Dibromethyl)-4,4'dipentylbicyclohexyl erhalten wird.
d) 4-Ethinyl-4,4'-dipentylbicyclohexyl
   Zu 47.50 g 4-(1,2-Dibromethyl)-4,4'-dipentylbicyclohexyl in 150 ml tert-Butanol werden bei RT 32.48 g KOtBu gegeben. Man erhitzt auf 60°C und rührt bei dieser Temperatur über Nacht Anschließend wird die Reaktionsmischung mit Wasser versetzt, mit verdünnter Salzsäure angesäuert und wie üblich aufgearbeitet, wodurch 4-Ethinyl-4,4'-dipentylbicyclohexyl erhalten wird.
e) 4-[4-(4,4'-Dipentylbicyclohexyl-4-yl)-buta-1,3-diinyl]-4,4'-dipentylbicyclohexyl
   Eine Mischung aus 5.00 g 4-Ethinyl-4,4'-dipentylbicyclohexyl, 12.5 ml Pyridin, 12.5 ml Methanol und 3.635 g Kupfer(II)-Acetat-Monohydrat wird 1.5 h bei RT gerührt und anschließend zum Sieden erhitzt. Man rührt für 10 h und arbeitet wie üblich auf, wodurch 4-[4-(4,4'-Dipentylbicyclohexyl-4-yl)-buta-1,3-diinyl]-4,4'-dipentylbicyclohexyl erhalten wird (K 77 SmB 198 I, Δn = 0.013, Δε = -0.27).

### Beispiel 2

### 4-[4-(4,4'-Dipentylbicyclohexyl-4-yl)-buta-1,3-diinyl]-4-methoxy-4'-propylbicyclohexyl und 4-[4-(4-Methoxy-4'-propylbicyclohexyl-4-yl)-buta-1,3-diinyl]-4-methoxy-4'-propylbicyclohexyl

a) 4-Ethinyl-4'-propylbicyclohexyl-4-ol
   Zu einer Lösung von 30.0 g 4'-Propylbicyclohexyl-4-on und 3.41 g Tetrabutylammoniumfluorid-Trihydrat in 300 ml THF werden 24.32 ml (Trimethylsilyl)acetylen bei -30°C getropft. Das Kühlbad wird nach 5 min. entfernt und die Reaktionsmischung für 2 h bei RT gerührt. Anschließend gibt man eine Suspension von 39.2 g Kaliumfluorid in Methanol hinzu und rührt für 3 Tage bei RT. Nach üblicher Aufarbeitung wird 4-Ethinyl-4'-propylbicyclohexyl-4-ol erhalten.
b) 4-Ethinyl-4-methoxy-4'-propylbicyclohexyl
   Zu einer auf -30°C gekühlten Lösung von 13.0 g 4-Ethinyl-4'-propylbicyclohexyl-4-ol in 24 ml THFwerden 28.85 ml einer 15%igen Lösung von Butyllithium in Hexan getropft. Bei -5°C wird anschließend eine Lösung von 4.67 ml Iodmethan in 36 ml Dimethylsulfoxid zur Reaktionsmischung getropft. Man rührt über Nacht bei RT und arbeitet wie üblich auf, wodurch 4-Ethinyl-4-methoxy-4'-propylbicyclohexyl erhalten wird.
c) 4-[4-(4,4'-Dipentylbicyclohexyl-4-yl)-buta-1,3-diinyl]-4-methoxy-4'-propylbicyclohexyl
   und
   4-[4-(4-Methoxy-4'-propylbicyclohexyl-4-yl)-buta-1,3-diinyl]-4-methoxy-4'-propylbicyclohexyl
   4.00 g 4-Ethinyl-4-methoxy-4'-propylbicyclohexyl, 5.04 g 4-Ethinyl-4,4'-dipentylbicyclohexyl, 10.65 g Kupfer(II)-acetat-monohydrat und 0.145 g Kupfer(I)-iodid werden in einer Mischung von 25 ml Pyridin und 25 ml Methanol über Nacht unter Rückfluß erhitzt. Nach üblicher Aufarbeitung erhält man eine Mischung der drei Kupplungsprodukte 4-[4-(4,4'-Dipentylbicyclohexyl-4-yl)-buta-1,3-diinyl]-4-methoxy-4'-propylbicyclohexyl (K 76 N 136 I, Δε = 0.95, Δn = 0.006) 4-[4-(4,4'-Dipentylbicyclohexyl-4-yl)-buta-1,3-diinyl]-4,4'-dipentylbicyclohexyl und 4-[4-(4-Methoxy-4'-propylbicyclohexyl-4-yl)-buta-1,3-diinyl]-4-methoxy-4'-propylbicyclohexyl (K 121 N (80) I, Δε=-1.77, Δn = -0.002) die sich chromatographisch leicht trennen läßt.
   Analog werden aus den entsprechenden Vorstufen die folgenden erfindungsgemäßen Verbindungen erhalten:

### Beispiele 3-24, 24a), 24b)

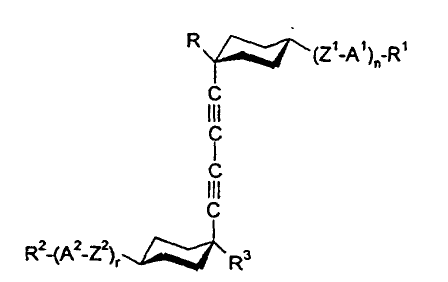

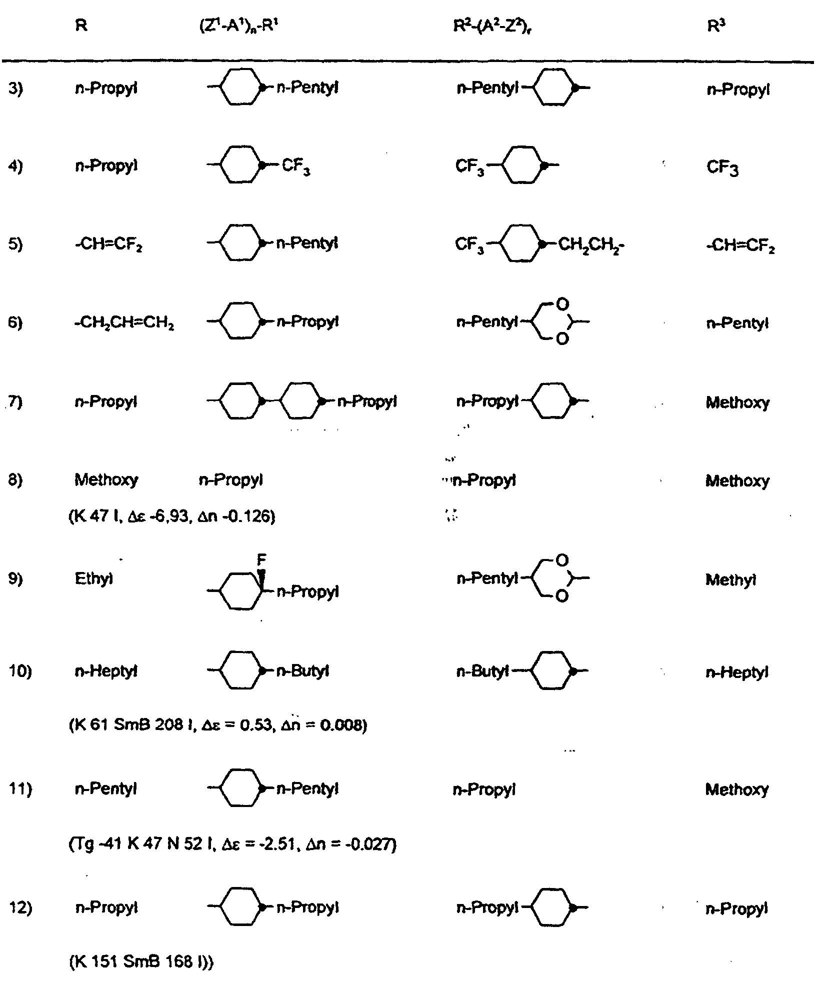

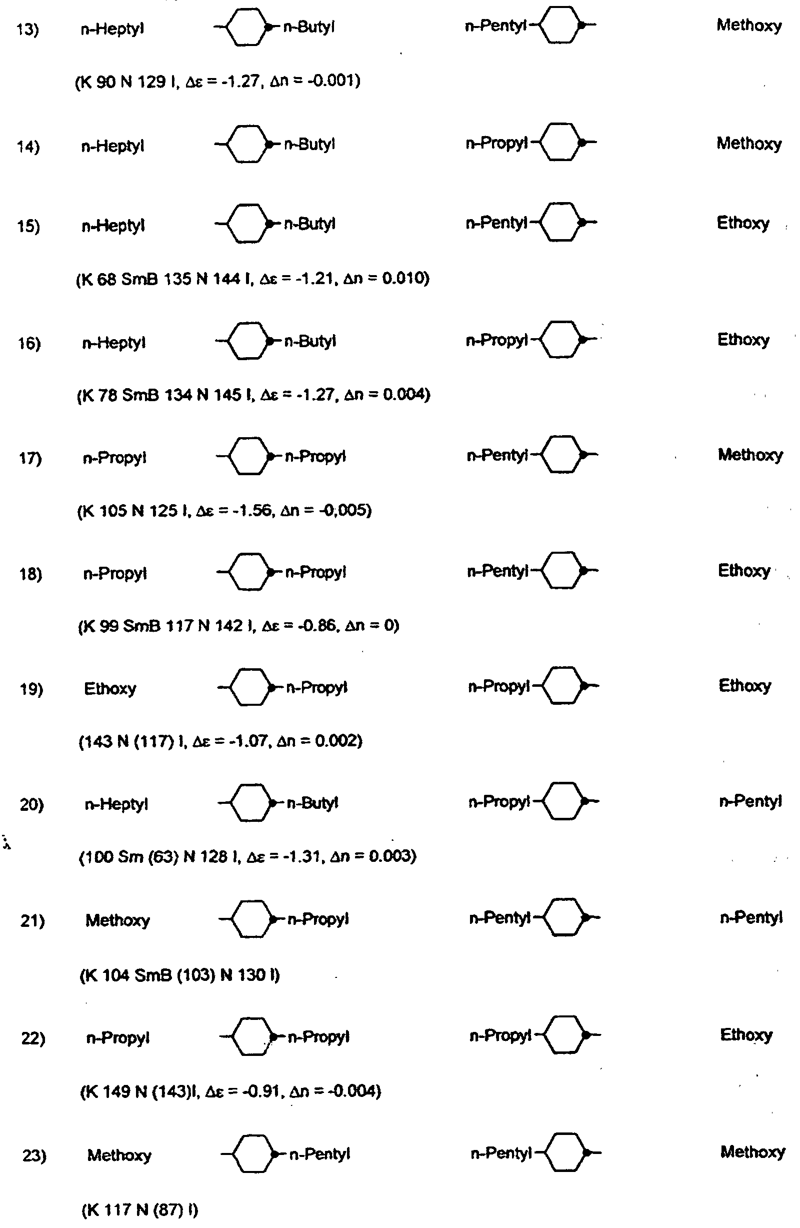

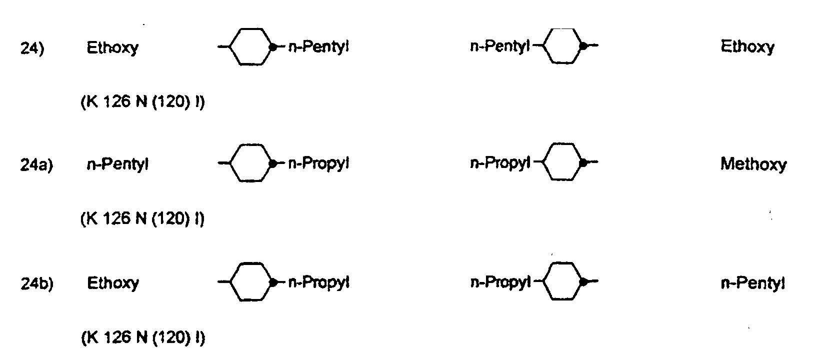

### Beispiele 25-31

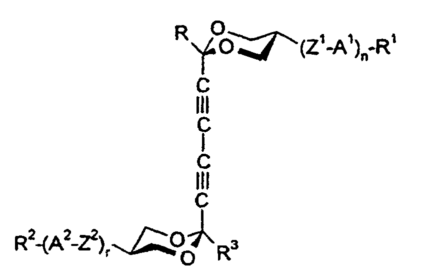

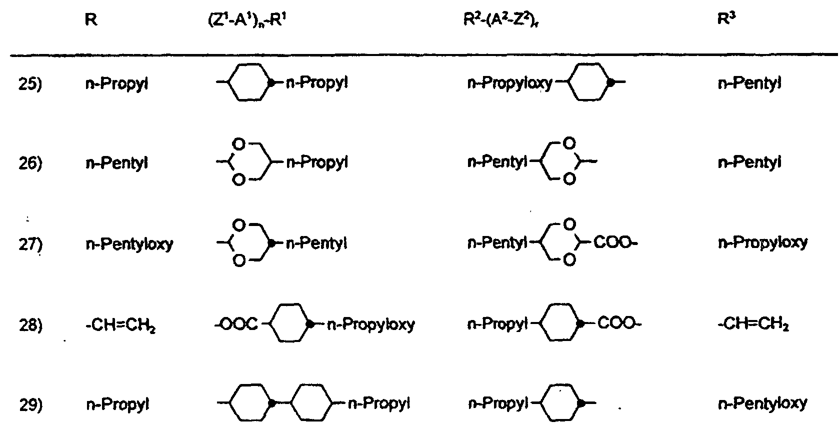

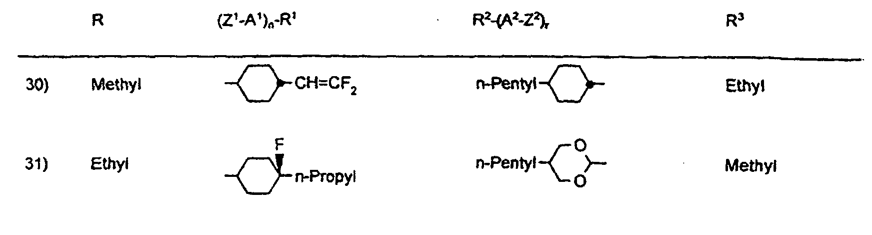

### Beispiele 32-39

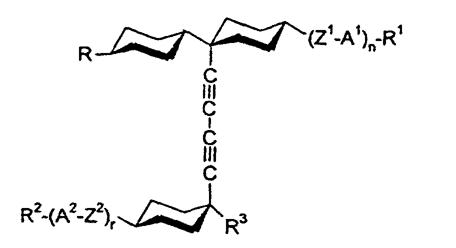

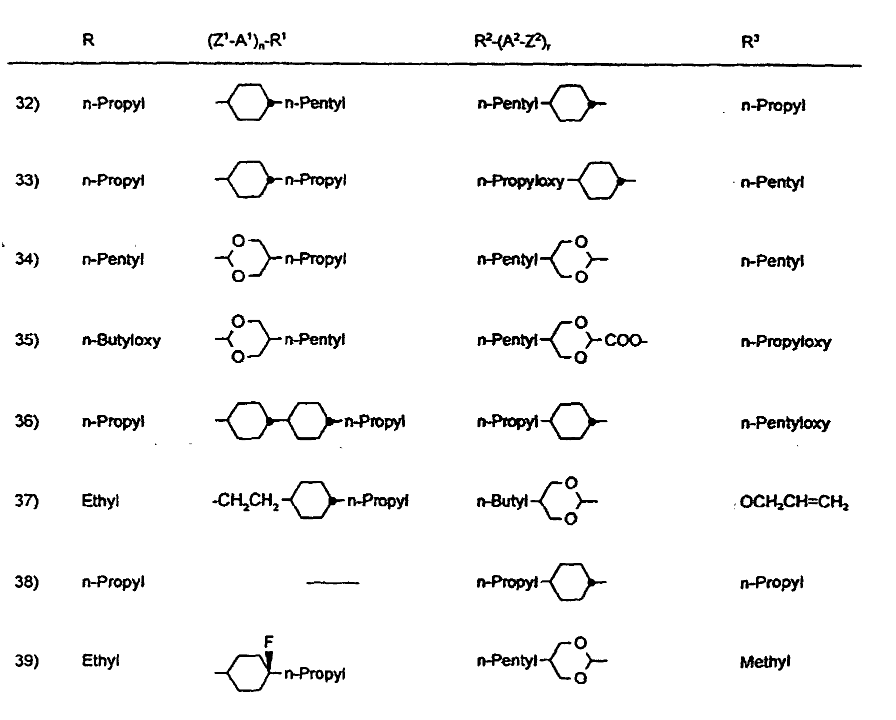

### Beispiele 40-45

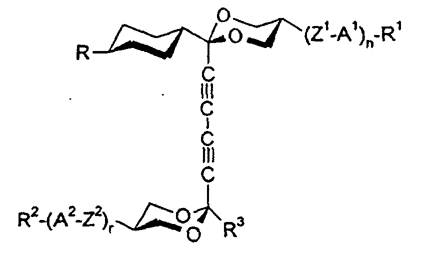

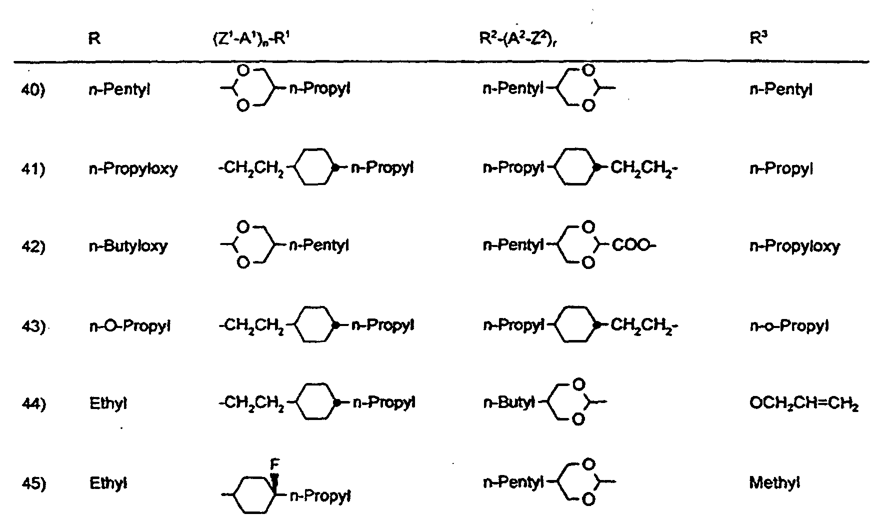

### Beispiele 46-50

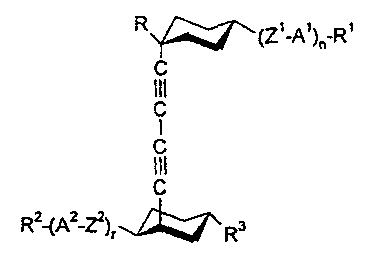

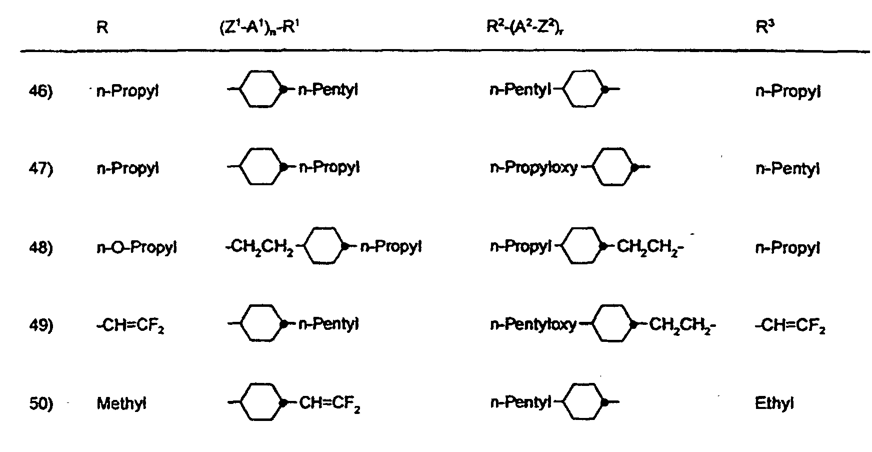

### Beispiele 51-53

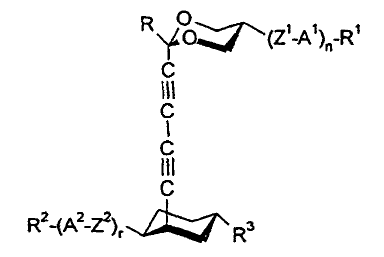

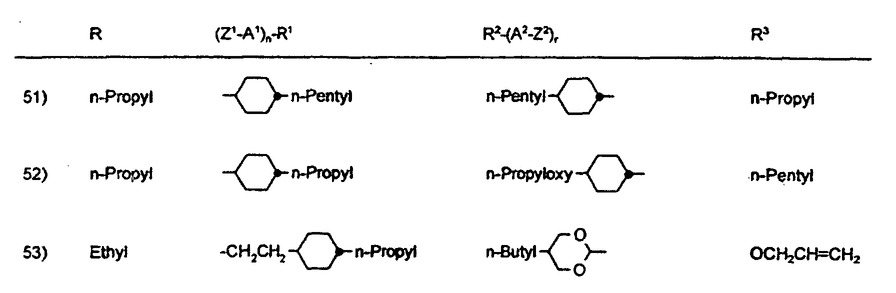

### Beispiele 54-56

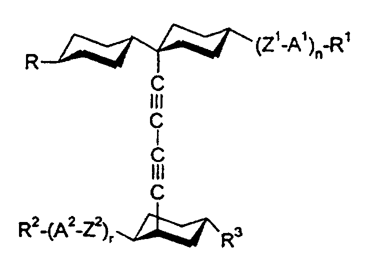

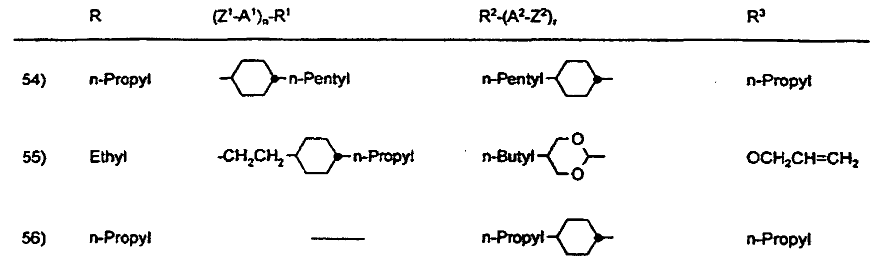

### Beispiele 57-62

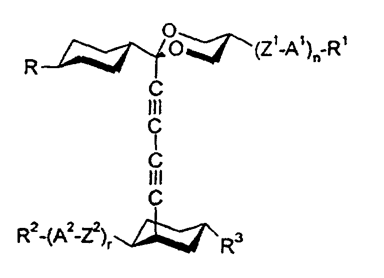

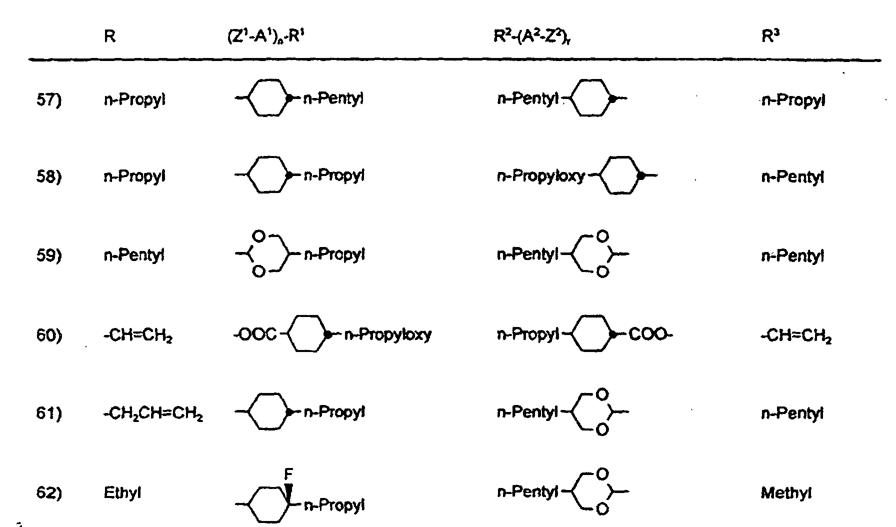

### Beispiele 62-67

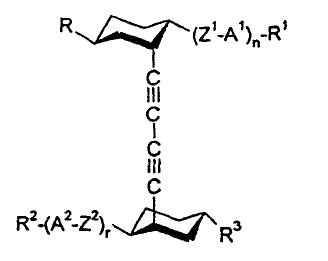

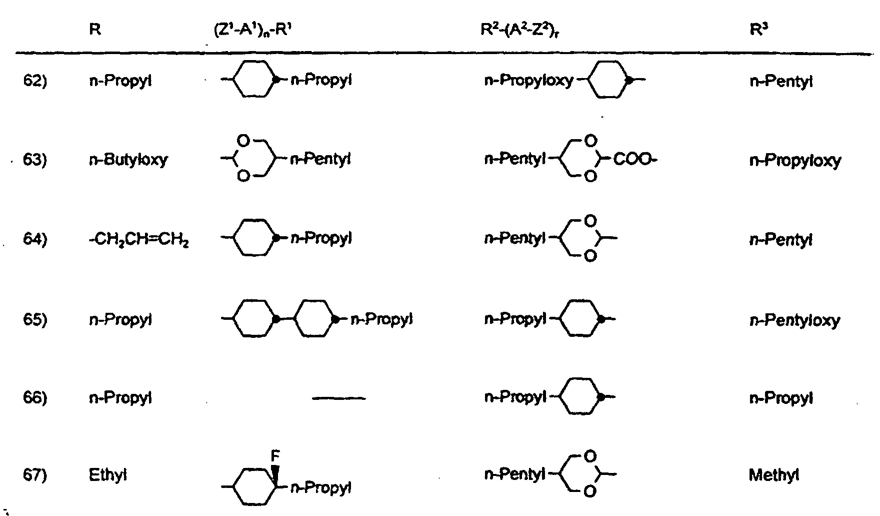

### Beispiele 68-72

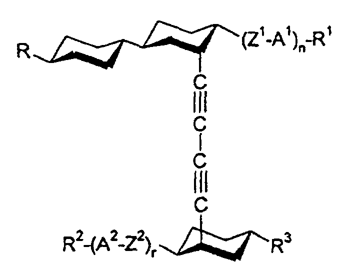

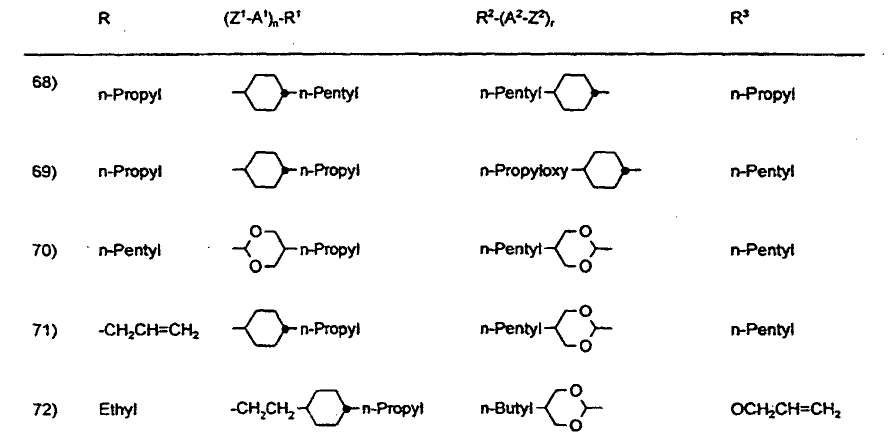

### Beispiele 73-81

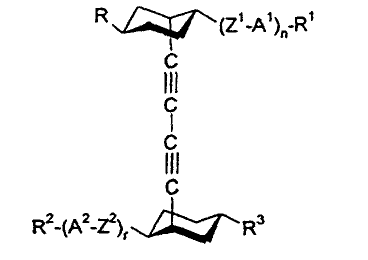

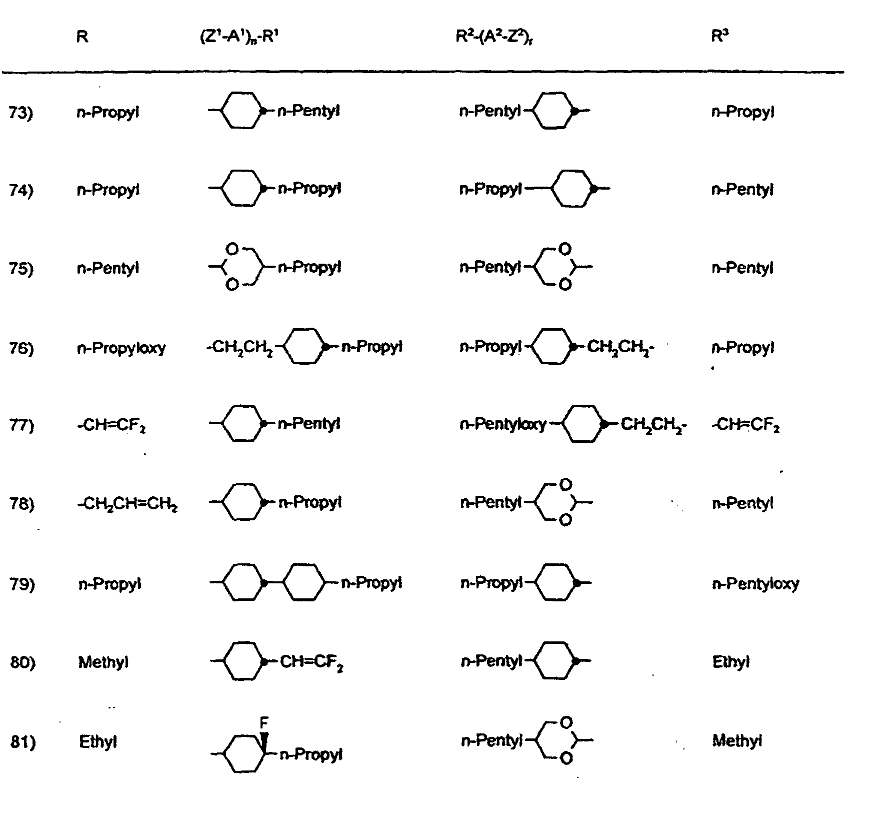

### Beispiele 81-85

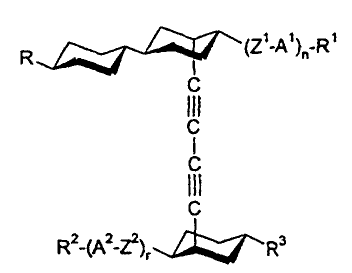

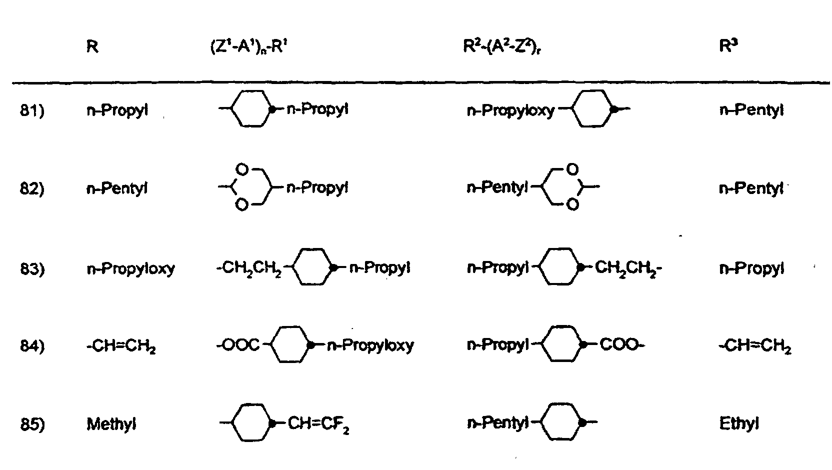

### Beispiel 86

### 4-[4-(4,4'-Dipentylbicyclohexyl-4-ylethinyl)-2-fluorphenylethinyl]-4,4'-dipentylbicyclohexyl

0.908 g 4-(4-Brom-2-fluorphenylethinyl)-4,4'-dipentylbicyclohexyl (erhältlich durch Wittig-Reaktion von 4,4'Dipentylbicyclohexyl-4-carbaldehyd mit 4-Brom-2-fluorbenzyltriphenylphoniumbromid, Addition von Brom an das erhaltene Alken und anschließende Dehydrobromierung mit einer Base), 0.715 g 4-Ethinyl-4,4'-dipentylbicyclohexyl und 0.104 g Tetrakis(triphenylphosphin)-palladium(0) werden in 5 ml Pyrrolidin über Nacht bei 80°C gerührt. Durch übliche Aufarbeitung wird 4-[4-(4,4'-Dipentylbicyclohexyl-4-ylethinyl)-2-fluorphenylethinyl]-4,4'-dipentylbicyclohexyl erhalten (K177I).

### Beispiel 87

### 4-[4-(4,4'-Dipentylbicyclohexyl-4-ylethinyl)-3-fluorphenylethinyl]-4-methoxy-4'-propylbicyclohexyl

Analog zu Beispiel 4 werden 1.913 g 4-(4-Brom-2-fluorphenylethinyl)-4,4'-dipentylbicyciohexyl mit 0.997 g 4-Ethinyl-4-methoxy-4'-propylbicyclohexyl in Gegenwart von 0.219 g Tetrakis(triphenylphosphin)-palladium(0) und 5 ml Pyrrolidin umgesetzt, wodurch 4-[4-(4,4'-Dipentylbicyclohexyl-4-ylethinyl)-3-fluorphenylethinyl]-4-methoxy-4'-propylbicyclohexyl erhalten wird (K 117 N (36) I, Δε -0.26, Δn -0.015).

Analog werden aus den entsprechenden Vorstufen die folgenden erfindungsgemäßen Verbindungen erhalten:

### Beispiel 88

### 4[4-(4,4'-Dipentybicyclohexyl-4-ylethinyl)-fluorphenyl-ethinyl]-4-methoxy-4'-propylbicyclohexyl

Analog zu Beispiel 87 wird die Titelverbindung unter Verwendung von 4-(4-Brom-3-fluorphenyl-ethinyl)4,4'-o-pentylbicyclohexyl erhalten (K 120 N (34) I, Δε=-1.22, Δn = -0.015).

### Beispiele 89-92

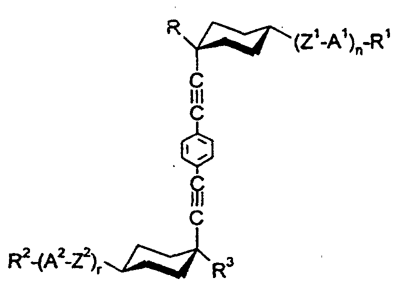

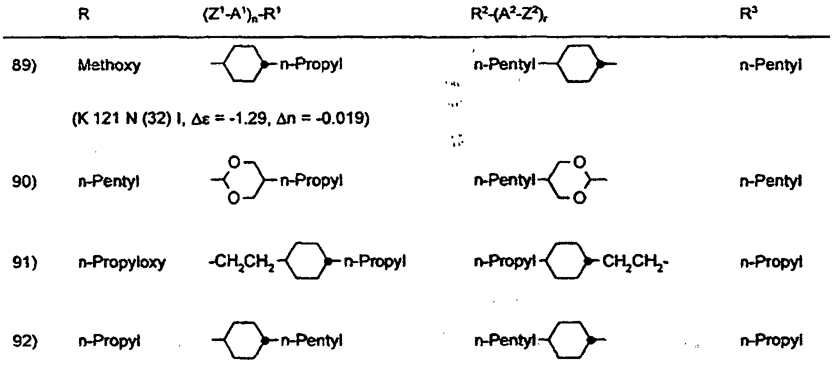

### Beispiele 93-94

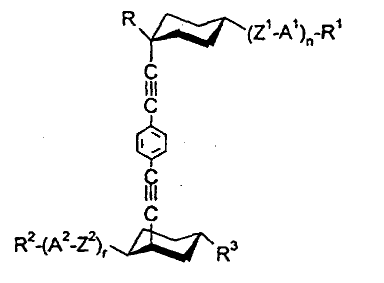

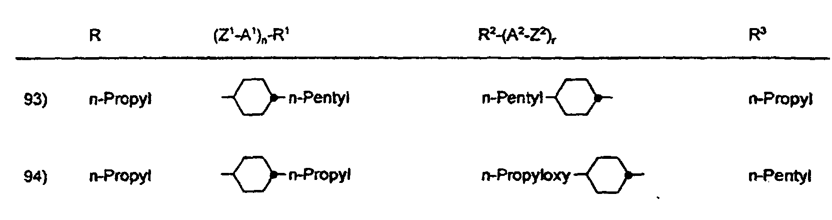

### Beispiel 95

### 4-Propylcyclohexancarbonsäure-1-(4,4'-dipentylbiclyclohexyl-4-ylethinyl)-4-pentylcyclohexylester

a) 1-(4,4'-Dipentylbicyclohexyl-4-ylethinyl)-4-pentylcyclohexanol
   6.00 g 4-Ethinyl-4,4'dipenthylbicyclohexyl werden in 50 ml THF vorgelegt und bei -20 °C 9.66 ml einer 15%igen Lösung von Butyllithium in Hexan zugetropft. Nach 10 Minuten werden 2.66 g 4-Pentylcyclohexanon in 10 ml THF zur Reaktionsmischung zugetropft. Man läßt auf RT erwärmen, säuert mit verdünnter Salzsäure an und arbeitet wie üblich auf, wodurch 1-(4,4'Dipentylbicyclohexyl-4-yl-ethinyl)-4-pentylcyclohexanol erhalten wird.
b) 4-Propylcyclohexancarbonsäure-1-(4,4'dipentylbicyclohexyl-4-ylethinyl)-4-pentylcyclohexylester
   1.10 g 1-(4,4'-Dipentylbicyclohexyl-4-ylethinyl)-4-pentylcyclohexanol und 0.394 g 4-Propylcyclohexancarbonsäure werden in 4 ml Dichlormethan gelöst und mit 0.283 g 4-(Dimethylamino)pyridin versetzt. Zur Reaktionsmischung werden danach 5.569 g N,N-Dicyclohexylcarbodiimid in 3 ml Dichlormethan gelöst getropft. Man rührt über Nacht und arbeitet wie üblich auf, wodurch 4-Propylcyclohexancarbonsäure-1-(4,4'-dipentylbicyclohexyl-4-ylethinyl)-4-pentylcyclohexylester erhalten wird (K 66 SmB 171 I, Δε -0.02).
   Analog erhält man aus den entsprechenden Vorstufen die folgenden erfindungsgemäßen Verbindungen:

### Beispiele 96-102

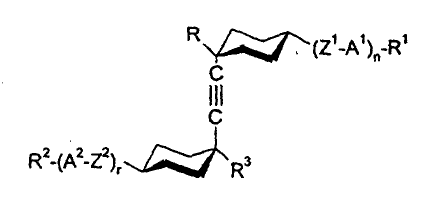

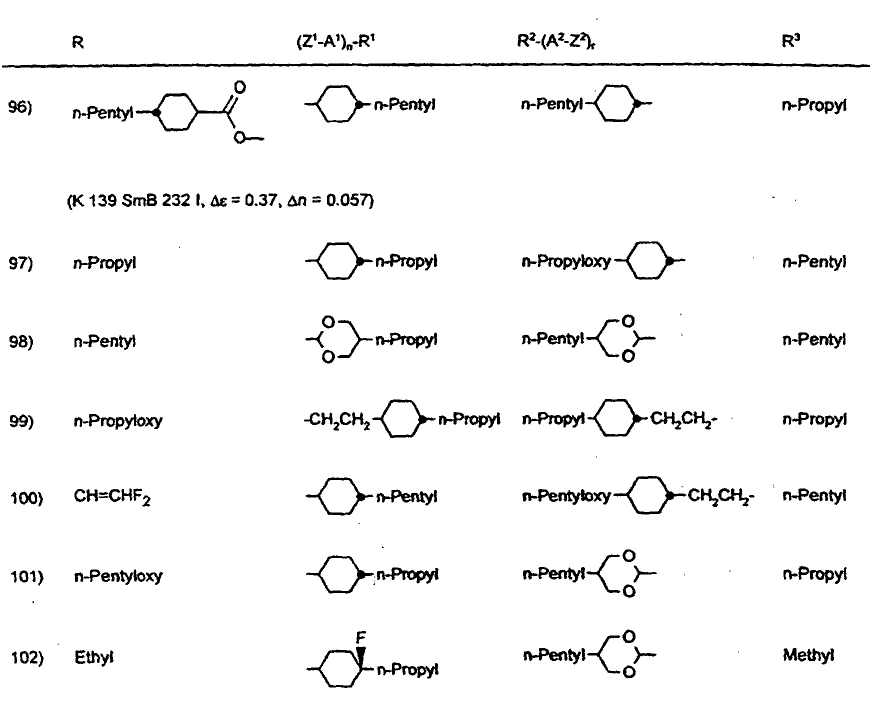

### Beispiele 103-108

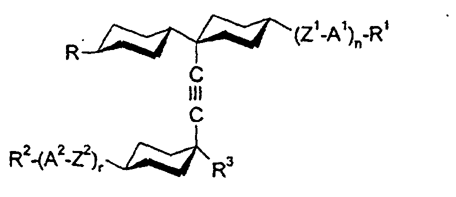

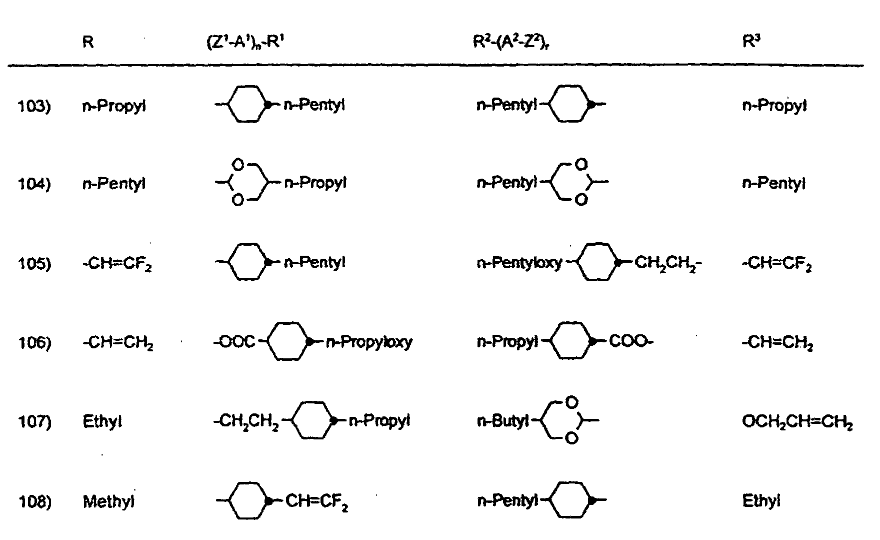

### Beispiele 109-114

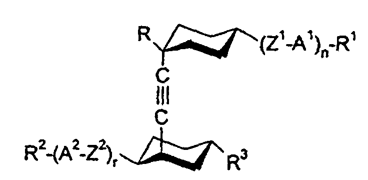

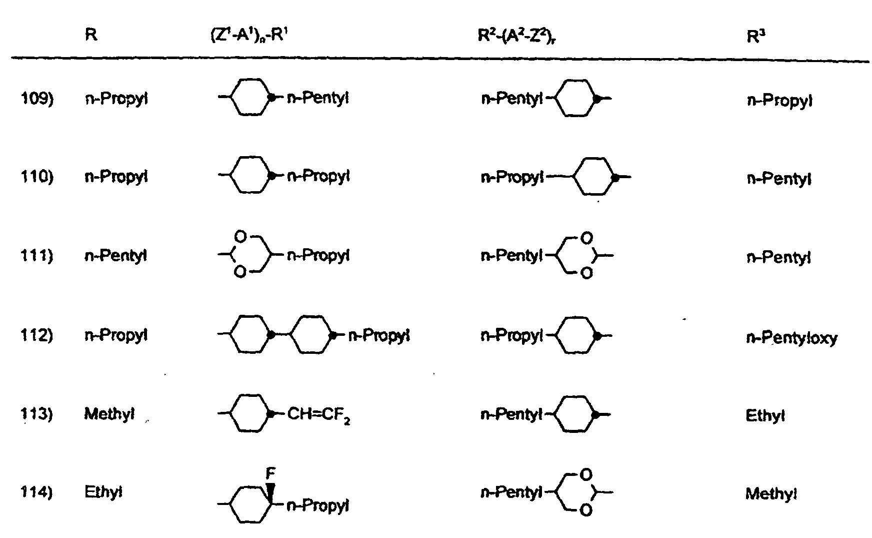

### Beispiele 115-118

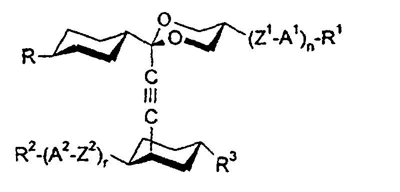

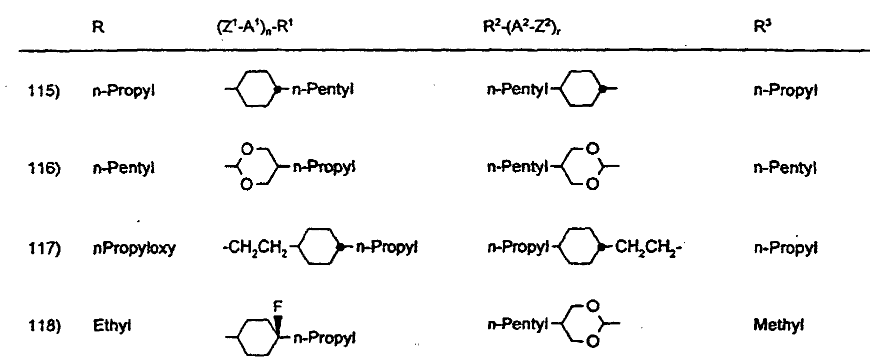

### Beispiele 119-125

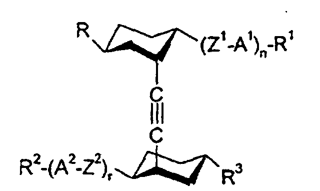

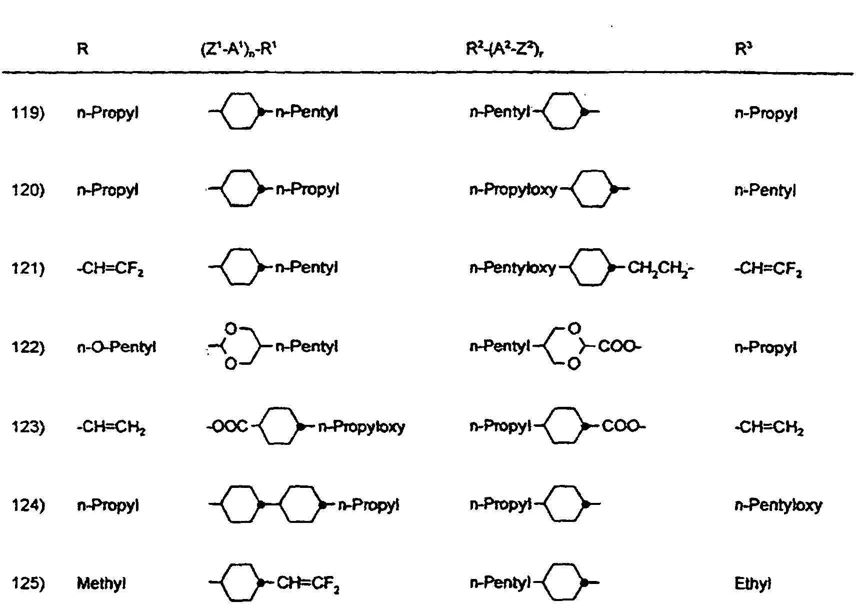

### Beispiele 126-131

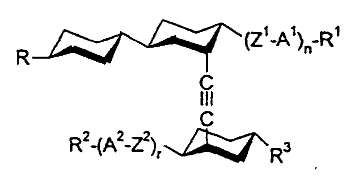

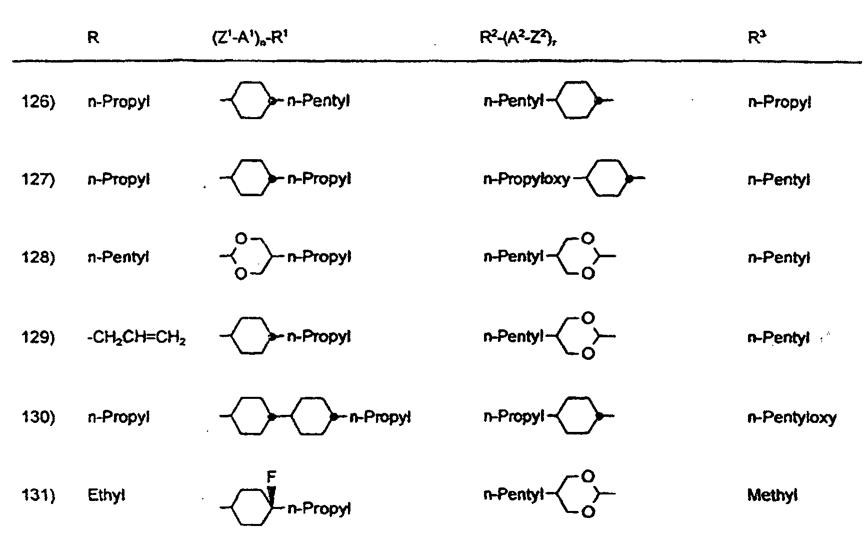

### Beispiel 132-135

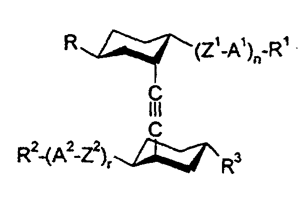

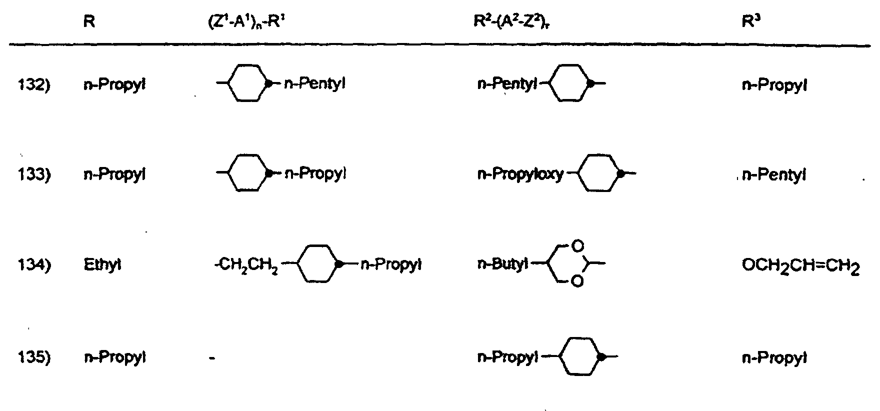

### Beispiele 136-140

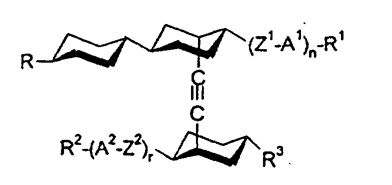

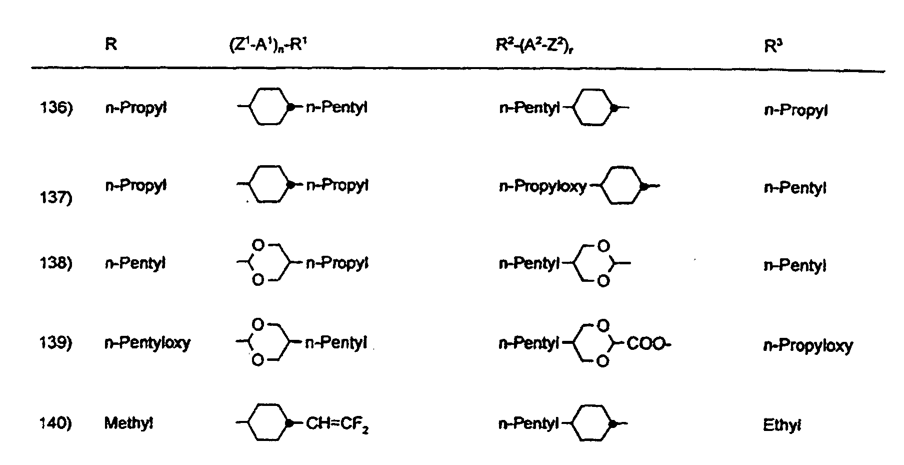

### Vergleichsbeispiele 141-146

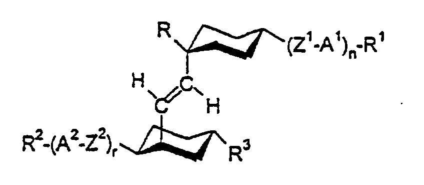

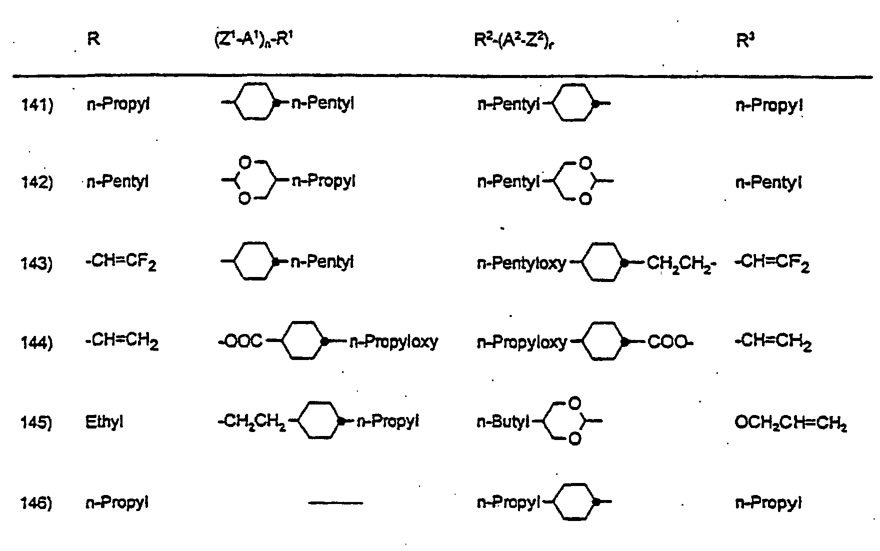

### Beispiele 147-152

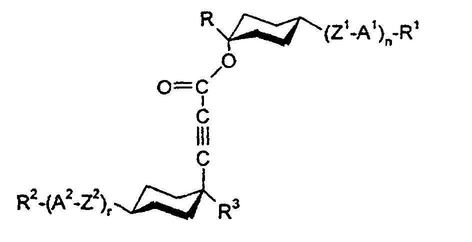

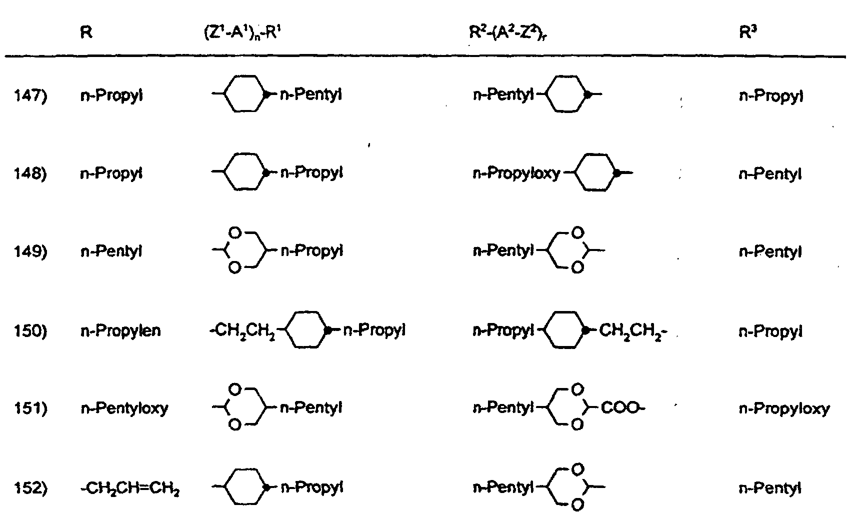

### Beispiele 153-157

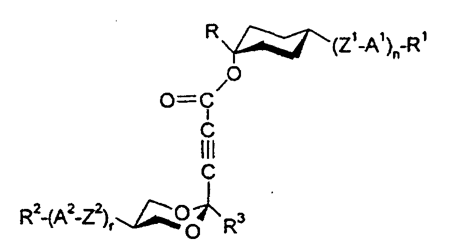

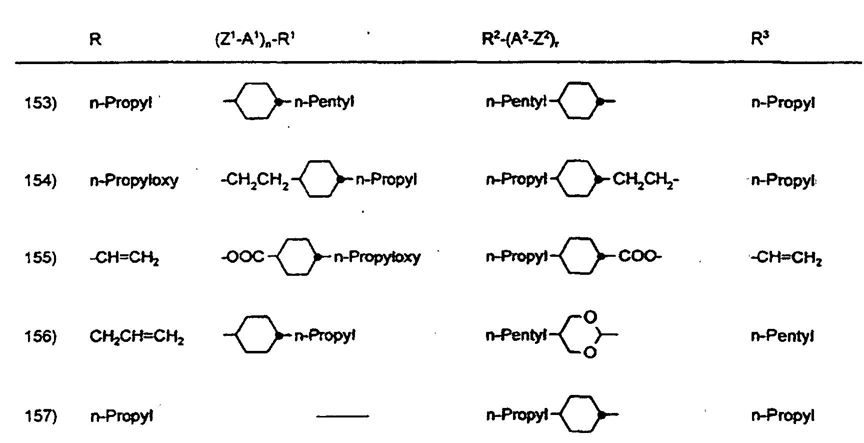

### Beispiele 157-164

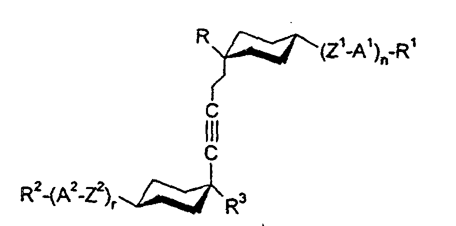

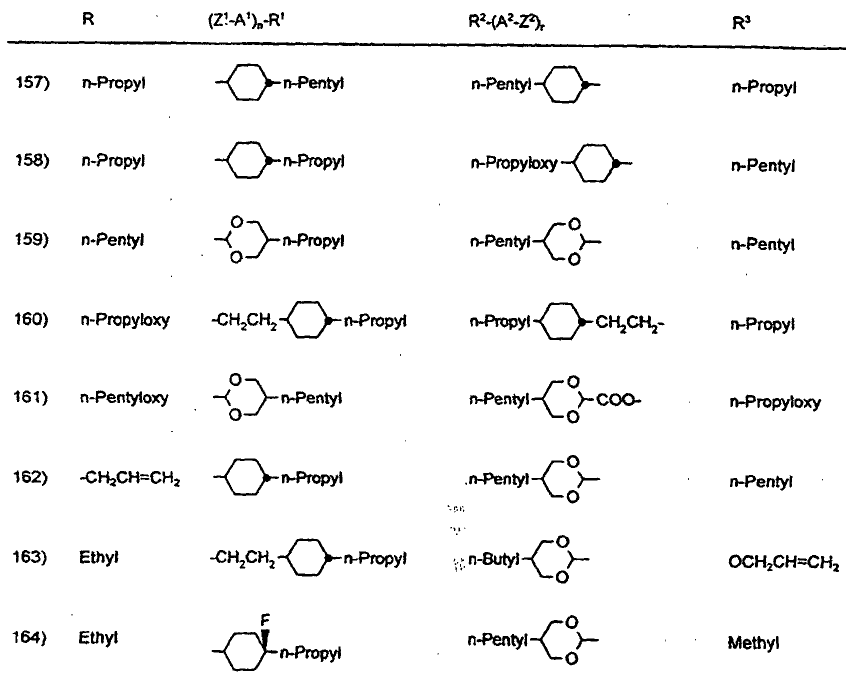

### Beispiele 165-168

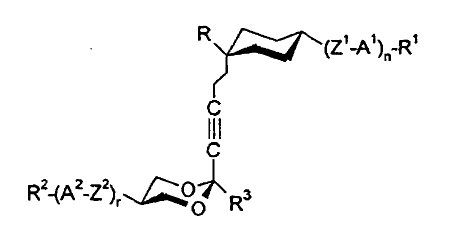

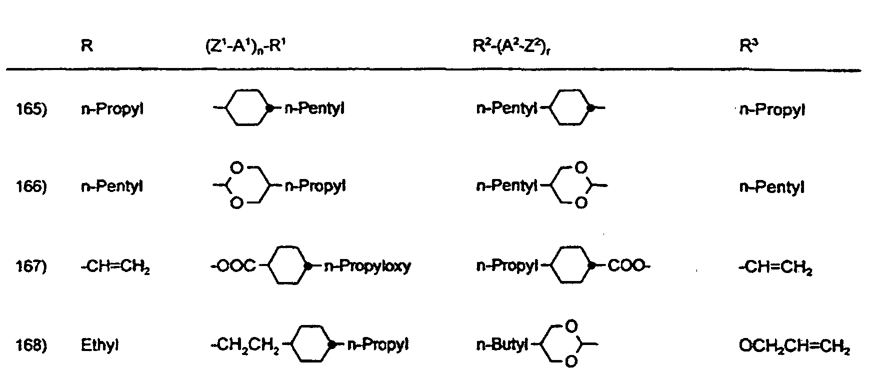

### Beispiele 169-173

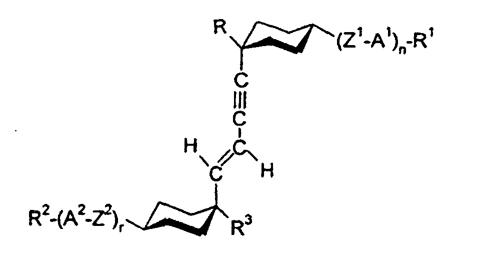

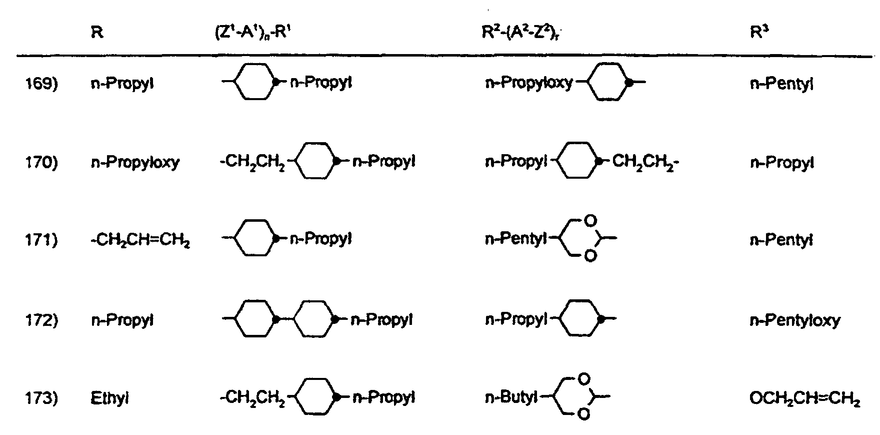

### Beispiele 174-176

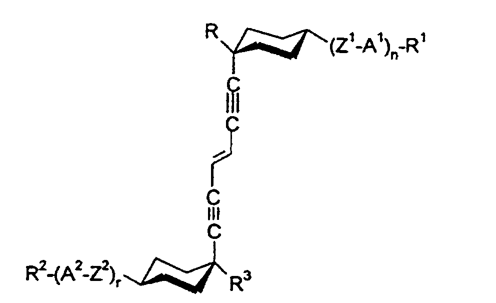

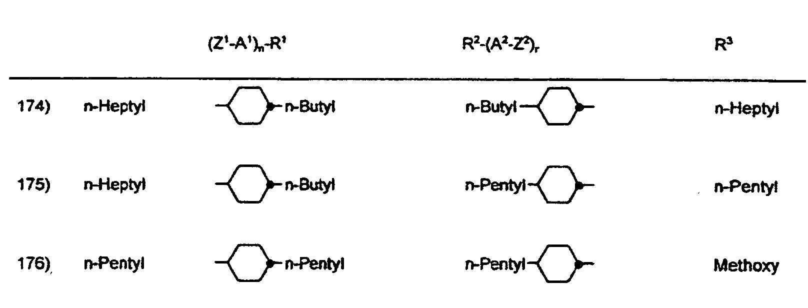

### Beispiele 177-184

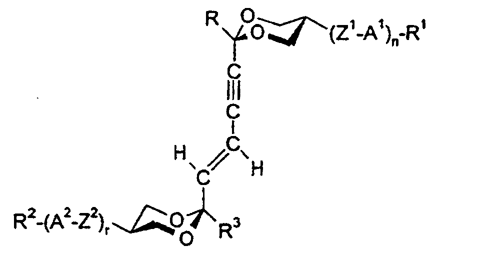

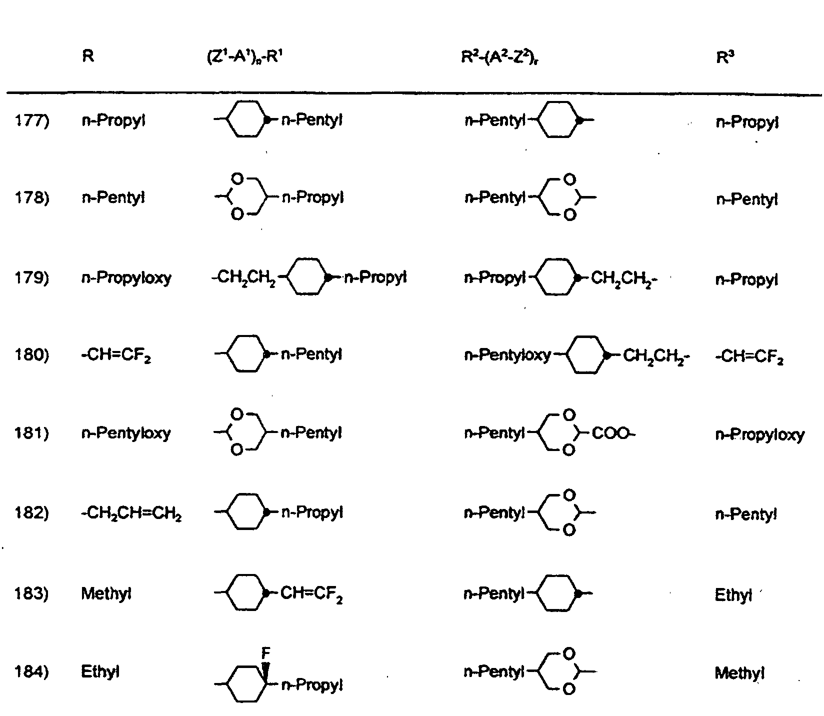

### Beispiele 185-190

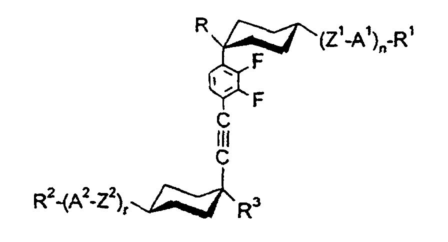

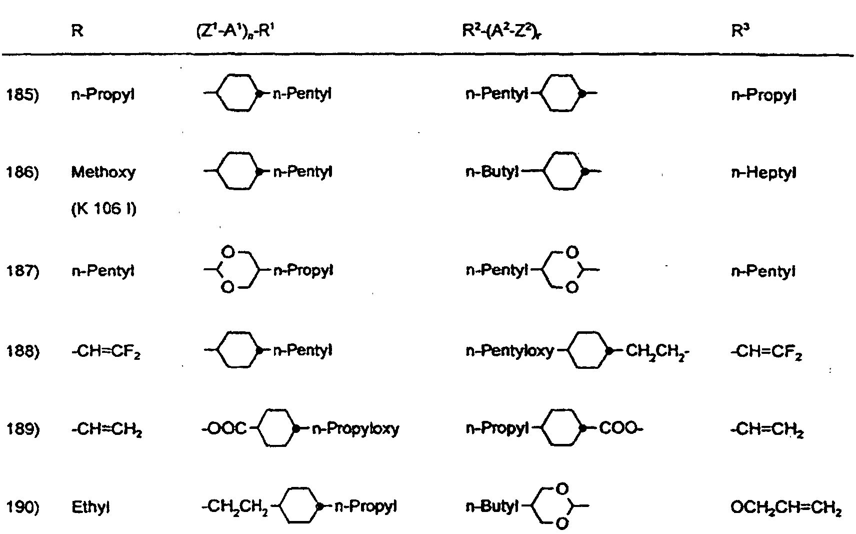

### Beispiele 191-193

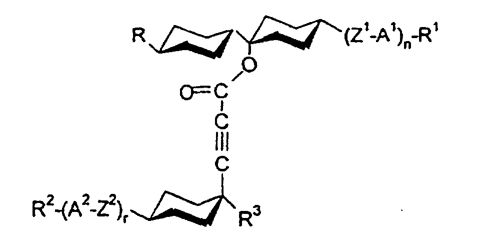

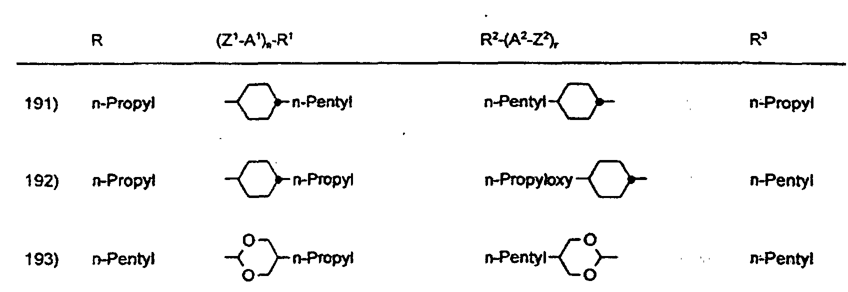

### Beispiele 194-198

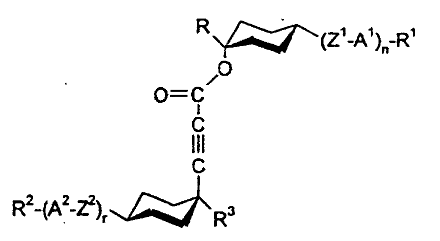

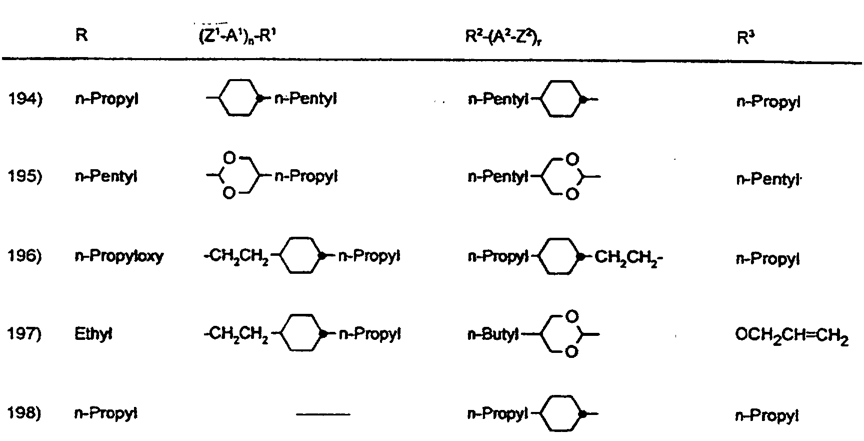

### Beispiele 199-203

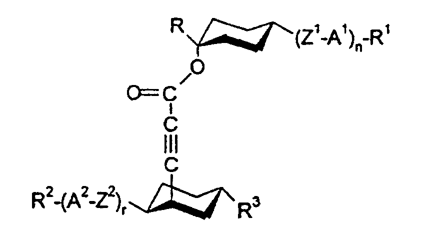

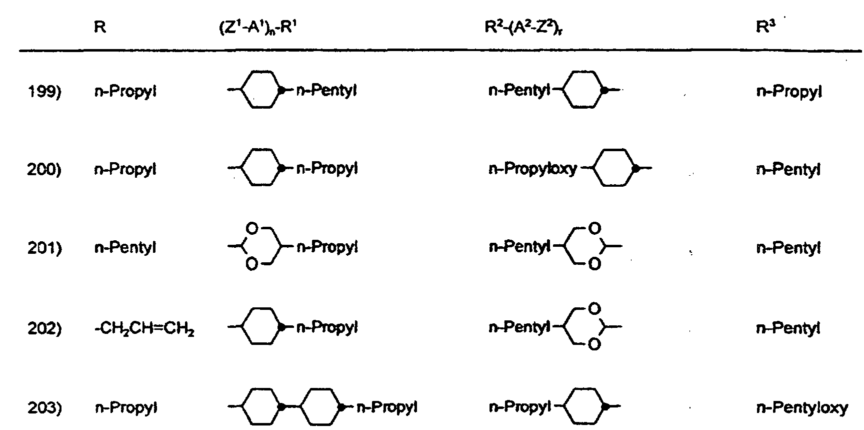

### Beispiele 204-208

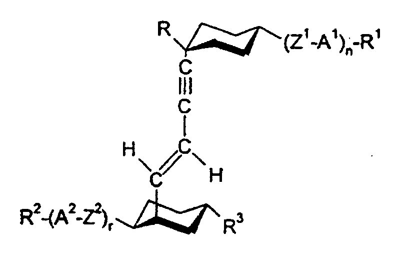

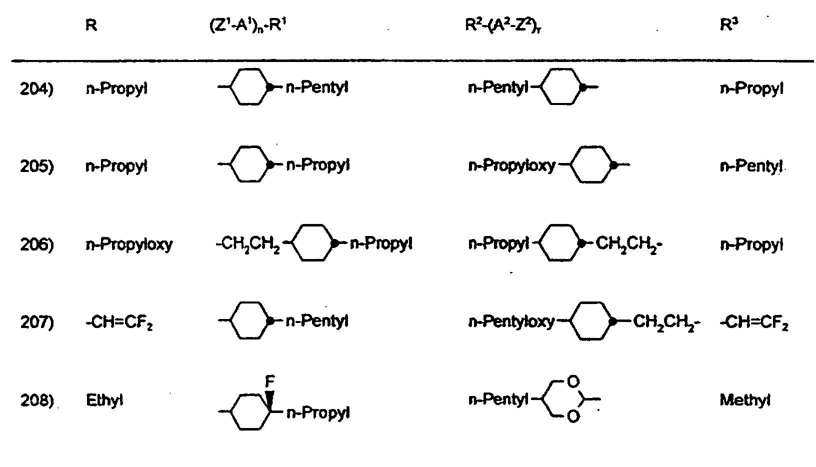

### Beispiele 209-212

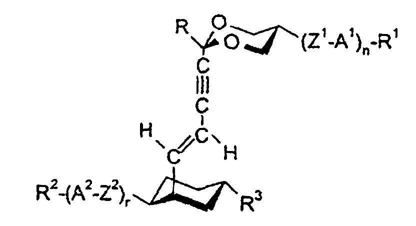

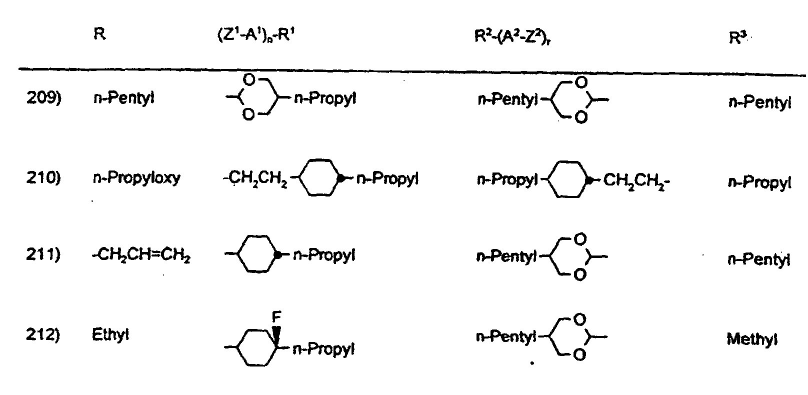

### Beispiele 213-218

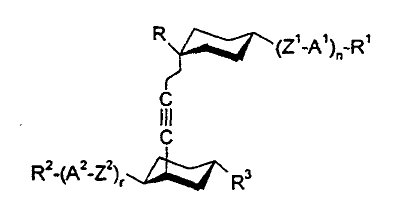

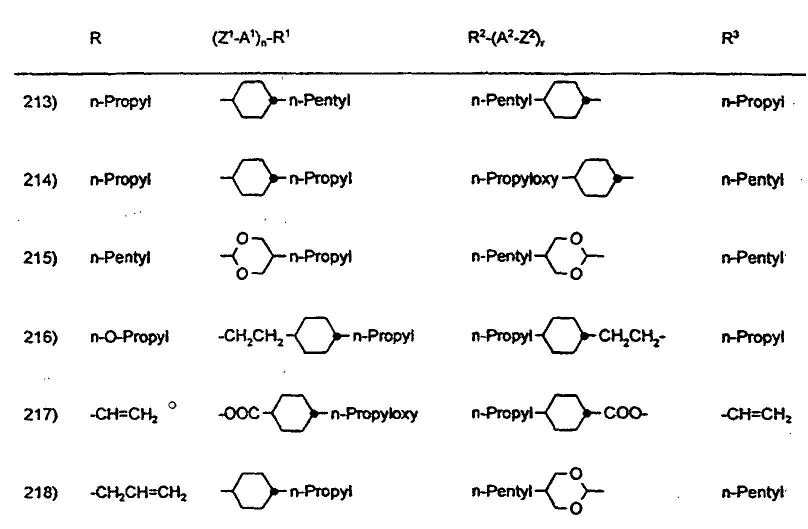

### Beispiele 219-221

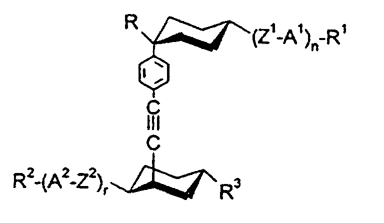

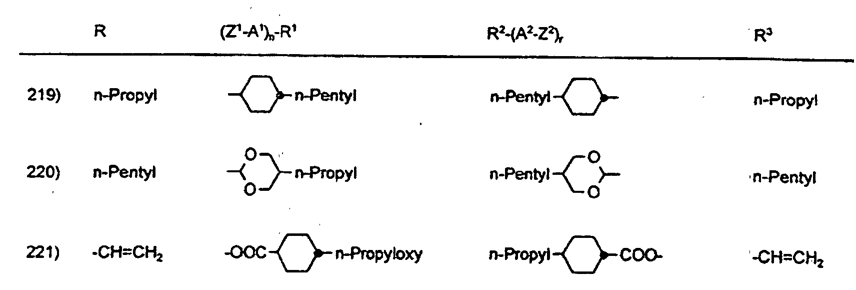

### Beispiele 222-226

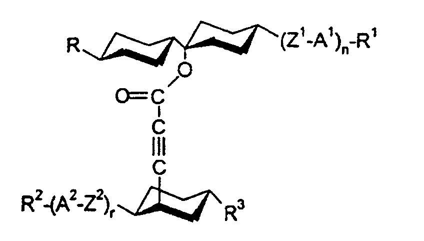

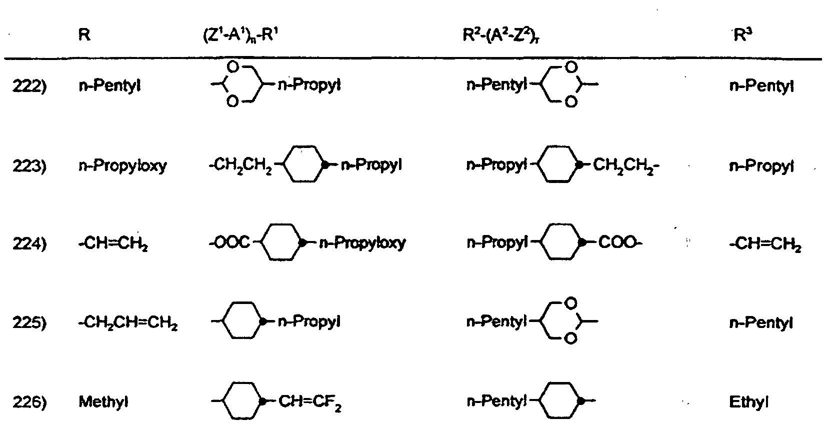

### Beispiele 227-230

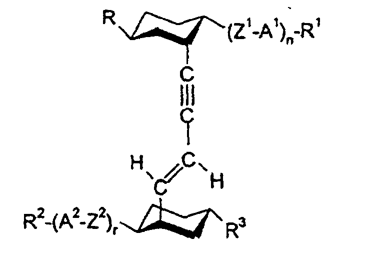

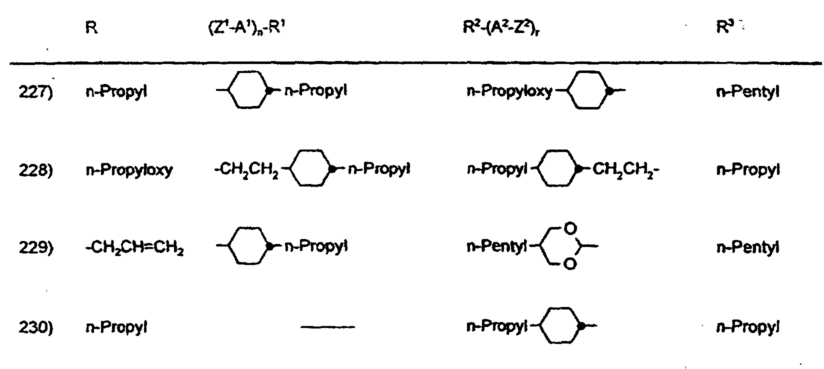

### Beispiele 231-234

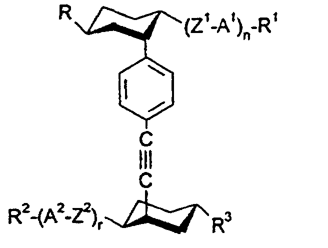

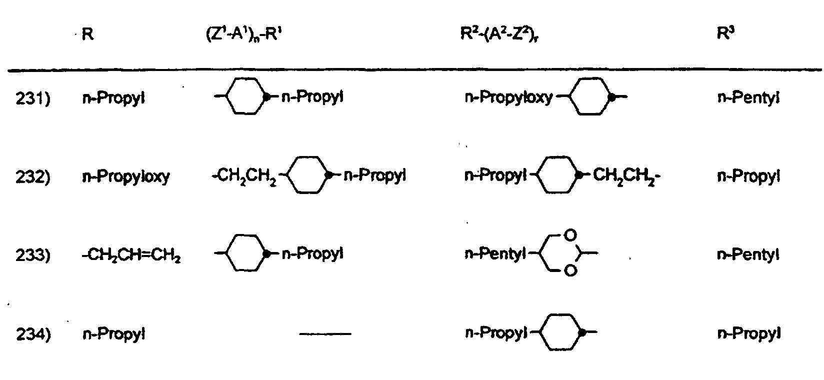

### Beispiele 235-238

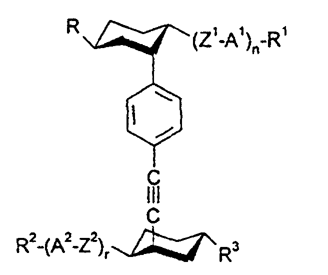

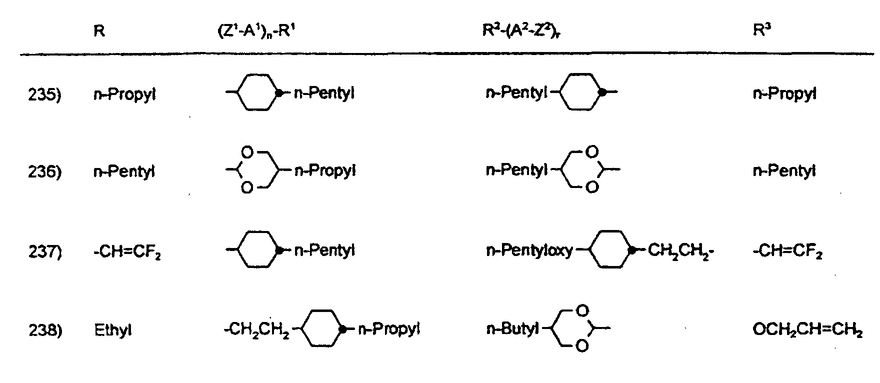

### Vergleichsbeispiele 239-241

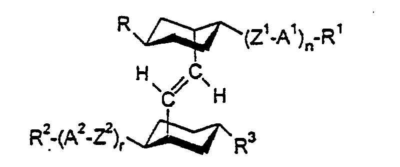

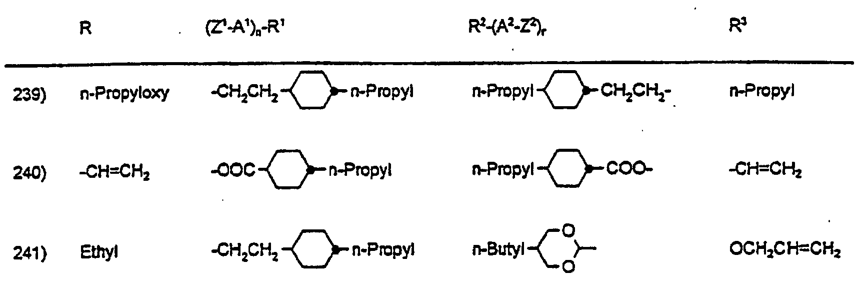

### Beispiele 242-245

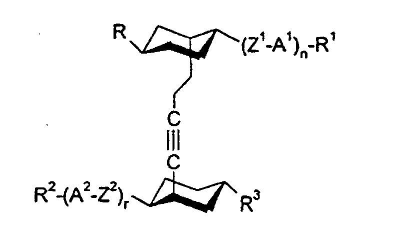

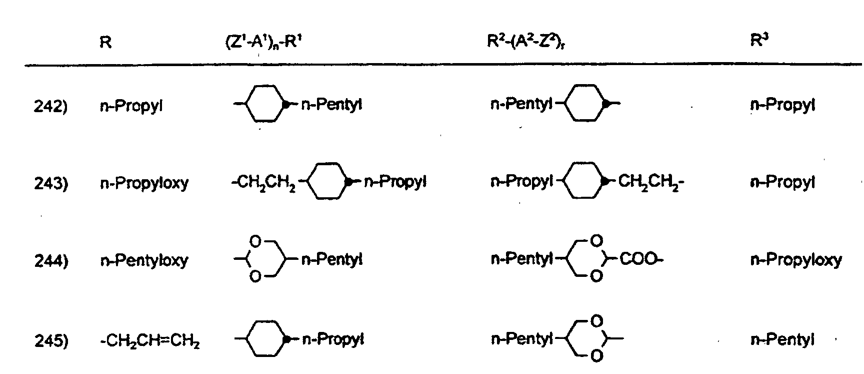

### Beispiel 246

### 1,6-Bis-(4,4'-dipentylbicyclohexyl-4-yl)-hexa-1,3,5-triin

a) 1,6-Bis-(4,4'-dipentylbicyclohexyl-4-yl)-hexa-2,4-diin-1,6-diol
   Zu einer Lösung von Natriumamid in flüssigem Ammoniak (hergestellt aus 9.2 g Natrium) werden 12.3 g Dichlorbutin in 20 ml Diethylether getropft. Nach 5 min wird die Lösung mit 58.55 g 4,4'-Dipentylbicyclohexyl-4-carbaldehyd in 10 ml Diethylether versetzt und die Reaktionsmischung nach 1 h mit Ammoniumchlorid zersetzt. Nach Verdampfen des Ammoniaks wird in Diethylether aufgenommen und wie üblich aufgearbeitet, wodurch 1,6-Bis-(4,4'-dipentylbicyclohexyl-4-yl)-hexa-2,4-diin-1,6-diol erhalten wird.
b) 1,6-Bis-(4,4'-dipentylbicyclohexyl-4-yl)-1,6-dichlorhexa-2,4-diin
   19.42 g 1,6-Bis-(4,4'-dipentyl-bicyclohexyl-4-yl)-hexa-2,4-diin-1,6-diol werden unter Kühlung mit 5 ml Thionylchlorid versetzt. Nach 2 h bei RT wird für 1 h auf 50°C erwärmt anschließend mit Eis zersetzt und wie üblich aufgearbeitet, wodurch das Produkt erhalten wird.
c) 1,6-Bis-(4,4'-dipentylbicyclohexyl-4-yl)-hexa-1,3,5-triin
   8.32 g 1,6-Bis-(4,4'-dipentylbicyclohexyl-4-yl)-1,6-dichlorhexa-2,4-diin werden in 30 ml Diethylether gelöst und zu einer Lösung von Natriumamid in flüssigem Ammoniak (hergestellt aus 1 g Natrium) getropft. Nach 30 min wird mit Ammoniumchlorid das überschüssige Amid zersetzt und nach dem Verdampfen des Ammoniaks der Rückstand wie üblich aufgearbeitet, wodurch 1,6-Bis-(4,4'-dipentylbicyclohexyl-4-yl)-hexa-1,3,5-triin erhalten wird (K 90 N (63) I, Δε = -1.56, Δn = -0.028).

Analog lassen sich unter Verwendung der entsprechenden Vorstufen die folgenden erfindungsgemäßen Verbindungen erhalten:

### Beispiele 247-252

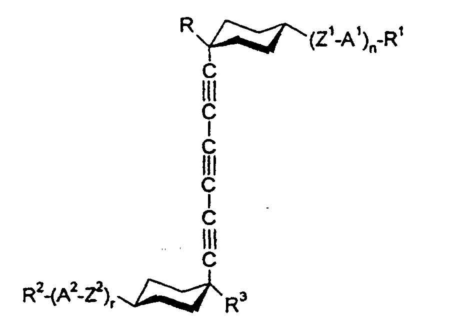

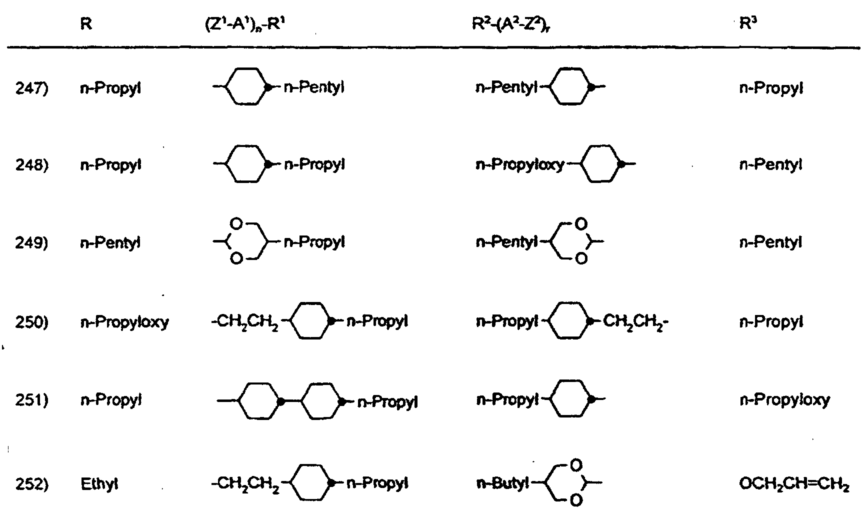

### Beispiele 253-255

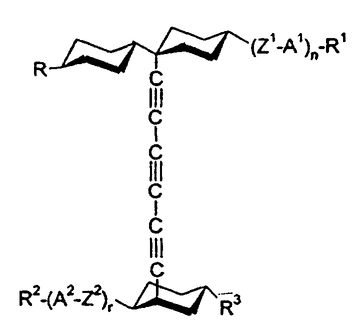

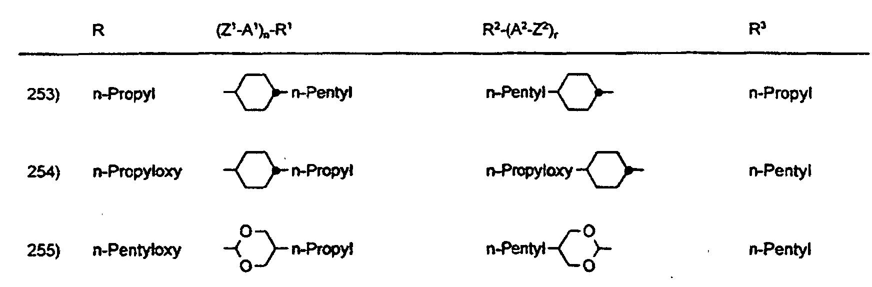

### Beispiele 256-259

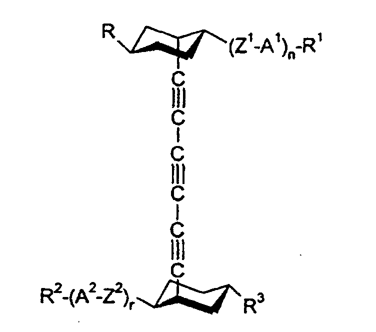

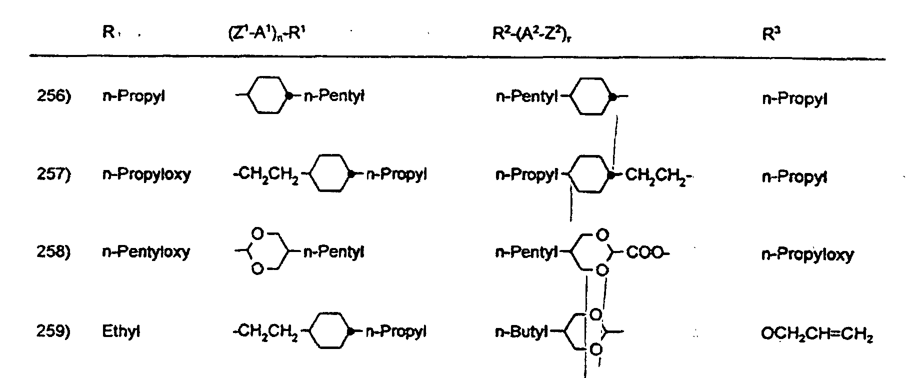

Analog lassen sich unter Verwendung der entsprechenden Vorstufen die folgenden erfindungsgemäßen Verbindungen erhalten:

### Beispiele 260-265

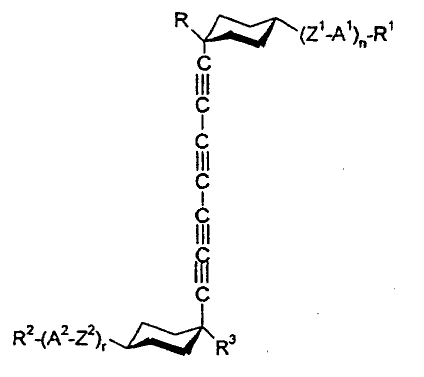

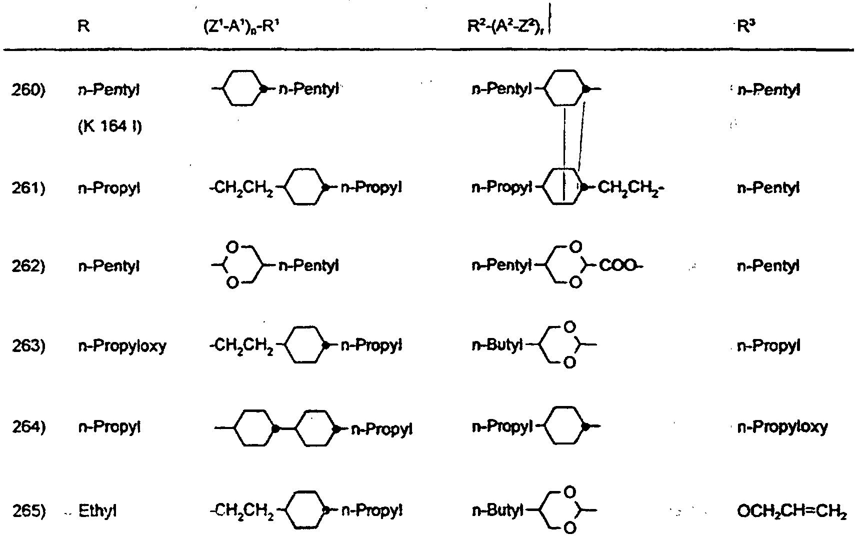

### Beispiel 266

Die Verbindung aus Beispiel 11 weist bei einer Meßtemperatur von 49.4 °C in Abhängigkeit der Wellenlänge λ des angewandten Lichts folgende Werte von n_{II}, n_{⊥} und Δn auf:

| λ | n_{II} | n_{⊥} | Δn |
|---|---|---|---|
| 436 | 1.5188 | 1.5188 | 0 |
| 509 | 1.5121 | 1.5107 | 0.0014 |
| 546 | 1.5096 | 1.5077 | 0.0019 |
| 589 | 1.5073 | 1.5052 | 0.0021 |
| 632 | 1.5054 | 1.5029 | 0.0025 |

## Patentansprüche

1. Cyclohexan-Derivate der Formel I 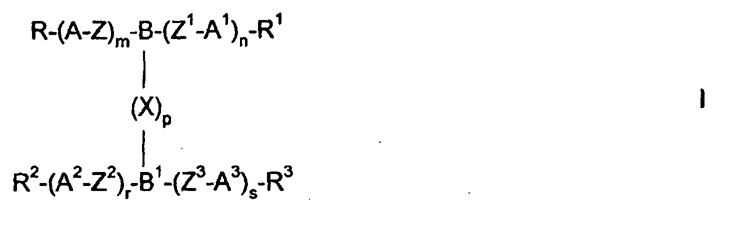 worin
B und B¹ unabhängig voneinander 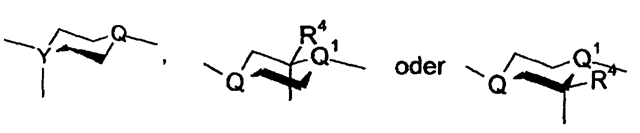
bedeuten,
wobei die Ringe B und B¹ jeweils über die axiale Bindung an die Gruppe (X)ₚ gebunden sind und in diesen Ringen auch ein oder zwei nicht benachbarte CH₂-Gruppen durch O ersetzt sein können,
Y bei mehrmaligem Auftreten unabhängig voneinander C oder Si,
Q, Q¹ unabhängig voneinander CH oder SiH
X bei mehrmaligem Auftreten unabhängig voneinander -C≡C-, -CH=CH-, -CF=CF-, -CF=CH-, -CH₂-, -COO- oder 1,4-Phenylen, worin auch eine oder mehrere CH-Gruppen unabhängig voneinander durch N oder CF ersetzt sein können, jedoch mit der Maßgabe, dass mindestens ein X -C≡C- ist,
p 1, 2, 3 oder 4,
R, R¹, R², R³, R⁴ unabhängig voneinander H, einen unsubstituierten, einen mindestens einfach durch Halogen substituierten Alkylrest mit 1-12 C-Atomen, worin auch eine oder mehrere CH₂-Gruppen jeweils unabhängig voneinander durch -O-, -S-, -CO-, 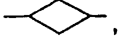 -CO-O-, -O-CO-, -O-CO-O- oder -CH=CH- so ersetzt sein können, daß Heteroatome nicht direkt verbunden sind, -CN, -F, -CF₃, -OCHF₂, -OCF₃, -OCHFCF₃, -OCH₂CF₃, -OCF₂CF₃ oder -CH=CF₂ bedeuten,
A, A¹, A² und A³ unabhängig voneinander 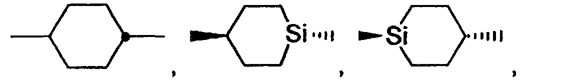 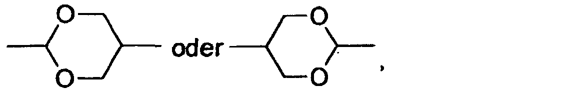 oder einen 1,4-Phenylenrest, worin auch eine oder zwei CH-Gruppen durch N ersetzt sein können, wobei die Ringe A, A¹, A² und A³ unabhängig von-einander durch eines oder mehrere F-Atome oder -NCS, -CN, -CF₃ oder -OCF₃ substituiert sein können,
Z, Z¹, Z², Z³ jeweils unabhängig voneinander -CO-O-, -O-CO-, -CH₂O-, -CH=CH-, -O-, -O-CH₂-, -CH₂CH₂-, -(CH₂)₃-, -(CH₂)₄-, -CF₂-CF₂-, -OCF₂- oder eine Einfachbindung
und
n, m, r, s unabhängig voneinander 0, 1 oder 2
bedeuten.

2. Cyclohexanderivate nach Anspruch 1, **dadurch gekennzeichnet**, dal sie bei einer reduzierten Temperatur von 0.9 und einer Wellenlänge von 589 nm einen Wert für die optische Anisotropie Δn von < 0,05 aufweisen.

3. Cyclohexanderivate nach Anspruch 1 oder 2, worin B und B¹ unabhängig voneinander 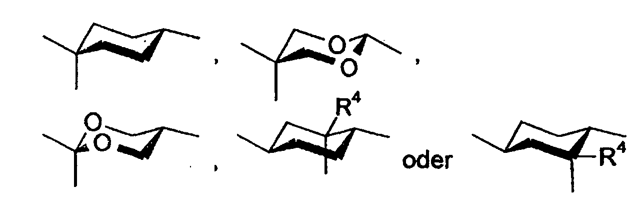 bedeuten, worin R⁴ die oben angegebene Bedeutung aufweist.

4. Cyclohexanderivate nach Anspruch 1, 2 oder 3, **dadurch gekennzeichnet, daß** Y C und Q und Q¹ CH bedeuten.

5. Cyclohexanderivate nach Anspruch 1, 2 ,3 oder 4, worin Z, Z¹, Z² und Z³ unabhängig voneinander -CH₂CH₂-, -COO-, -OOC- oder eine Einfachbindung bedeuten.

6. Cyclohexanderivate nach einem der Ansprüche 1 bis 5, **dadurch gekennzeichnet, daß** R, R¹, R² und R³ unabhängig voneinander -CN, -F, -OCF₃, -CF₃, -OCF₂CF₃, -CH=CF₂ , geradkettiges Alkyl oder Alkoxy mit 1 bis 10 C-Atomen oder Alkenyl oder Alkenyloxy mit 2 bis 10 C-Atomen bedeuten.

7. Cyclohexanderivate nach einem der Ansprüche 1 bis 6, **dadurch gekennzeichnet, daß** die Gruppen (X)ₚ eine der folgenden Bedeutungen aufweisen:
-C≡C-, -C≡C-C≡C, -C≡C-C≡C-C≡C-, -C≡C-C≡C-C≡C-C≡C-, -C≡C-CH=CH-, -C≡C-CH=CH-C≡C-, -CH=CH-C≡C-CH=CH-, -C≡C-COO-, -CH₂-CH₂-C≡C-, -CH₂-CH₂-C≡C-C≡C-, -CH₂-CH₂-C≡C-C≡C-,

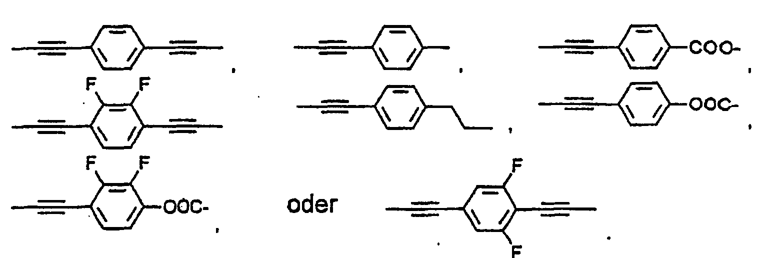

8. Cyclohexanderivate nach einem der vorhergehenden Ansprüche, worin A, A¹, A² und/oder A³ unabhängig voneinander eine der folgenden Bedeutungen annimmt:

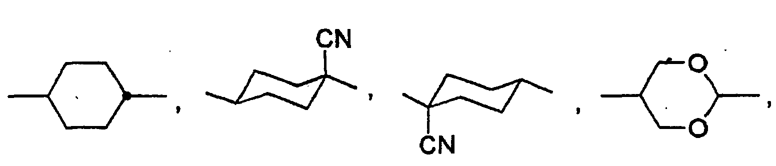

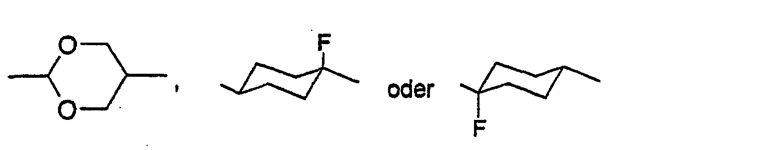

9. Cyclohexanderivate nach einem der vorhergehenden Ansprüche, worin m, n, r und s unabhängig voneinander 0 oder 1 bedeuten.

10. Verwendung von Verbindungen der Formel I nach einem der Ansprüche 1 bis 9 als Komponenten flüssigkristalliner Medien.

11. Flüssigkristallines Medium mit mindestens zwei flüssigkristallinen Komponenten, **dadurch gekennzeichnet, daß** es mindestens eine Verbindung der Formel I nach einem der Ansprüche 1 bis 9 enthält.

12. Flüssigkristall-Anzeigeelement, **dadurch gekennzeichnet, daß** es ein flüssigkristallines Medium nach Anspruch 11 enthält.

13. Reflektives oder transflektives Flüssigkristall-Anzeigeelement, **dadurch gekennzeichnet, daß** es als Dielektrikum ein flüssigkristallines Medium nach Anspruch 11 enthält.

14. Elektrooptisches Anzeigeelement, **dadurch gekennzeichnet, daß** es als Dielektrikum ein flüssigkristallines Medium nach Anspruch 11 enthält.

15. Verwendung der Verbindungen der Formel I nach einem der Ansprüche 1 bis 9 als Ausgangsstoffe flüssigkristalliner Polymere.

16. Verwendung der Verbindungen der Formel I nach einem der Ansprüche 1 bis 9 zur Herstellung optischer Schalter oder optischer Filter.

17. Verbindungen der Formel I nach einem der Ansprüche 1 bis 9, die in Abhängigkeit der Wellenlänge des verwendeten Lichtes eine Inversion des Wertes der optischen Anisotropie aufweisen.

## Claims

1. Cyclohexane derivatives of the formula I 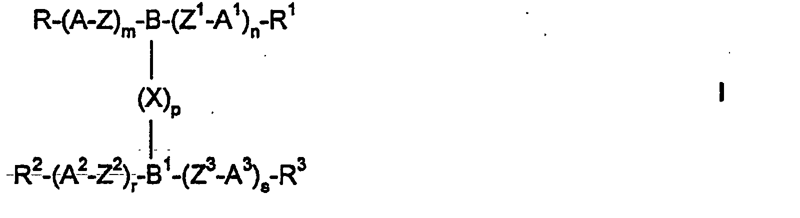 in which
B and B¹, independently of one another, are 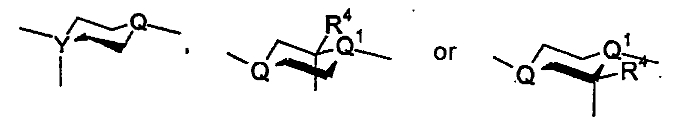 where the rings B and B¹ are each bonded to the (X)ₚ group via the axial bond, and, in addition, one or two non-adjacent CH₂ groups in these rings may be replaced by O,
Y, independently of one another in the case of multiple occurrence, is C or Si,
Q and Q¹, independently of one another, are CH or SiH,
X, independently of one another in the case of multiple occurrence, is -C≡C-, -CH=CH-, -CF=CF-, -CF=CH-, -CH₂-, -COO- or 1,4-phenylene,
in which, in addition, one or more CH groups may be replaced, independently of one another, by N or CF, but with the proviso that at least one X is -C≡C-,
p is 1, 2, 3 or 4,
R, R¹, R², R³ and R⁴, independently of one another, are H, an alkyl radical having 1-12 C atoms which is unsubstituted or at least monosubstituted by halogen, and in which, in addition, one or more CH₂ groups may each be replaced, independently of one another, by -O-, -S-, -CO-, 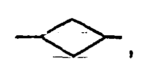 -CO-O-, -O-CO-, -O-CO-O- or -CH=CH- in such a way that heteroatoms are not bonded directly, or are -CN, -F, -CF₃, -OCHF₂, -OCF₃, -OCHFCF₃, -OCH₂CF₃, -OCF₂CF₃ or -CH=CF₂,
A, A¹, A² and A³, independently of one another, are 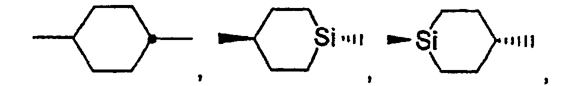 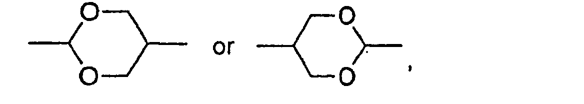 or a 1,4-phenylene radical, in which, in addition, one or two CH groups may be replaced by N, where the rings A, A¹, A² and A³ may be substituted, independently of one another, by one or more F atoms or -NCS, -CN, -CF₃ or -OCF₃,
Z, Z¹, Z² and Z³ are each, independently of one another, -CO-O-, -O-CO-, -CH₂O-, -CH=CH-, -O-, -O-CH₂-, -CH₂CH₂-, -(CH₂)₃-, -(CH₂)₄-, -CF₂-CF₂-, -OCF₂- or a single bond,
and
n, m, r and s, independently of one another, are 0, 1 or 2.

2. Cyclohexane derivatives according to Claim 1, **characterised in that** they have a value for the optical anisotropy Δn of < 0.05 at a reduced temperature of 0.9 and a wavelength of 589 nm.

3. Cyclohexane derivatives according to Claim 1 or 2, in which B and B¹, independently of one another, are 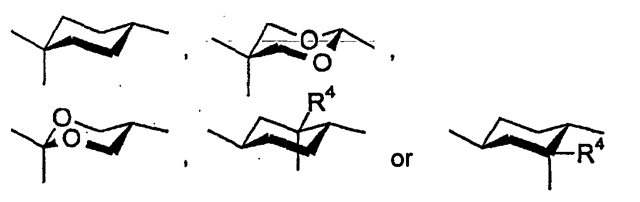 in which R⁴ is as defined above.

4. Cyclohexane derivatives according to Claim 1, 2 or 3, **characterised in that** Y is C, and Q and Q¹ are CH.

5. Cyclohexane derivatives according to Claim 1, 2, 3 or 4, in which Z, Z¹, Z² and Z³, independently of one another, are -CH₂CH₂-, -COO-, -OOC- or a single bond.

6. Cyclohexane derivatives according to one of Claims 1 to 5, **characterised in that** R, R¹, R² and R³, independently of one another, are -CN, -F, -OCF₃, -CF₃, -OCF₂CF₃, -CH=CF₂, straight-chain alkyl or alkoxy having from 1 to 10 C atoms or alkenyl or alkenyloxy having from 2 to 10 C atoms.

7. Cyclohexane derivatives according to one of Claims 1 to 6, **characterised in that** the (X)ₚ groups have one of the following meanings:
-C≡C-, -C≡C-C≡C-, -C≡C-C≡C-C≡C-, -C≡C-C≡C-C≡C-C≡C-, -C≡C-CH=CH-, -C≡C-CH=CH-C≡C-, -CH=CH-C≡C-CH=CH-, -C≡C-COO-, -CH₂-CH₂-C≡C-, -CH₂-CH₂-C≡C-C≡C-, -CH₂-CH₂-C≡C-CH=CH-,

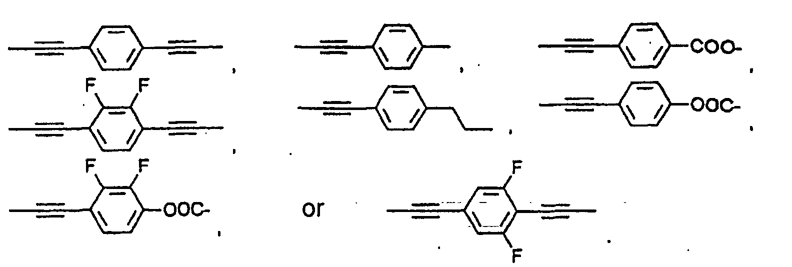

8. Cyclohexane derivatives according to one of the preceding claims, in which A, A¹, A² and/or A³, independently of one another, adopts one of the following meanings:

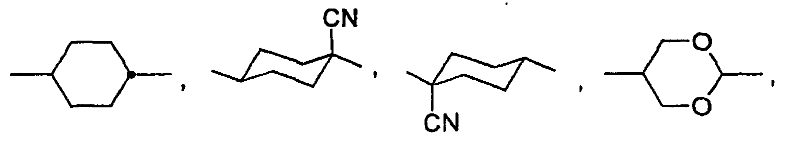

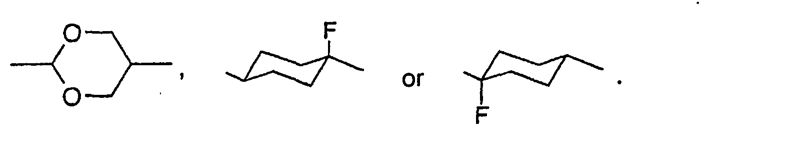

9. Cyclohexane derivatives according to one of the preceding claims, in which m, n, r and s, independently of one another, are 0 or 1.

10. Use of compounds of the formula I according to one of Claims 1 to 9 as components of liquid-crystalline media.

11. Liquid-crystalline medium having at least two liquid-crystalline components, **characterised in that** it comprises at least one compound of the formula I according to one of Claims 1 to 9.

12. Liquid-crystal display element, **characterised in that** it contains a liquid-crystalline medium according to Claim 11.

13. Reflective or transflective liquid-crystal display element, **characterised in that** it contains, as dielectric, a liquid-crystalline medium according to Claim 11.

14. Electro-optical display element, **characterised in that** it contains, as dielectric, a liquid-crystalline medium according to Claim 11.

15. Use of the compounds of the formula I according to one of Claims 1 to 9 as starting materials for liquid-crystalline polymers.

16. Use of the compounds of the formula I according to one of Claims 1 to 9 for the production of optical switches or optical filters.

17. Compounds of the formula I according to one of Claims 1 to 9 which have an inversion of the value for the optical anisotropy depending on the wavelength of the light used.

## Revendications

1. Dérivés du cyclohexane de formule I 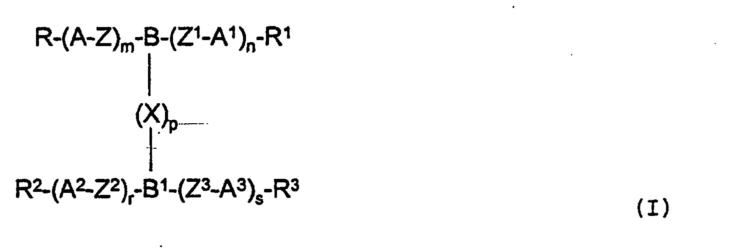 dans laquelle
B et B¹ représentent indépendamment l'un de l'autre 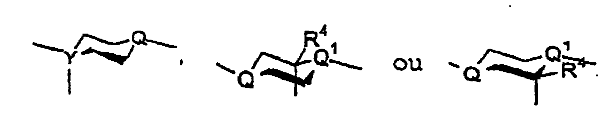
où les cycles B et B¹ sont liés au groupe (X)ₚ chacun par la liaison axiale et dans ces cycles également un ou deux groupes CH₂ non voisins peuvent être remplacés par O,
Y représente en cas d'occurrence répétée indépendamment l'un de l'autre C ou Si,
Q, Q¹ indépendamment l'un de l'autre CH ou SiH
X en cas d'occurrence répétée indépendamment l'un de l'autre -C≡C-, -CH=CH-, -CF=CF-, -CF=CH-, -CH₂-, -COO- ou 1,4-phénylène, dans lequel également un ou plusieurs groupes CH indépendamment l'un de l'autre peuvent être remplacés par N ou CF, cependant dans la mesure où au moins un X est -C≡C-,
p 1, 2, 3 ou 4,
R, R¹, R², R³, R⁴ indépendamment l'un de l'autre H, un reste alkyle avec 1 à 12 atomes de C non substitué, substitué au moins une fois par un halogène, dans lequel également un ou plusieurs groupes CH₂ chacun indépendamment l'un de l'autre peuvent être remplacés par -O-, -S-, -CO-, 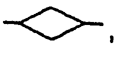 -CO-O, -O-CO-, -O-CO-O- ou -CH=CH-, de telle façon que les hétéroatomes ne sont pas directement liés, -CN, -F, -CF₃, -OCHF₂, -OCF₃, -OCHFCF₃, -OCH₂CF₃, -OCF₂CF₃ ou -CH=CF₂-,
A, A¹, A² et A³ indépendamment l'un de l'autre 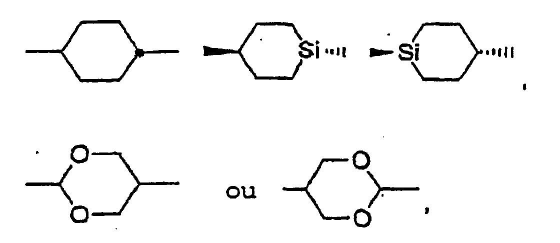 ou un reste 1,4-phénylène, dans lequel également un ou deux groupes CH peuvent être remplacés par N, moyennant quoi les cycles A, A¹, A² et A³ indépendamment l'un de l'autre peuvent être substitués par un ou plusieurs atomes de F ou -NCS, -CN, -CF₃ ou -OCF₃.
Z, Z¹, Z², Z³ chacun indépendamment l'un de l'autre -CO-O-, -O-CO-, -CH₂-O-, -CH=CH-, -O-, -OCH₂-, -CH₂CH₂-, -(CH₂)₃-, -(CH₂)₄-, -CF₂-CF₂-, -OCF₂- ou une liaison simple et
n, m, r, s indépendamment l'un de l'autre 0, 1 ou 2,

2. Dérivés du cyclohexane selon la revendication 1, **caractérisés en ce qu'**ils présentent une valeur d'anisotropie optique Δn de < 0,05 à une température réduite de 0,9 et une longueur d'onde de 589 nm.

3. Dérivés du cyclohexane selon la revendication 1 ou 2, dans lesquels B et B¹ indépendamment l'un de l'autre représentent 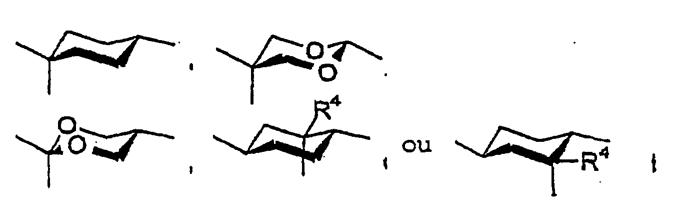 dans lesquels R⁴ présente la signification donnée ci-dessus.

4. Dérivés du cyclohexane selon la revendication 1, 2 ou 3, **caractérisés en ce que** Y représente C et Q et Q¹ CH.

5. Dérivés du cyclohexane selon la revendication 1, 2, 3 ou 4, dans lesquels Z, Z¹, Z² et Z³ indépendamment l'un de l'autre représentent -CH₂-CH₂-, -COO-, -OOC- ou une liaison simple.

6. Dérivés du cyclohexane selon l'une des revendications 1 à 5, **caractérisés en ce que** R, R¹, R² et R³ indépendamment l'un de l'autre représentent -CN, -F, -OCF₃, -CF₃, -OCF₂CF₃, -CH=CF₂-, un alkyle ou alcoxy à chaîne linéaire avec 1 à 10 atomes de C ou un alcényle ou alcényloxy avec 2 à 10 atomes de C.

7. Dérivés du cyclohexane selon l'une des revendications 1 à 6, **caractérisés en ce que** les groupes (X)ₚ présentent une des significations suivantes : -C≡C-, -C≡C-C≡C-, -C≡C-C≡C-C≡C-, -C≡C-C≡C-C≡C-C≡C-, -C≡C-CH=CH-, -C≡C-CH=CH-C≡C-, -CH=CH-C≡C-CH=CH-, -C≡C-COO-, -CH₂-CH₂-C≡C-, -CH₂-CH₂-C≡C-C≡C-, -CH₂-CH₂-C≡C-CH=CH-,

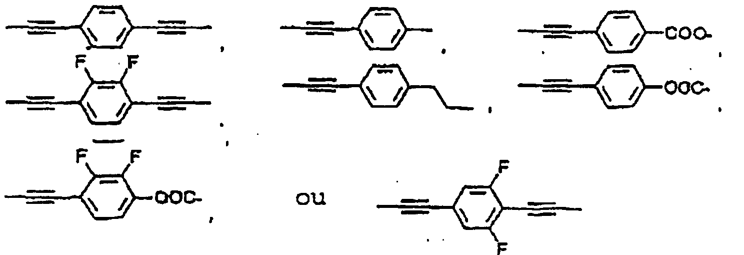

8. Dérivés du cyclohexane selon l'une des revendications mentionnées ci-dessus, dans lesquels A, A¹, A² ou/et A³ indépendamment l'un de l'autre prend l'une des significations suivantes :

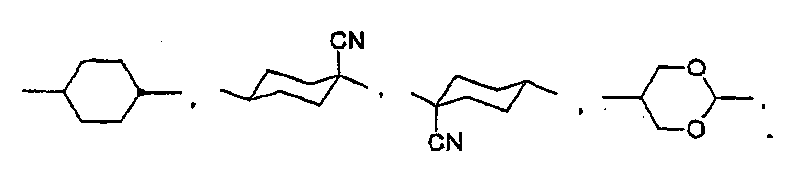

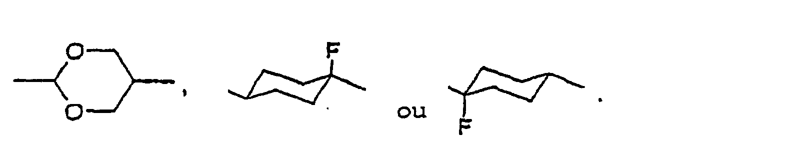

9. Dérivés du cyclohexane selon l'une des revendications mentionnées ci-dessus, dans lesquels m, n, r et s indépendamment l'un de l'autre signifient 0 ou 1.

10. Utilisation de composés de formule I selon l'une des revendications 1 à 9 en tant que composants de milieux liquides cristallins.

11. Milieu liquide cristallin avec au moins deux composants liquides cristallins, **caractérisé en ce qu'**il comprend au moins un composé de formule I selon l'une des revendications 1 à 9.

12. Cellule de visualisation à cristaux liquides, **caractérisé en ce qu'**il comprend un milieu liquide cristallin selon la revendication 11.

13. Cellule de visualisation à cristaux liquides réflectif ou transflectif, **caractérisé en ce qu'**il contient en tant que matière diélectrique un milieu liquide cristallin selon la revendication 11.

14. Cellule de visualisation électro-optique, **caractérisé en ce qu'**il contient en tant que matière diélectrique un milieu liquide cristallin selon la revendication 11.

15. Utilisation des composés de formule I selon l'une des revendications 1 à 9 en tant que produit de départ de polymères liquides cristallins.

16. Utilisation des composés de formule I selon l'une des revendications 1 à 9 pour fabriquer un interrupteur optique ou un filtre optique.

17. Composés de formule I selon l'une des revendications 1 à 9, qui présentent en fonction de la longueur d'onde de la lumière utilisée une inversion de la valeur de l'anisotropie optique.
